(19) 

Europäisches
Patentamt
European
Patent Office
Office européen
des brevets

(11) **EP 4 159 873 A1**

(12) **EUROPEAN PATENT APPLICATION**
published in accordance with Art. 153(4) EPC

(43) Date of publication:
**05.04.2023 Bulletin 2023/14**

(21) Application number: **21812570.6**

(22) Date of filing: **28.05.2021**

(51) International Patent Classification (IPC):
***C12Q 1/6869*** (2018.01)    ***C12Q 1/02*** (2006.01)
***C12Q 1/686*** (2018.01)

(52) Cooperative Patent Classification (CPC):
**C12Q 1/02; C12Q 1/686; C12Q 1/6869**

(86) International application number:
**PCT/JP2021/020338**

(87) International publication number:
**WO 2021/241721 (02.12.2021 Gazette 2021/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.05.2020 JP 2020094141**

(71) Applicant: **RIKEN**
**Wako-shi, Saitama 351-0198 (JP)**

(72) Inventors:
• **SHIROGUCHI Katsuyuki**
**Wako-shi, Saitama 351-0198 (JP)**

• **JIN Jianshi**
**Wako-shi, Saitama 351-0198 (JP)**
• **YAMAMOTO Reiko**
**Wako-shi, Saitama 351-0198 (JP)**

(74) Representative: **Marks & Clerk LLP**
**15 Fetter Lane**
**London EC4A 1BW (GB)**

Remarks:
The complete document including Reference
Table(s) and the Sequence Listing(s) can be
downloaded from the EPO website

(54) **METHOD FOR TREATING CELL POPULATION AND METHOD FOR ANALYZING GENES INCLUDED IN CELL POPULATION**

(57) The present invention provides a method for treating a cell population and a method for analyzing a cell population (e.g., microbiota). The present invention can comprise obtaining a droplet population from a cell dispersion comprising an isolated cell population, the droplet population comprising aqueous droplets, at least some of which each comprise one cell and one-molecule cellular barcode.

FIG. 1

**Description**

Technical Field

The present invention relates to a method for treating a cell population and a method for analyzing genes included in a cell population.

Background Art

To essentially understand how the composition of a commensal microbiota contributes to the health of a host,[1,2] the microbiota should be simply defined at a cell level because a cell is a fundamental physical unit of a microbiota.[3-5] However, such a definition is difficult with the current leading-edge techniques.[6-8]

Interactions between a microbiota and a host thereof are associated with homeostasis and many diseases of the host.[13-16] To further understand the mechanism of the microbiota-host interactions in an integrated manner, it is important not only to study the microbiota, but also to link compositional analyses of the microbiota to other analyses, such as metabolomics and/or transcriptomics for both the microbiota and the host.[5] Achieving this purpose requires measurement of concentrations based on a commonly usable unit such as, for example, the number of cells per weight and/or the number of molecules per volume. In relation to this point, techniques to count the number of nucleic acid molecules present in a cell have been developed (Patent Literatures 1 to 3). With these counting techniques, the number of molecules is estimated by labeling each molecule with a unique nucleic acid sequence (barcode) and counting the number of barcode types. In Patent Literatures 1 to 3, errors occurring during amplification of nucleic acids or reading errors occurring during sequencing can cause errors in the counted number of molecules. A technique to reduce these errors has also been developed (Patent Literature 4). Patent Literature 4 has proposed a method of removing errors and correcting the counted number in consideration of the natures of errors occurring during amplification of nucleic acids and reading errors occurring during sequencing. However, it has been difficult to measure the microbiota composition at a cell level with the current techniques.[6-8] Additionally, a microbiota comprises an enormous number of bacteria of a large number of bacterial species.[17] However, a high throughput cell quantification method with a high taxonomic resolution ability has not been developed so far.

High throughput methods based on sequencing of 16S rRNA gene amplicons using next-generation sequencing techniques have contributed to studies on bacterial diversity.[22,23] However, conventional methods have the following fundamental limitations because they amplify the 16S rRNA genes from a purified bulk bacterial genome and measure the number of amplified molecules. 1) Since a different species has a different copy number of the 16S rRNA gene on the genome, and the copy number is unknown for the majority of species, it is difficult to measure the numbers of cells and compare the numbers of cells of different species. 2) Identification of 16S rRNA sequences is not accurate because of sequencing and amplification errors, resulting in a low taxonomic resolution ability. In fact, sequencing errors have been corrected using molecular barcodes,[24-26] but mainly amplification errors due to chimera generation cannot be sufficiently removed.[27]

Citation List

Patent Literature

Patent Literature 1: U.S. Patent No. 9260753B
Patent Literature 2: U.S. Patent No. 10287630B
Patent Literature 3: U.S. Patent No. 10584382B
Patent Literature 4: International Publication No. WO 2018/235938

Summary of Invention

The present invention provides a method for treating a cell population and a method for analyzing genes included in a cell population.

The present inventors developed a novel method for quantifying cell types in a bacterial microbiota and the cell concentration for each cell type using a high throughput method. The present inventors also found a method that addresses a state in which genes to be analyzed exist in multiplicate in one cell. The method enables fine classification of unknown cells (e.g., microorganisms) having gene multiplication and estimates the numbers of the cells by classifying gene groups to be analyzed into cell-based operational taxonomic units (cOTUs).

**EP 4 159 873 A1**

According to the present invention, the following inventions are provided:

[1] A method for treating a cell population, the method comprising

(A) obtaining a droplet population from a cell dispersion comprising an isolated cell population, the droplet population comprising aqueous droplets, at least some of which each comprise one cell and one-molecule cellular barcode.

[2] A method for analyzing nucleotide sequences of genes included in a cell population, the method comprising

(A) obtaining a droplet population from a cell dispersion comprising an isolated cell population, the droplet population comprising aqueous droplets, at least some of which each comprise one cell and one-molecule cellular barcode; and
(B) obtaining an amplification product of the cellular barcode and an amplification product of a predetermined gene in each obtained droplet, further obtaining a linked product comprising nucleotide sequences of the cellular barcode and all or some of the predetermined gene, and collecting the obtained linked product from the droplets into an aqueous solution and sequencing the obtained linked product to determine the nucleotide sequence of the predetermined gene and the nucleotide sequences of the cellular barcode.

[3] The method according to [2], wherein, in the (B), the amplification product of the cellular barcode has a first region derived from a first primer, the amplification product of the predetermined gene has a second region derived from a second primer, the first region and the second region have complementary sequence portions hybridizable with each other, the first primer and the second primer each have one or more tag molecules linked thereto, and the tag molecule is not included in the linked product; and
the method further comprising removing the amplification product having a tag molecule from the linked products collected into the aqueous solution using a column or bead carrying a molecule having an affinity for the tag molecule in the (B).
[4] The method according to [2] or [3], further comprising
(C-1) clustering the determined nucleotide sequences based on the determined nucleotide sequence of the cellular barcode to obtain a plurality of first clusters.
[5] The method according to [4], further comprising
(D-1) estimating the number of cells included in the cell population or the number of cells having a specific predetermined gene from the number of the obtained first clusters.
[6] The method according to [2] or [3], further comprising
(C-2) clustering the determined nucleotide sequences based on the determined nucleotide sequence of the predetermined gene to obtain a plurality of second clusters.
[7] The method according to [6], further comprising
(D-2) estimating the number of cell types included in the cell population from the number of the obtained second clusters.
[8] The method according to [2] or [3], further comprising
(C-3) clustering the determined nucleotide sequences based on the determined nucleotide sequence of the cellular barcode to obtain a plurality of first clusters, and clustering the determined nucleotide sequences based on the determined nucleotide sequence of the predetermined gene to obtain a plurality of second clusters.
[9] The method according to [8], further comprising
(D-3) determining the first cluster into which the nucleotide sequence of the predetermined gene is classified from the nucleotide sequence of a cellular barcode linked to the nucleotide sequence of the predetermined gene classified into at least one second cluster based on information on combinations of the obtained nucleotide sequence of the cellular barcode and the obtained nucleotide sequence of the predetermined gene, and estimating the number of cells classified into the second cluster from the number of the first clusters into which the cellular barcode is classified.
[10] The method according to [8], further comprising
(C-4) when sequences classified into one identical first cluster are classified into different second clusters, classifying the second clusters into one identical cell-based operational taxonomic unit (cOTU).
[11] The method according to [10], further comprising
(E) estimating (i) the number of cOTUs included in the cell population and/or (ii) the number of cells included in a specific cOTU for each of a first cell population and a second cell population different from the first cell population, and comparing (i) the number of cOTUs and/or (ii) the number of cells included in the specific cOTU estimated for the first cell population with (i) the number of cOTUs and/or (ii) the number of cells included in the specific cOTU estimated for the second cell population.

3

[12] The method according to [11], comprising

(F) comparing (i) the number of cOTUs and (ii') the number of cells included in the specific cOTU estimated for the first cell population with (i) the number of cOTUs and (ii') the number of cells included in the specific cOTU estimated for the second cell population.

[13] The method according to any of [1] to [12], wherein the cell population is a microbiota.

[14] The method according to [13], wherein the microbiota is a microbiota in the body or on the body surface.

[15] The method according to [13], wherein the microbiota is a microbiota in the gastrointestinal tract.

[16] The method according to [11] or [12], wherein the first cell population and the second cell population are microbiotas obtained from different sites of an identical subject.

[17] The method according to [11] or [12], wherein the first cell population and the second cell population are microbiotas obtained from an identical site of different subjects.

[18] The method according to [11] or [12], wherein the first cell population and the second cell population are microbiotas obtained from an identical site of an identical subject at different time point.

[19] The method according to any of [1] to [18], wherein the cell population includes unknown cells.

Brief Description of Drawings

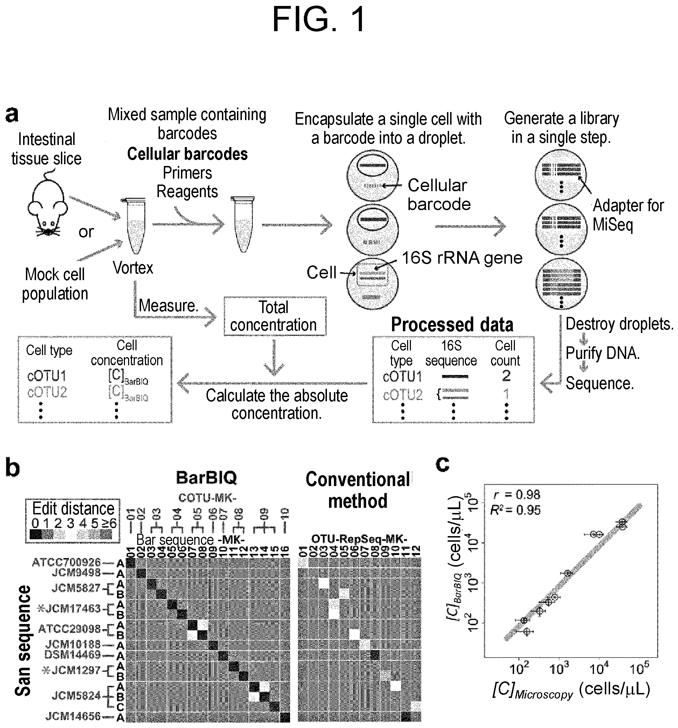

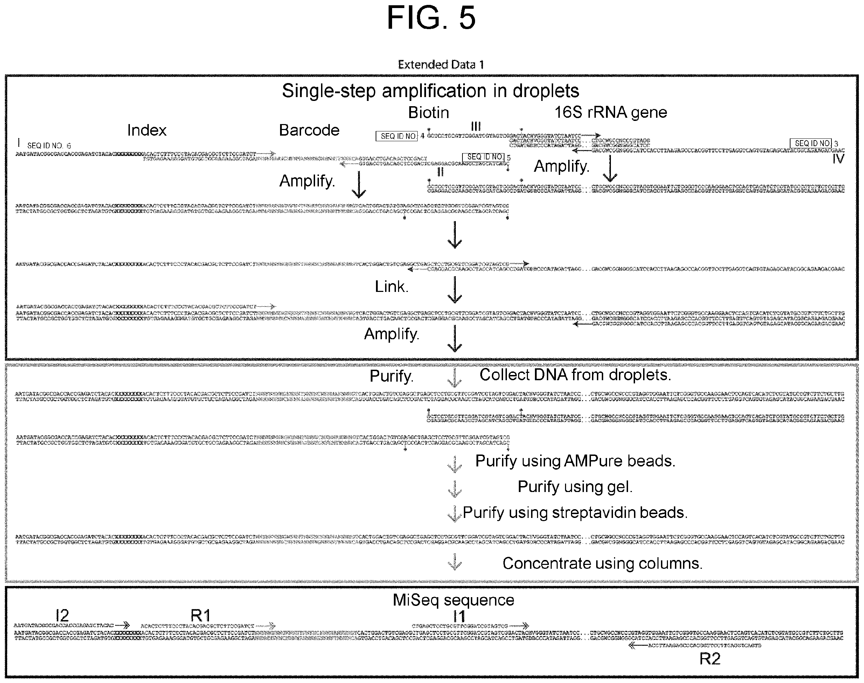

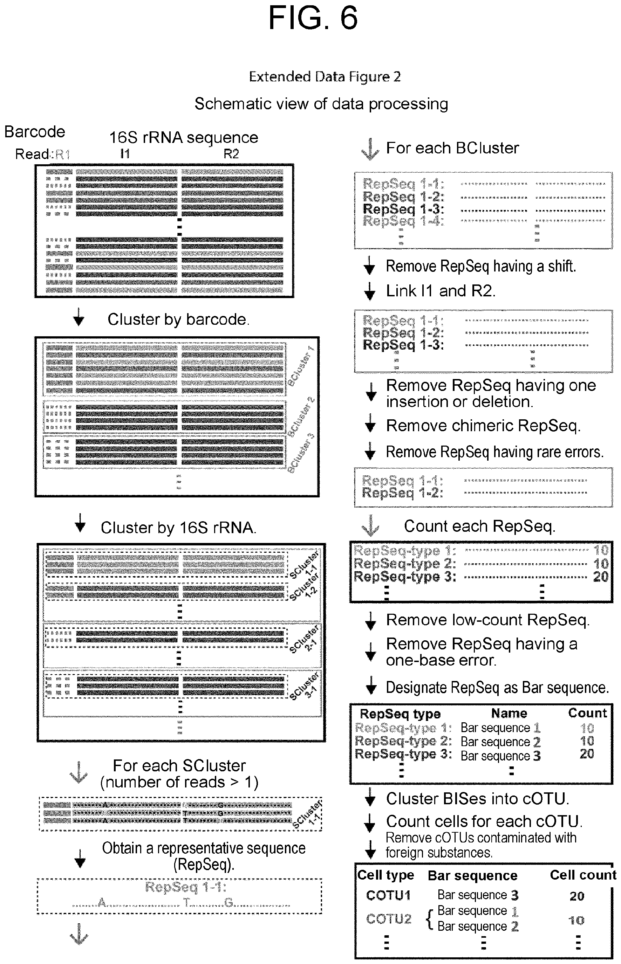

[Figure 1] Figure 1 illustrates BarBIQ and efficacy thereof. Figure 1a is a schematic view of BarBIQ. A sample was suspended in a solution, and then the mixture was vortexed to break bacterial aggregates. Cellular barcode, a DNA molecule that comprises nucleotides unique to a cell (a nucleotide sequence different for each one molecule) and a prime site for amplification; primers, DNA primers for amplifying each of the 16S rRNA gene and the cellular barcode, for linking the amplification products of both, and for attaching a sequencing adapter; reagents, reagents for DNA amplification. See Figure 5 for a schematic view for details of library generation, purification, and sequencing. See Figure 6 for details of data processing. Figure 1b shows efficacy of BarBIQ using a mock cell population. Edit distance, Levenshtein distance[29] defined as a minimum number of substitutions, insertions, and deletions; San sequence, 16S rRNA sequence identified by Sanger sequencing; ATCC/JCM/DSM-<number>, strain ID; A, B, or C, San sequence for each strain; Bar sequence-MK-XX (01-16), a sequence identified by BarBIQ (Bar sequence); COTU-MK-XX (01-10), cell-based operational taxonomic unit (cOTU); red asterisk symbol, a Bar sequence having a one-nucleotide difference; OTU-RepSeq-MK-XX (01-12), a sequence representing an OUT. Figure 1c shows a comparison of cell concentrations of 10 strains in the mock cell population measured by BarBIQ ($[C]_{BarBIQ}$) and a microscopic imaging ($[C]_{Microscopy}$) (data from Tables 1 and 2). Blue line, a fitting line with a slope fixed at 1 on a logarithmic scale; r, Pearson coefficient; R2, a coefficient of determination. Error bar, a standard deviation (n = 3 for $[C]_{BarBIQ}$; n = 5 for $[C]_{Microscopy}$).

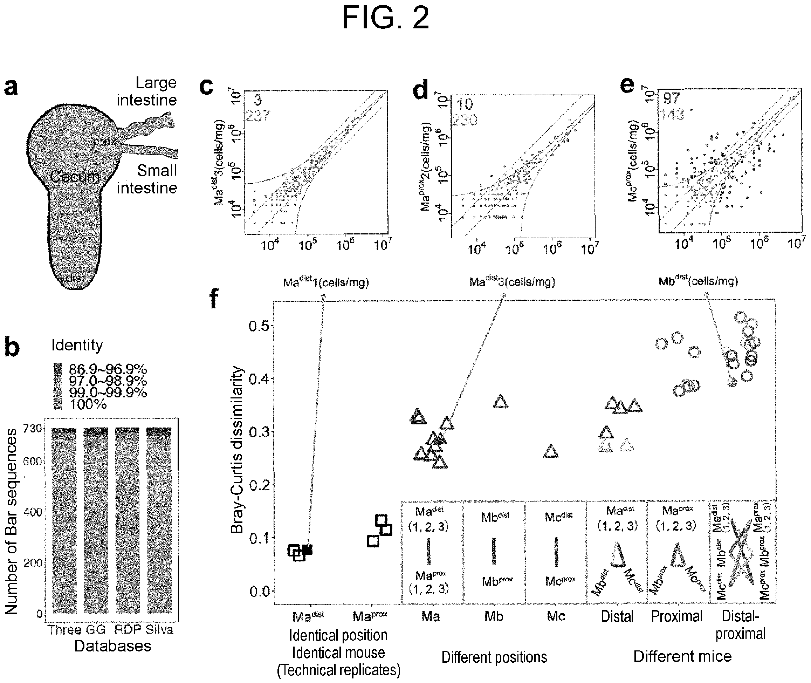

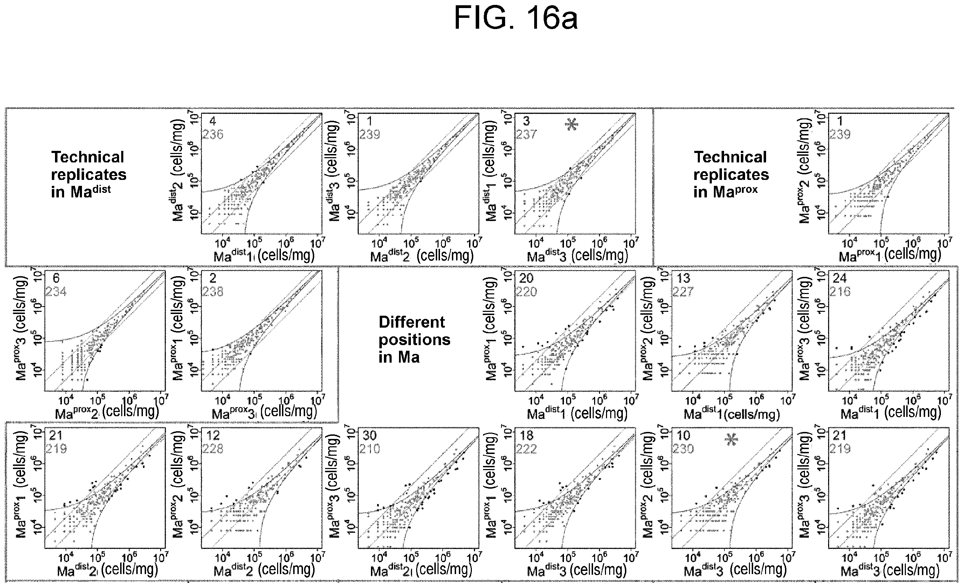

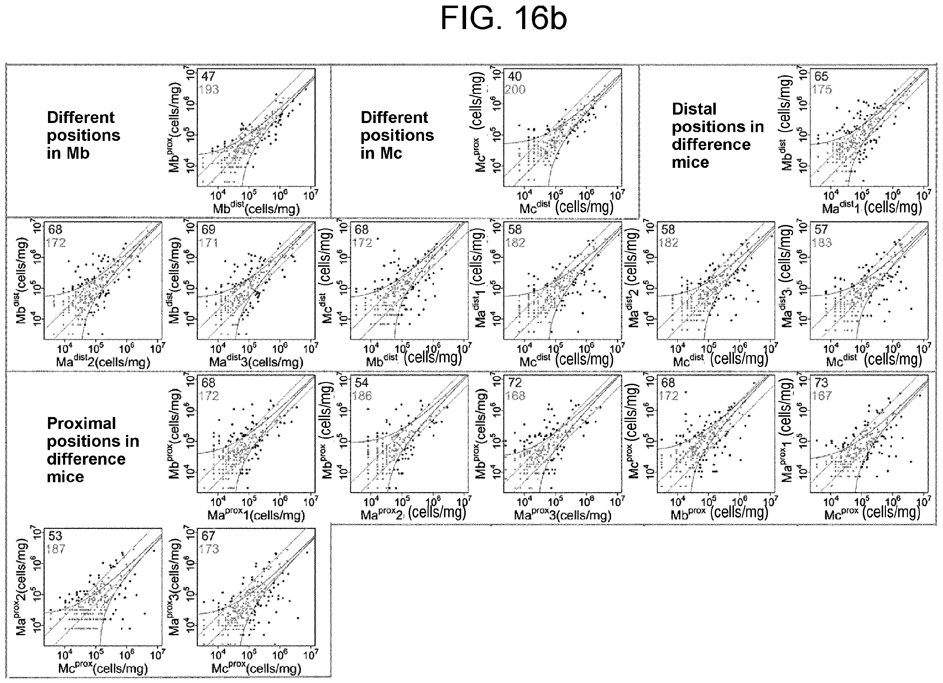

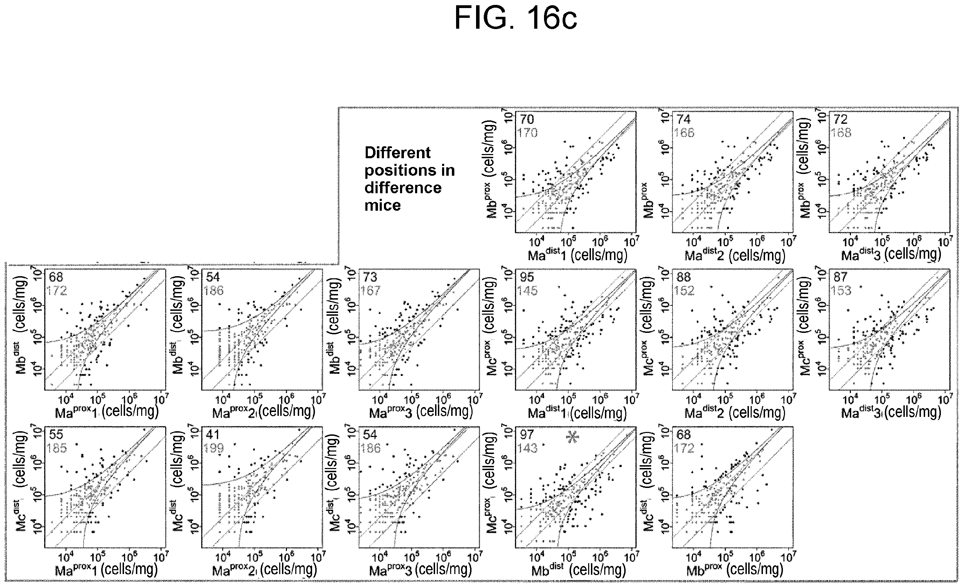

[Figure 2] Figure 2 shows comprehensive analyses of the murine cecal microbiota. Figure 2a shows distal (dist) and proximal (prox) sampling positions in the murine cecum. Figure 2b shows sequence identity profiles of Bar sequences. Identity, each Bar sequence and identity between the closest 16S rRNA sequence in three common public databases, GreenGene (GG), Ribosomal Database Project (RDP), and Silva; Three, a combination of all three databases. Figure 2c shows a comparison of cell concentrations in cOTUs between technical replicates (see Figure 16 for other replicates). Magenta line, the Poisson distribution and a theoretical confidence interval of sampling noises based on normalization by the total concentration (99.9%); light blue line, two-fold change; blue dot, a cOTU showing a different concentration; inserted number, the number of blue or grey dots; Ma, Mb, and Mc, mice; dist and prox, positions; 1, 2, and 3, technical replicates. Figures 2d and 2e show the same comparisons for different samples as shown in Figure 2c with examples of different minimum (Figure 2d) and maximum (Figure 2e) numbers of cOTUs (blue dots). See Figure 16 for comparisons of other samples. Figure 2f shows Bray-Curtis dissimilarity in microorganisms between samples. The labels have the same meanings as in Figures 2c to 2e.

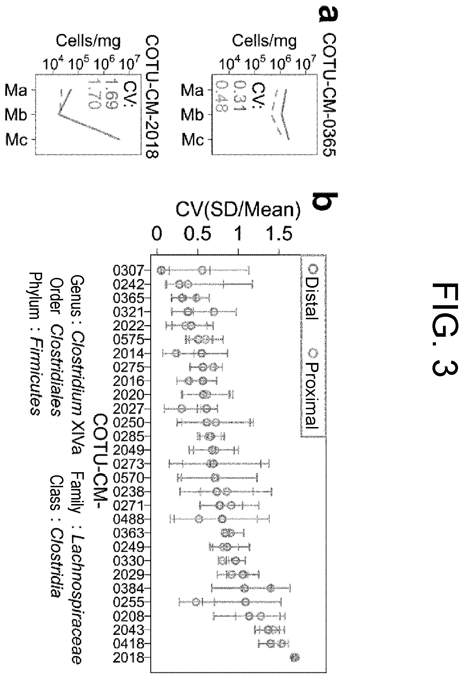

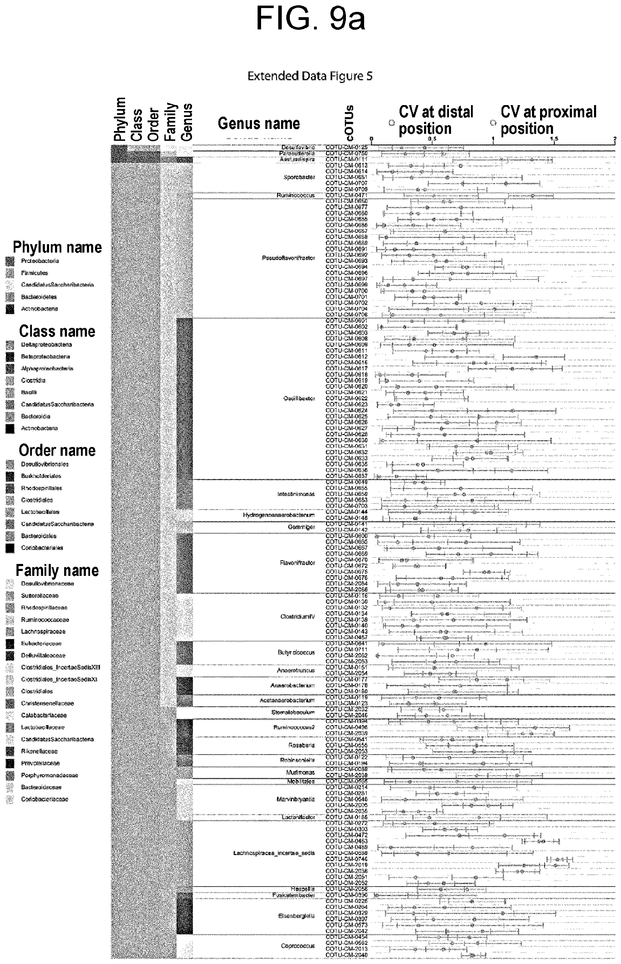

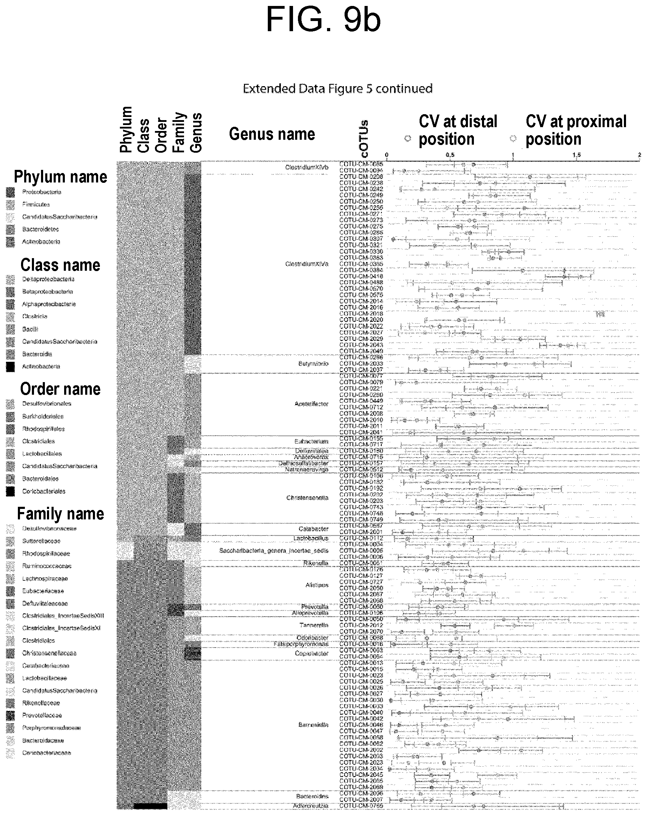

[Figure 3] Figure 3 shows variations in cell concentrations for individual cOTUs among mice. Figure 3a shows examples of cell concentrations in a cOTU at distal (red solid line) and proximal (light blue broken line) positions in three mice (Ma, Mb, and Mc). CV, coefficient of variation. Figure 3b shows CV for the cOTU of the genus Clostridium XIVa (all detected genera are shown in Figures 9a and 9b). COTU-CM-<number>, cOTU ID; distal and proximal, positions; error bar, 95% confidence interval of CV for each cOTU obtained by a simulation assuming sampling noises and technical errors in measurement of total concentrations.

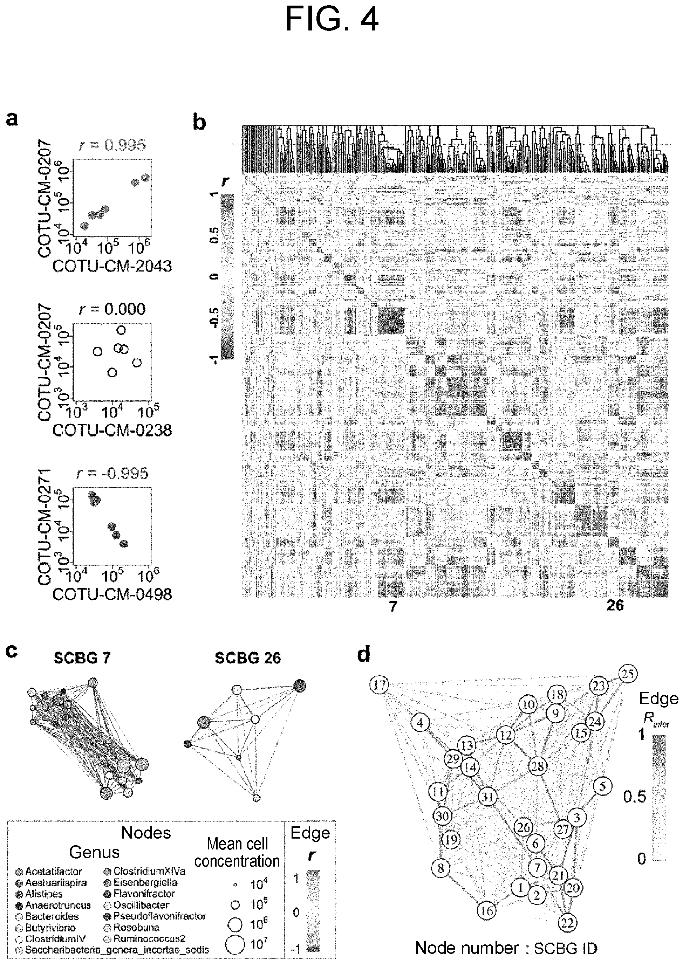

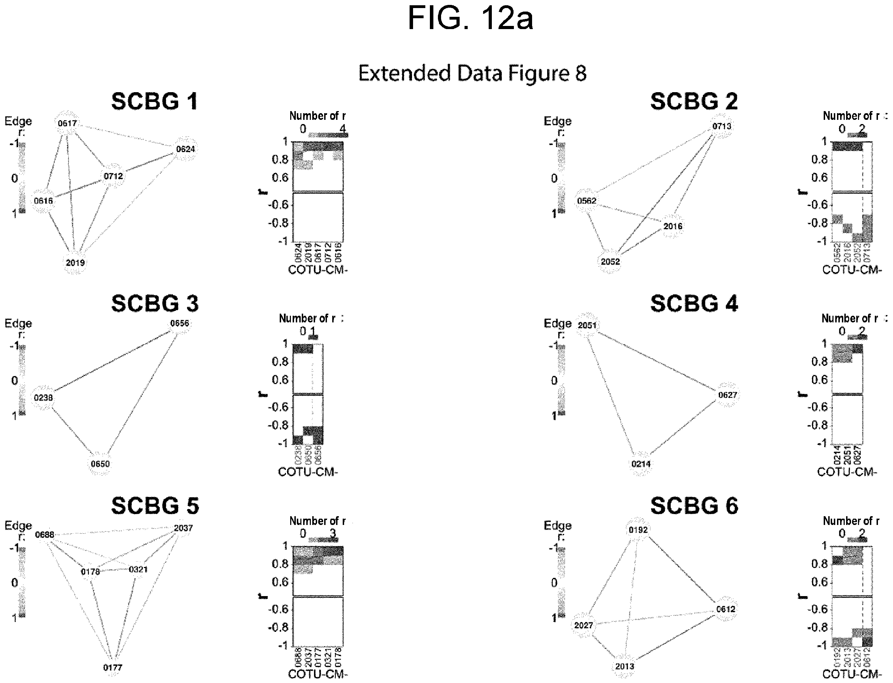

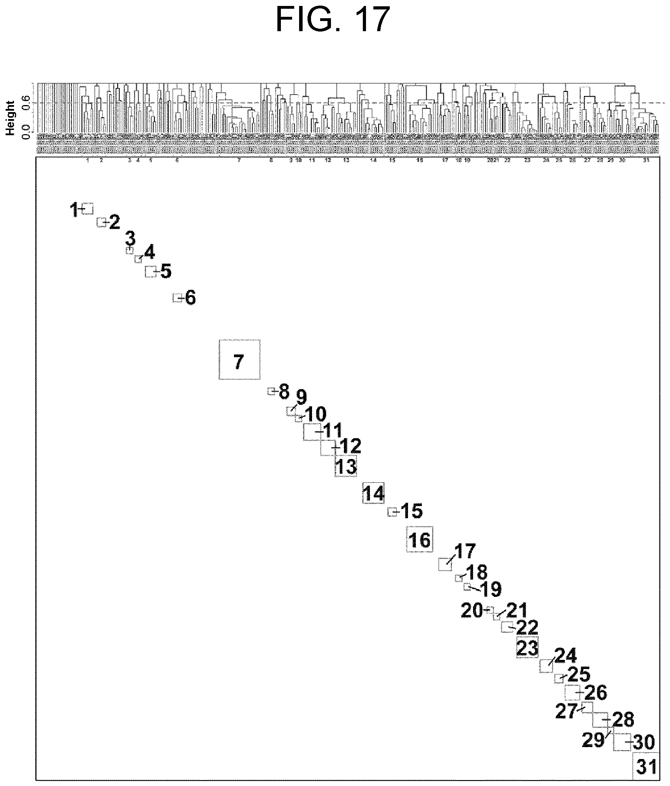

[Figure 4] Figure 4 shows bacterial correlation networks. Figure 4a shows examples of correlations based on richness of cOTU pairs. Dot, cell concentration (cells/mg) for six samples ($Ma^{dist}1$, $Ma^{prox}1$, $Mb^{dist}$, $Mb^{prox}$, $Mc^{dist}$, and $Mc^{prox}$); r, Pearson coefficient. Figure 4b shows definitions of strongly correlated bacteria groups (SCBGs). Dendrogram, hierarchical clustering of 296 cOTUs detected commonly in all six samples based on the defined distance, 1 - minimum ($|r'|$) [$r' \in (r - OCI, r + OCI)$]; red broken line, a threshold of 0.6; heat map, r for all cOTUs; white gap in the heat map, an interval showing separation of branches lower than the threshold of 0.6 in both vertical and horizontal directions; number at the bottom, SCBG ID. A dendrogram having names and IDs of cOTUs for all SCBGs is shown in Figure 17. Figure 4c shows the cOTU networks in SCBG7 and SCBG26 visualized using a force-directed layout.[39] Node, cOTU; Node size, the mean cell concentration for a cOTU in six samples as shown in Figure 4a; edge color, r between cOTUs linked at the end. The visualized networks in all SCBGs are shown in Figures 12a to 12f. Figure 4d shows a network of SCBGs visualized using a force-directed layout. Edge color, $R_{inter}$, an interrelationship between two SCBGs.

[Figure 5] Figure 5 is a schematic view including sequence information for library generation, purification, and sequencing in BarBIQ. I, II, III, and IV, designed primers designated as P5-index-R1P-barcode-R, Biotin-Link-barcode-F, Biotin-link-805R, and P7-R2P-341F; Index (XXXXXXXX), eight designed nucleotides; barcode, random and fixed nucleotides (other three kinds of barcodes are shown in Table 3); N, A, C, G, or T in the sequence; I2, R1, and R2, Illumina sequencing primers for MiSeq; I1, a customized sequencing primer.

[Figure 6] Figure 6 is a schematic view of BarBIQ data processing. Black arrow, a processing step; red arrow, description of an operand at a next step; barcode, a cellular barcode; R1, a read of R1; I1 and R2, I1 and R2 reads that obtained by trimming low-quality ends and the primer portions; BCluster, a cluster obtained by clustering by barcode; SCluster, a subcluster obtained by clustering by 16S rRNA sequence in each BCluster; shifted RepSeq, RepSeq resulting from an insertion or deletion that occurred in the primer portion of a read; RepSeq having one insertion or deletion, RepSeq resulting from an error of one-nucleotide insertion or deletion that occurred in the remaining portion of the read after trimming; chimeric RepSeq, RepSeq that can occur from PCR chimera formation; RepSeq having rare errors, RepSeq resulting from errors of one indel (insertion or deletion) and one substitution, one indel and two substitutions, or two indels in the remaining portion of the read after trimming; RepSeq type, sequence type of RepSeq; low-count RepSeq, RepSeq type detected in a small number of BClusters; RepSeq having a one-nucleotide error, RepSeq type having a one-nucleotide difference from another RepSeq, with the number of RepSeqs detected between the former and latter RepSeq types being smaller than a threshold; Bar sequence, a sequence identified by BarBIQ; cOTU, cell-based operational taxonomic unit.

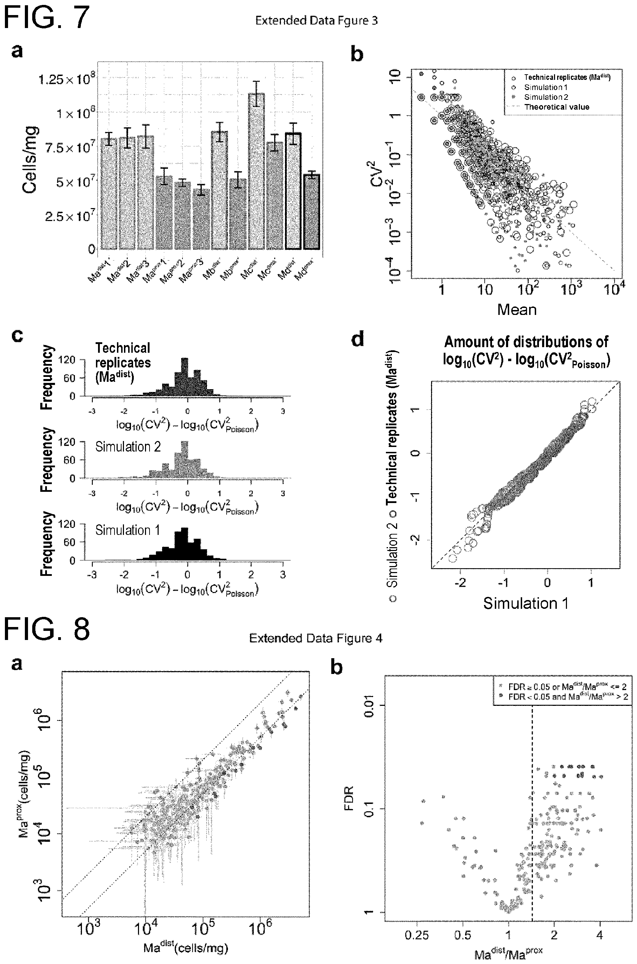

[Figure 7] Figure 7 shows absolute cell concentrations for each cOTU and total concentrations used to calculate sampling noises in each cOTU during BarBIQ measurement. Figure 7a shows the total bacterial concentrations in each sample measured by droplet digital PCR (see the BarBIQ method section in the Examples). Ma, Mb, Mc, and Md (not sequenced for Md), mice; dist and prox, positions (see Figure 2a); 1, 2, and 3, technical replicates; error bar, standard deviation (n = 5). Figure 7b shows $CV^2$ (CV, coefficient of variation) of counts for each cOTU in three technical replicates in $Ma^{dist}$ as a function of mean counts. Simulations 1 and 2 and theoretical values were obtained based on the Poisson distribution. Figure 7c shows distributions of $\log_{10}(CV^2) - \log_{10}(CV_{Poisson}^2)$. CV, CV of each cOTU; $CV_{Poisson}$, theoretical CV based on the Poisson distribution. Figure 7d shows a Q-Q plot[45] of distributions of $\log_{10}(CV^2) - \log_{10}(CV_{Poisson}^2)$ between the measurement for $Ma^{dist}$ and Simulation 1 and between Simulation 1 and Simulation 2. The distributions of $\log_{10}(CV^2) - \log_{10}(CV_{Poisson}^2)$ were comparable between the measurement and the simulations, indicating that the noises in each detected cOTU were mainly due to sampling.

[Figure 8] Figure 8 shows comparisons between position-dependent cell concentrations in each cOTU in mouse Ma. Figure 8a shows a comparison of the mean cell concentrations in each cOTU for three technical replicates between the distal position ($Ma^{dist}$) and the proximal position ($Ma^{prox}$) in mouse Ma. Error bar, standard deviation (n = 3); red dot, with FDR < 0.05 and the mean change factor of > 2 (see Figure 8b); broken line, with the change factor = 2. Figure 8b is a Volcano plot that shows differences in cell concentrations in cOTUs between the distal and proximal positions in Ma. The false discovery rate (FDR) was determined by a function p.adjust (R package: stats) using the BH method based on the p value of all 240 cOTUs calculated by function t test (R package: stats) using two-sided two-group t test (n = 3)[46]; $Ma^{dist}/Ma^{prox}$, a ratio of the mean cell concentration in $Ma^{dist}$ to the mean cell concentration in $Ma^{prox}$; broken line, the ratio of the total concentrations in $Ma^{dist}$ and $Ma^{prox}$.

[Figure 9a] Figure 9a shows CVs (coefficient of variations) for taxa of all cOTUs. Left, classification from phylum to genus. Right, CV for each cOTU at the distal and proximal position. COTU-CM-<number>, cOTU ID; error bar, 95% confidence interval obtained in simulation on the assumption of sampling noises and technical errors in measurement of total concentrations.

[Figure 9b] Same as above.

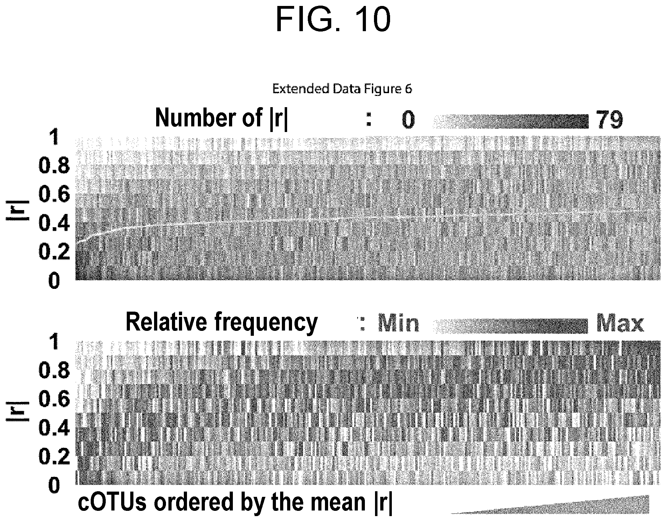

[Figure 10] Figure 10 shows characteristics of correlations of each cOTU to other cOTUs in the whole network. The upper column shows distributions of |r| between a given cOTU and all the other cOTUs, and |r| is an absolute Pearson correlation coefficient. cOTUs were aligned by the mean |r| for each cOTU along the horizontal axis (cyan line). The lower column shows distributions of |r| for each cOTU shown by relative frequency. The values in each line of the upper diagram were normalized by the minimum value (as 0) and the maximum value (as 1) thereof (i.e., normalization along the horizontal axis). This analysis enables finding of the "master bacteria," which are bacteria (i.e., cOTUs) highly correlated to the majority of other bacteria in the bacterial correlation network.

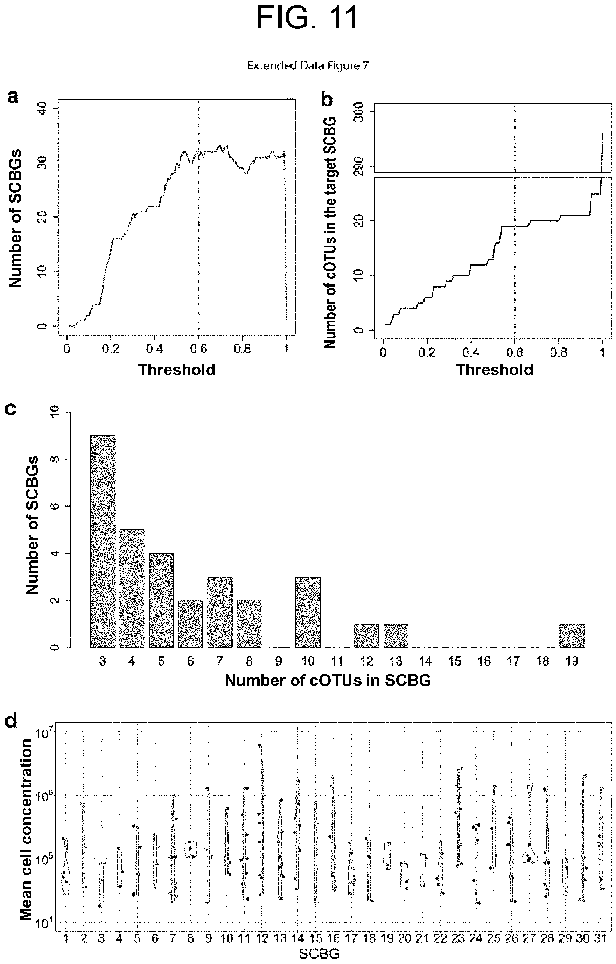

[Figure 11] Figure 11 shows an analysis of strongly correlated bacteria groups (SCBGs). Figure 11a shows the number of SCBGs as a function of thresholds of heights of the dendrogram (Figure 4b). Red dotted line, a threshold of 0.6. Figure 11b shows the number of cOTUs in the SCBG including the largest number of cOTUs as a function of the thresholds. Figure 11c shows distribution of numbers of cOTUs in the SCBGs when the threshold was 0.6. Figure 11d shows the mean cell concentrations in cOTUs in each SCBG for the samples $Ma^{dist}1$, $Ma^{prox}1$, $Mb^{dist}$, $Mb^{prox}$, $Mc^{dist}$, and $Mc^{prox}$. Black dots indicate that all cOTUs in the SCBGs positively correlated. Purple dots and light blue dots indicate that all cOTUs positively correlated. cOTUs in different subgroups showed a negative correlation.

[Figure 12a] Figure 12a shows networks and r distributions indicating the intensity of relative correlations between each cOTU and other cOTUs in each SCBG. Left diagrams show networks of SCBGs visualized using a force-directed layout.[39] Node, a cOTU; node number, cOTU ID; edge color, r between linked cOTUs; ID colors, the same meanings as dot colors in Figure 11d. Right graphs show distributions of r between a given cOTU and all the other cOTUs in the SCBG. First, cOTUs were divided into subgroups (ID colors), and then each cOTU in each subgroup was aligned along the mean of all positive r (blue line).

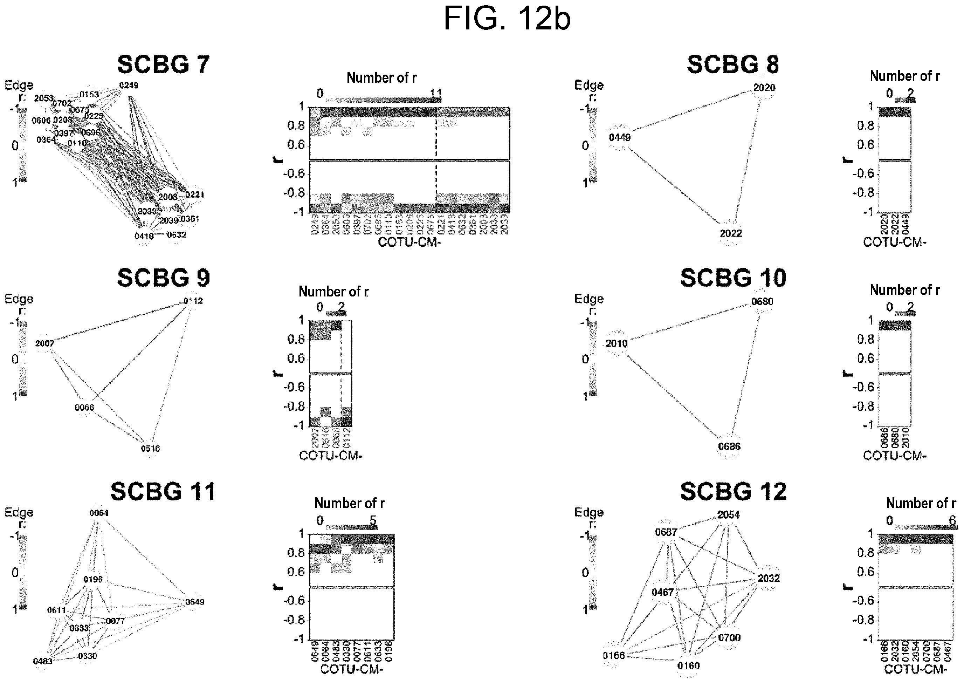

[Figure 12b] Same as above.

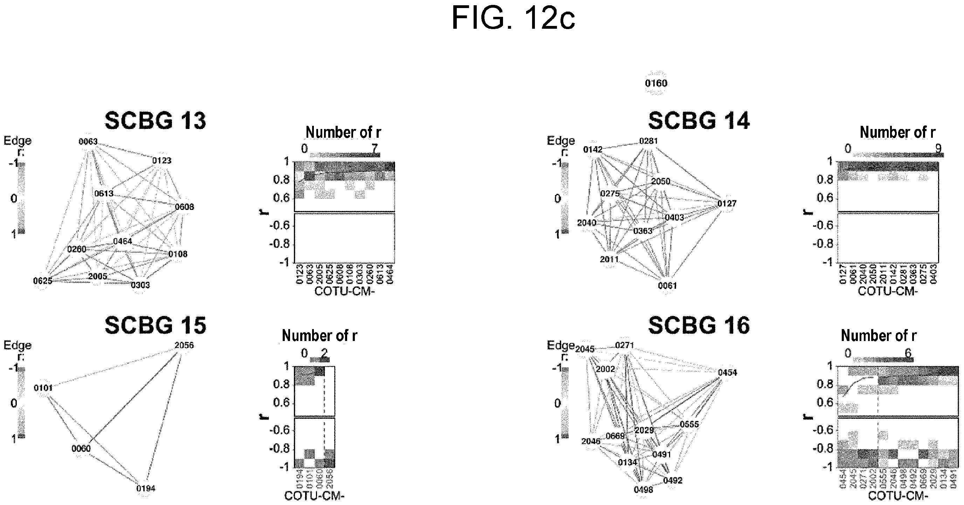

[Figure 12c] Same as above.

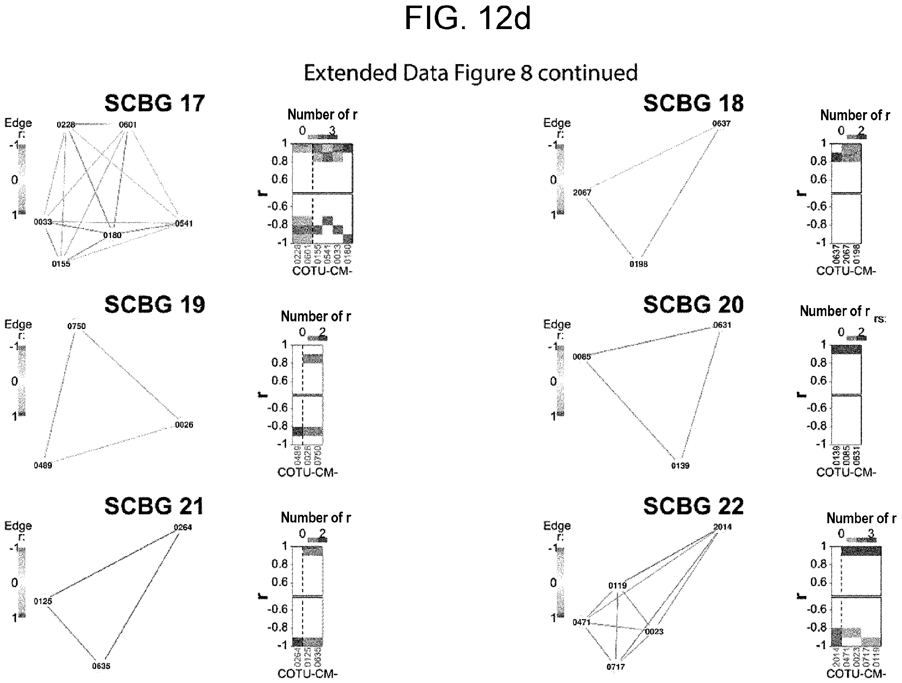

[Figure 12d] Same as above.

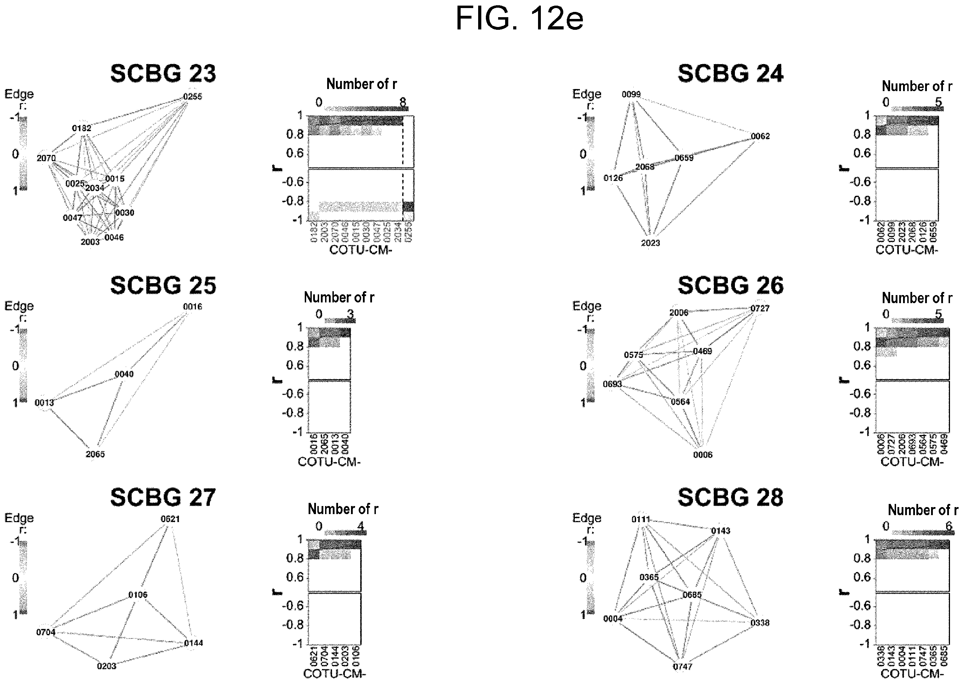

[Figure 12e] Same as above.

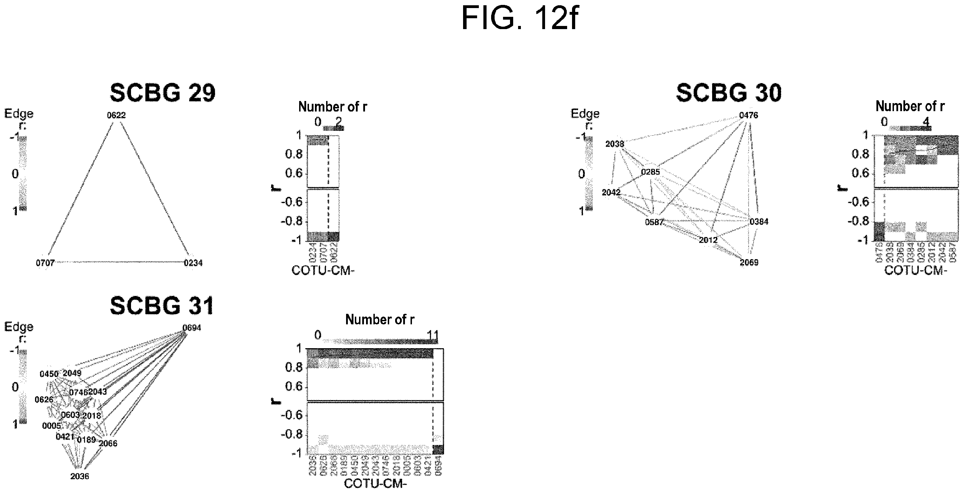

[Figure 12f] Same as above.

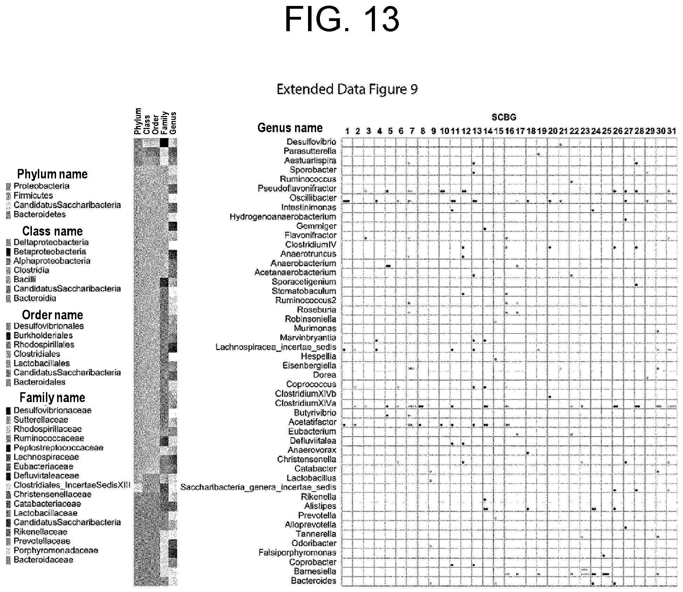

[Figure 13] Figure 13 shows classification of cOTUs in SCBGs from phylum to genus. Colors of dots, the same meanings as the colors of dots in Figure 11d. All SCBGs include a plurality of genera, and > 60% (19/31) of SCBGs include even a plurality of phyla, indicating SCBGs do not much correlate to taxa. Meanwhile, the present inventors found cOTUs from a plurality of SCBGs in all detected genera including $\geq$ 2 cOTUs, indicating that analyses at a level lower than the genus level, actually at a cOTU level, are important to understand the bacterial networks in the microbiota.

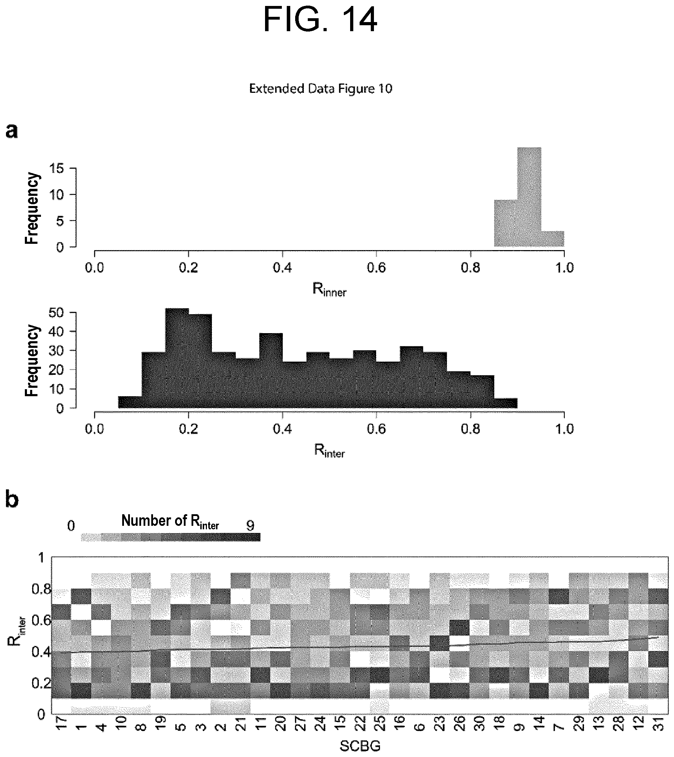

[Figure 14] Figure 14 shows correlations among SCBGs. Figure 14a shows distributions of $R_{inner}$ and $R_{inter}$. Row, distributions of $R_{inter}$ between given SCBGs and all other SCBGs. SCBGs were aligned according to distributions of the mean (blue line) along the horizontal axis.

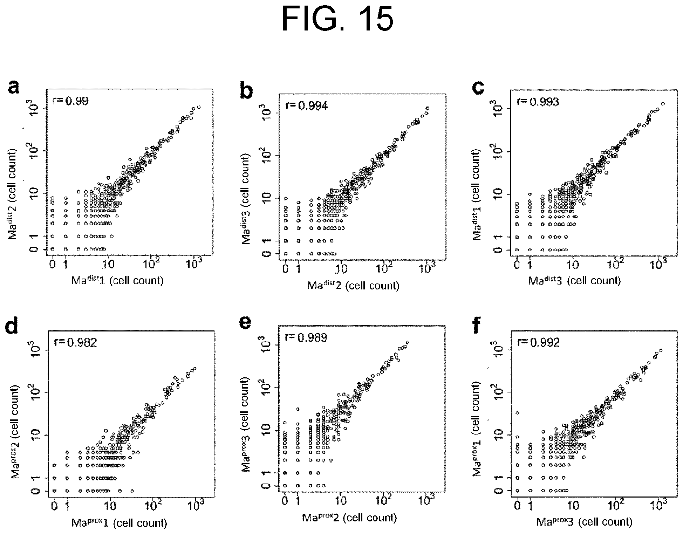

[Figure 15] Figure 15 shows comparisons between technical replicates for two samples $Ma^{dist}$ and $Ma^{prox}$ based on each cOTU count obtained by sequencing. Ma, a mouse; dist and prox, positions; 1, 2, and 3, technical replicates; r, Pearson coefficient.

[Figure 16a] Figure 16a shows comparisons of the cell concentrations for each cOTU between technical replicates and between samples. Three examples $Ma^{dist}1$-$Ma^{dist}3$, $Ma^{dist}3$-$Ma^{prox}2$, and $Mb^{dist}$-$Mc^{prox}$ (red asterisk symbols) are shown in Figure 16c. Ma, Mb, and Mc, mice; dist and prox, positions; 1, 2, and 3, technical replicates. Dot, a cOTU; magenta line, theoretical confidence interval (99.9%) of normalized sampling noises based on the Poisson distribution; light blue line, 2-fold change; blue dot, a cOTU showing a different concentration; insertion number for each, number of blue or grey dots.

[Figure 16b] Same as above.

[Figure 16c] Same as above.

[Figure 17] Figure 17 shows SCBG IDs. The upper diagram shows trees of the same dendrogram as in Figure 4d and have cOTU IDs. In the lower diagram: positions of red squares, positions of SCBGs in the heat map shown in Figure 4d; blue number, ID of each SCBG.

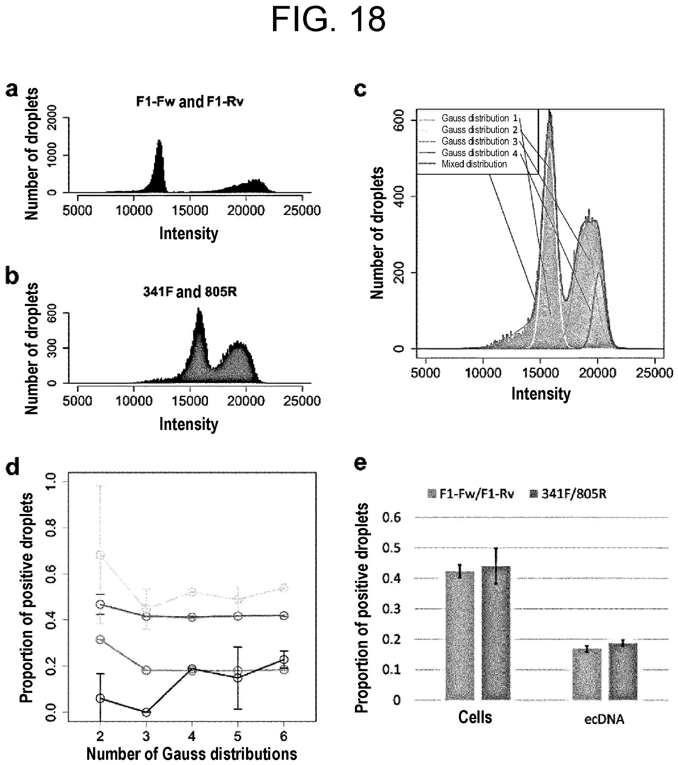

[Figure 18] Figure 18 shows comparisons of ddPCR measurements using primer sets F1-Fw/F1-Rv and 341F/805R for the same sample. Figure 18a shows a distribution of fluorescence intensity of droplets measured by ddPCR of the cecal cell sample using primers F1-Fw and F1-Rv. Figure 18b shows the same measurements as in Figure 18a, except for using different primers 341F and 805R. Figure 18c shows four different Gaussian distributions corresponding to the distributions of fluorescence intensity of Figure 18b and the sum of the mixed four Gaussian distributions. Figure 18d shows the proportion of positive droplets calculated based on fitting as a function of the number of fitted Gaussian distributions. Light blue, a cell sample subjected to amplification using primers F1-Fw and F1-Rv; blue, the same cell sample as used for light blue, except for using primers 341F and 805R; red, an extracellular sample amplified using primers F1-Fw and F1-Rv; black, the same extracellular sample as used for red, except for using primers 341F and 805R; error bar, standard deviation of three independent fittings (having a different random initial value). Figure 18e shows a comparison of ddPCR measurements between using primers F1-Fw/F1-Rv and using primers 341F/805R for the same sample; Proportion of positive droplets, calculated based on the fitting using 4 Gaussian distributions; Cells, the cellular sample; error bars, standard deviations, n=4d.

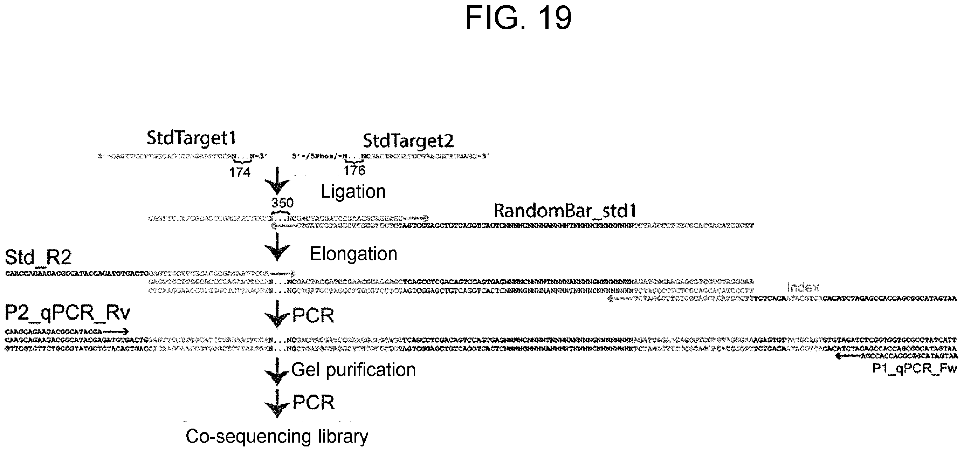

[Figure 19] Figure 19 is a schematic view including the sequence information for preparation of a spike-in control. StdTarget1, StdTarget2, RandomBar_std1, Std_R2, P2_qPCR_Rv, and P1_qPCR_Fw, synthesized DNA oligonucleotide; "5Phos" in StdTarget2, phosphorylation at the 5' end of the oligonucleotide; index, eight nucleotides; N in the sequence, A, C, G, or T.

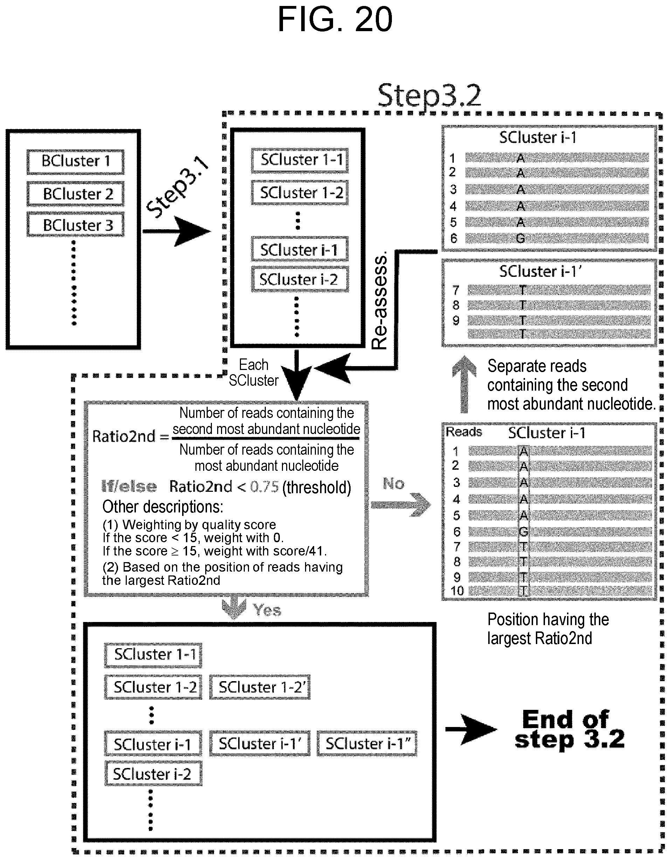

[Figure 20] Figure 20 is a logic diagram of Step 3.2.

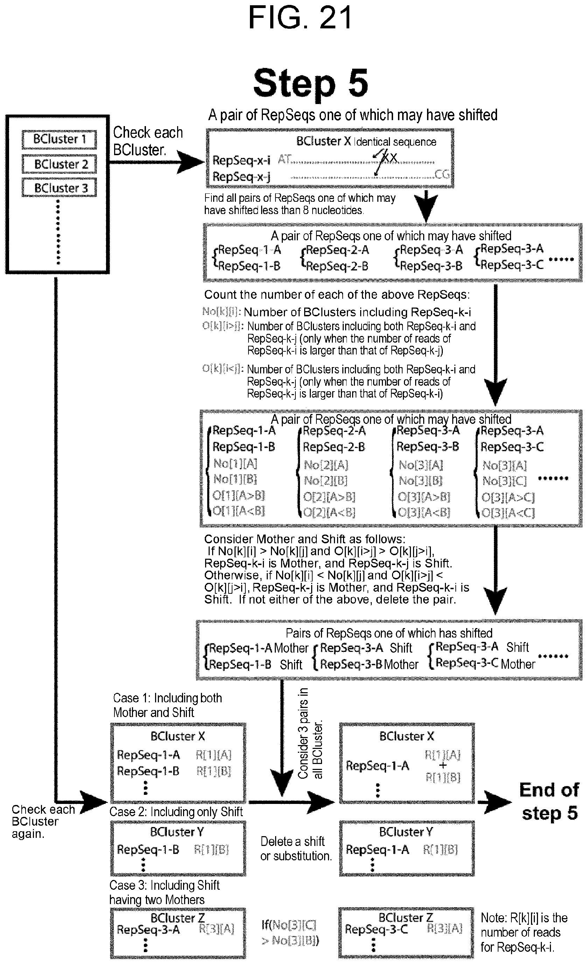

[Figure 21] Figure 21 is a logic diagram of Step 5.

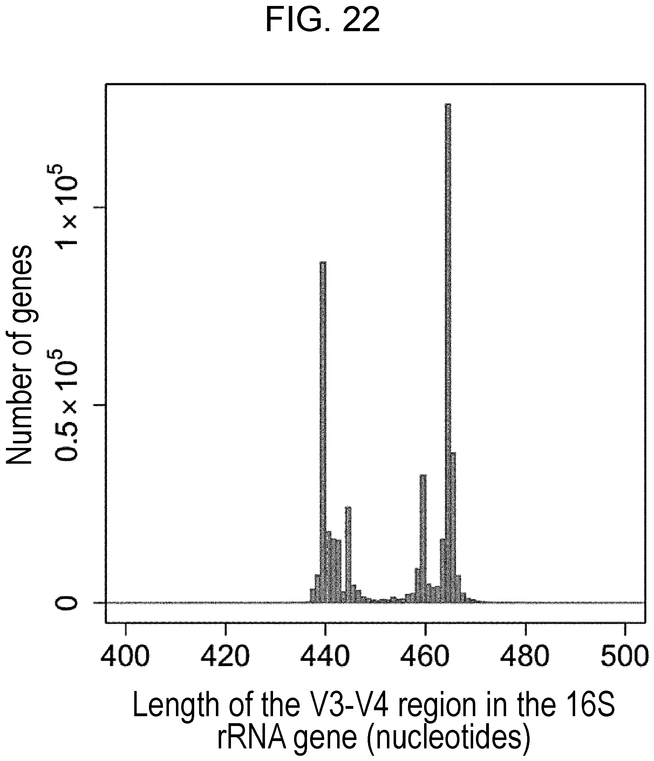

[Figure 22] Figure 22 shows distributions of lengths of the 16S rRNA genes in the V3-V4 region registered in the Silva database. Only the 16S rRNA genes matching the primers 341F and 805R were used (86.4% of all). Length, the number of nucleotides between the first nucleotide of the region matching 341R and the last nucleotide of the region matching 805R. A total of 99.94% of the full-lengths of the corresponding the 16S rRNA genes are in the range from 400 to 500 nucleotides.

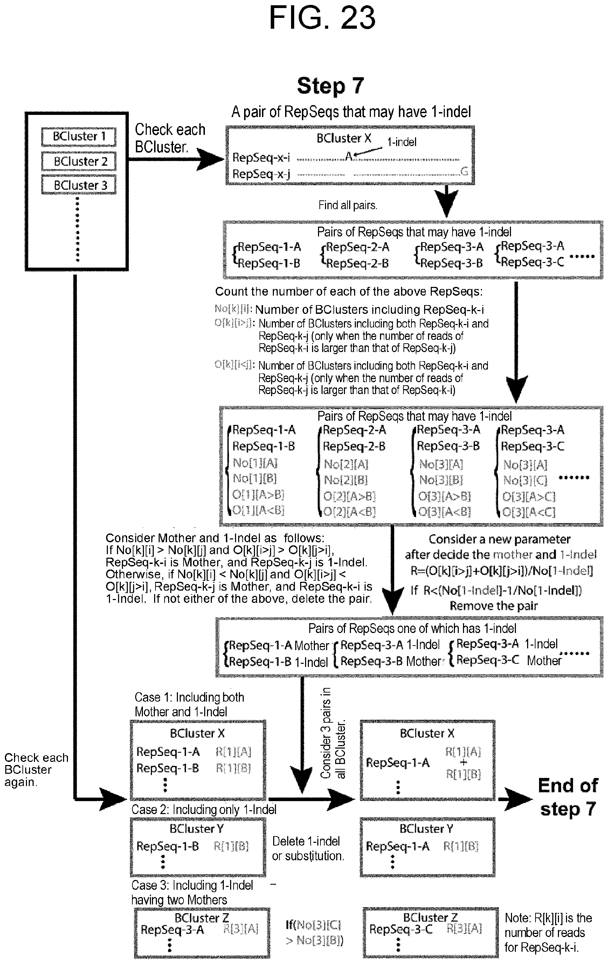

[Figure 23] Figure 23 is a logic diagram of Step 7.

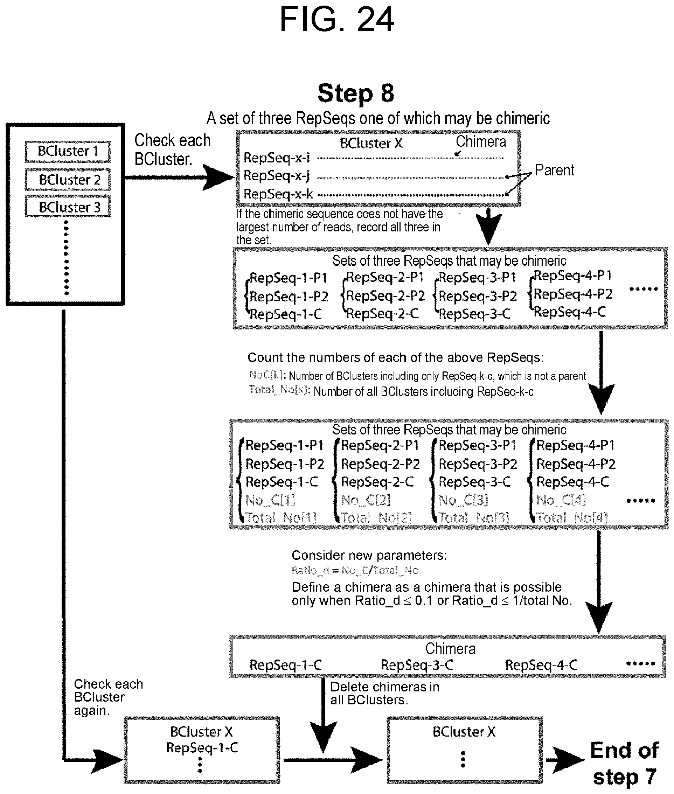

[Figure 24] Figure 24 is a logic diagram of Step 8.

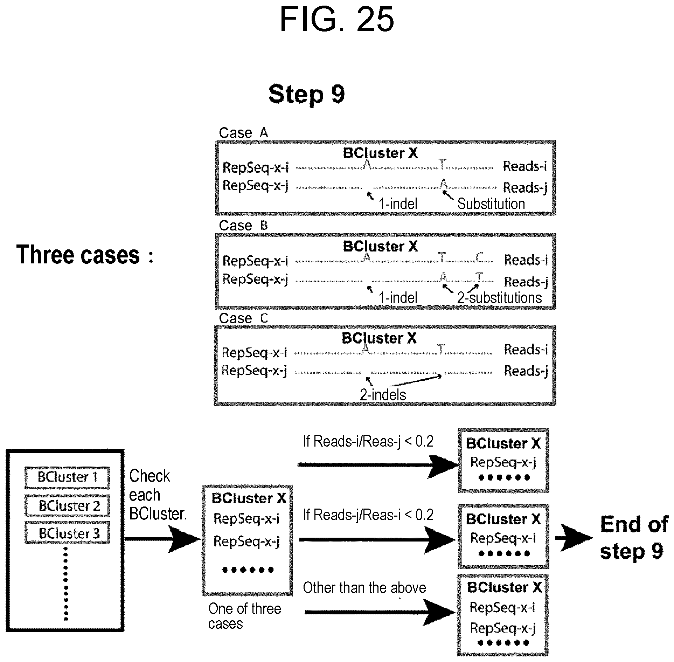

[Figure 25] Figure 25 is a logic diagram of Step 9.

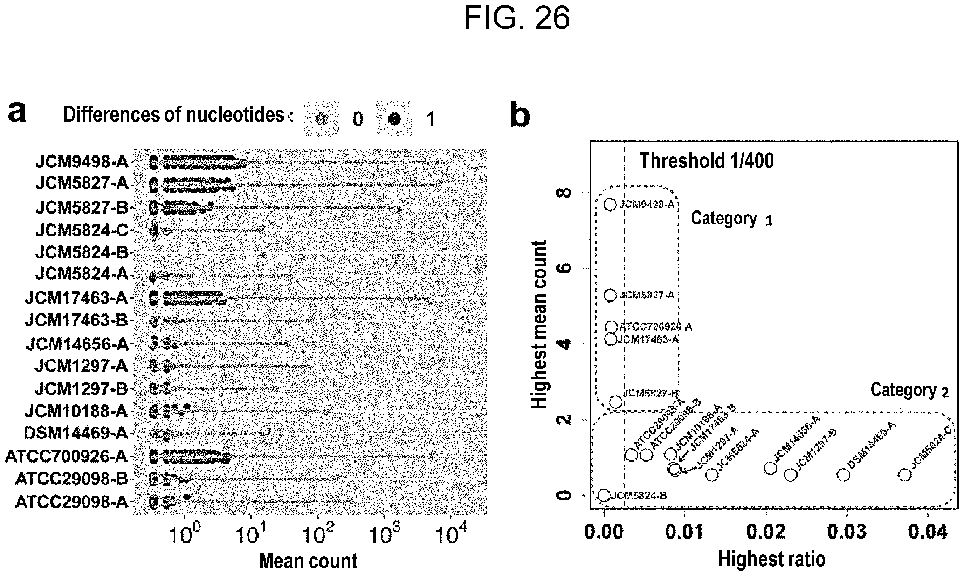

[Figure 26] Figure 26 shows characterization of RepSeq types with one-nucleotide difference by San sequencing. Figure 26a shows the mean counts of RepSeq types grouped based on San sequence (based on the Mock-a, Mock-b, and Mock-c data). Nucleotide differences, the number of nucleotide differences between RepSeq types and the closest San sequence (San sequence as ID group) in each group. Figure 26b shows the highest mean count of RepSeq types with one-nucleotide difference for the ratio of the mean count to the mean counts of matching RepSeq types in each group. Sky blue label, group ID.

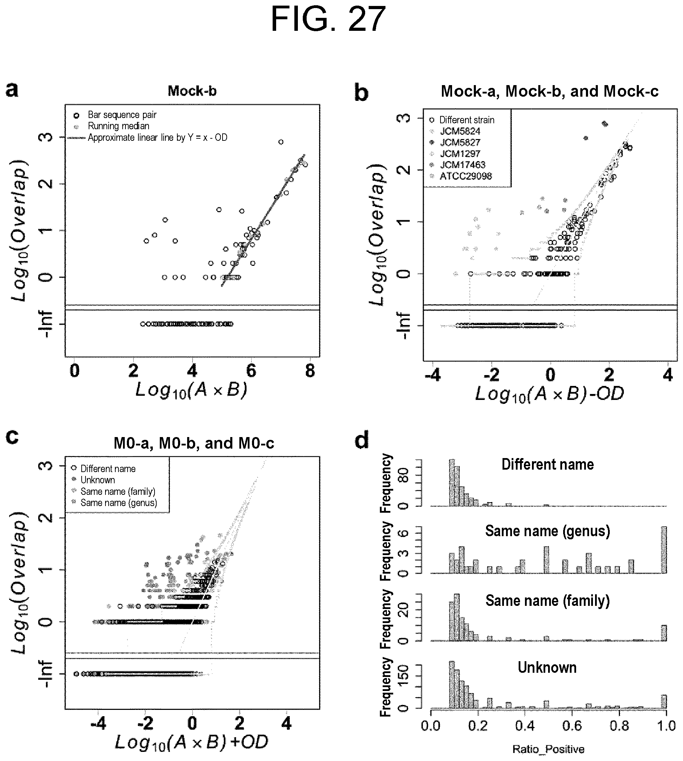

[Figure 27] Figure 27 shows clustering of Bar sequences into cOTUs. Figure 27a shows $\log_{10}$(Overlap) for $\log_{10}$(A $\times$ B) based on the Mock-b data. Dot, all possible Bar sequence pairs; overlap, A, and B, the numbers of BClusters including Bar sequences, only BS_A, and only BS_B, respectively (BS_A and BS_B are two Bar sequences in the pair). Blue broken line, 95% confidence interval of fitting. Figure 27b shows $\log_{10}$(Overlap) for $\log_{10}$(A $\times$ B) + OD based on the Mock-a, Mock-b, and Mock-c data. Dot, all possible Bar sequence pairs by three samplings (three dots for the identical Bar sequence pair); different strain, Bar sequence of the pair matching the San sequence identified from a different strain; JCM/ATCC number, Bar sequence of the pair matching the San sequence identified from a predetermined strain; green line, a one-sided 99.9% confidence interval of distributions of $\log_{10}$ (Overlap) obtained by simulation; yellow line, x = y; OD, $\log_{10}$(Droplets/$\mu$) estimated by fitting in Figure 27a. It should be noted that Bar sequences contaminated with foreign substances were excluded from this plot (see Step 14). In Figure 27c, data were based on the M0-a, M0-b, and M0-c data as in Figure 27b. The name of each Bar sequence is based on the Silva database. Different name, different mapped names for Bar sequences of one pair; identical name (family), the same mapped name for Bar sequences of one pair. Only the names of the family or higher order taxa have been determined. Identical name (genus), the same mapped name for Bar sequences of one pair. Only the names of the genus or higher order taxa have been determined. Unknown, only one or neither of Bar sequences of one pair is registered in the database. Figure 27d shows distributions of Ratio_Positive (see Step 12). The results of specimens Ma$^{dist}$1-3, Ma$^{prox}$1-3, Mb$^{dist}$, Mb$^{prox}$, Mc$^{dist}$, and Mc$^{prox}$.

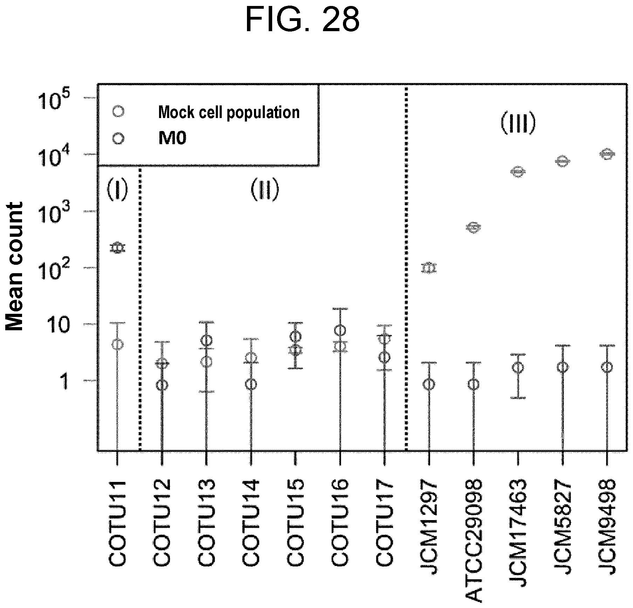

[Figure 28] Figure 28 shows comparisons of the mean counts (of three repeated measurements) of detected cOTUs between the mock cell population and M0. cOTUs not detected in M0 are not shown. JCM/ATCC, cOTUs matching the predetermined strain; cOTU <number>, cOTUs not matching any designed strain; I, II, and III, three categories (see Step 14).

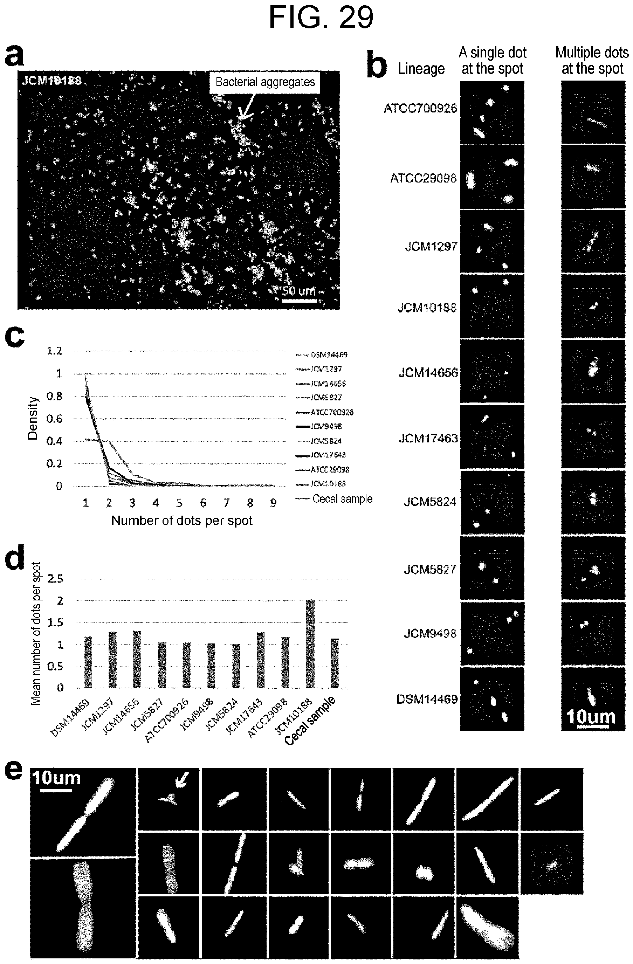

[Figure 29] Figure 29 shows breaking of bacterial agglutinates. Figure 29a shows JCM10188 bacterial aggregates before vortexing. Figure 29b shows examples of spots including one dot or a plurality of dots after vortexing. Figure 29c shows distributions of the number of dots per spot for each bacterial strain and the cecal sample after vortexing. Figure 29d shows the mean number of dots per spot for each bacterial strain and the cecal sample after vortexing. Figure 29f shows all spots including a plurality of dots among a total of 208 identified spots in the cecal sample. Yellow arrow, only this example seems to have two different shape dots in the identical spot.

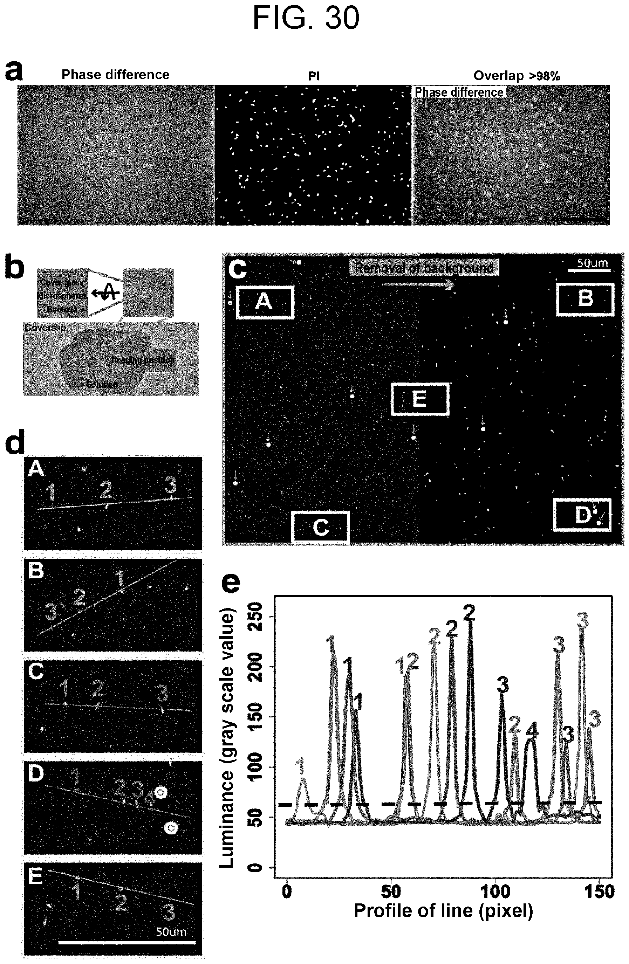

[Figure 30] Figure 30 shows measurements of bacteria by microscopic imaging. Figure 30a shows a comparison of phase difference light and fluorescent light (PI) of *Escherichia coli* (DH5$\alpha$) in the visual field. Figure 30b shows an overview of the number of bacteria obtained by microscopic imaging. Figure 30c shows the ATCC700926 strain illuminated with fluorescence and phase difference and stained with PI. The threshold for removing the background is shown in Figure 30e. Red arrow, microspheres also observed by phase-difference illumination. Figure 30d shows enlarged images of A to E in Figure 30c. Color line, line profile used for measurement of brightness using ImageJ; number, a number for a bright spot (i.e., bacterium) shown in Figure 30e. Figure 30e shows brightness (gray values) measured along the line profile in Figure 30d. Broken line, threshold for removing the background (see Figure 30c).

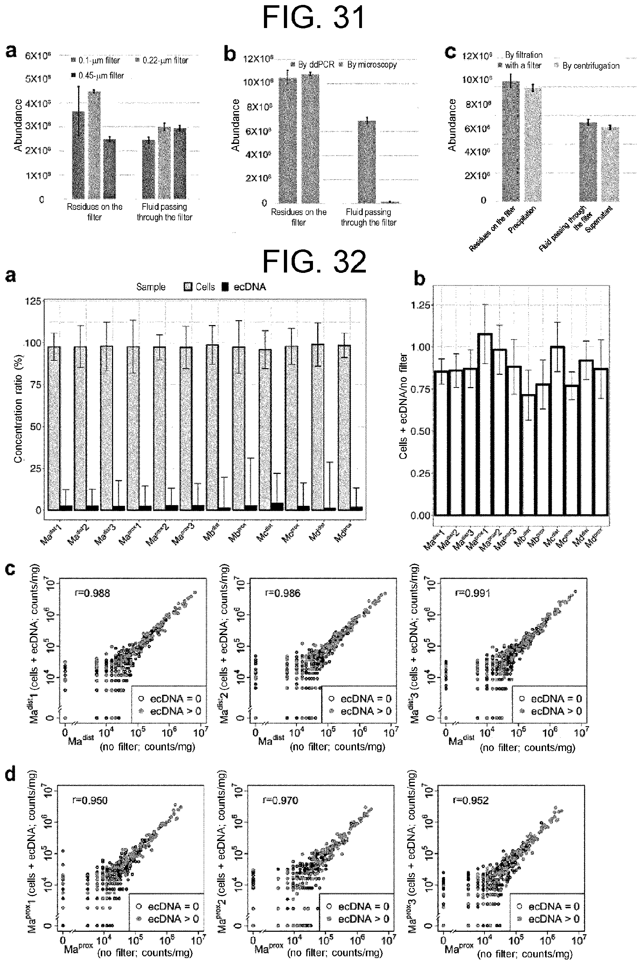

[Figure 31] Figure 31 shows controlled separations of ecDNA and cells. Figure 31a shows a comparison of different filtrates obtained by using Ultrafree (trademark)-MC centrifugal filters with a pore size of 0.1 $\mu$m, 0.22 $\mu$m, or 0.45 $\mu$m. Residues on the filter, samples that remained on the filter membrane; fluid that passed through the filter, liquid that passed through the filter membrane; abundance, total copy number measured by ddPCR. Figure 31b shows a comparison of the abundances of cells and ecDNA after filtration between measured by ddPCR and microscopic imaging. Abundance, the total number of copies measured by ddPCR or the total number of bright spots measured by fluorescence imaging. Figure 31c shows a comparison of separated ecDNA and cells using filtration and centrifugation. Abundance, same as in Figure 31a.

[Figure 32] Figure 32 shows extracellular DNA and cells in the cecal sample. Figure 32a shows the ratio of the cell and ecDNA concentrations normalized by the total concentration. The total (100%) was defined as the sum of the cell and ecDNA concentrations. Error bar, propagation error calculated from the standard deviation of the cell and ecDNA concentrations based on calculation (n = 5). Figure 32b shows the ratio of the total concentration of isolated cells and ecDNA to the total concentration of unfiltered samples. Error bar, propagation standard deviation (n = 5). c and d, comparisons of the total cell and ecDNA concentrations and the unfiltered sample concentrations of Ma$^{dist}$ and Ma$^{prox}$ at each cOTU concentration. Red dots are cOTUs in which ecDNA was detected, and black dots are cOTUs in which ecDNA was not detected. The results were compared for each of filtrations repeated three times.

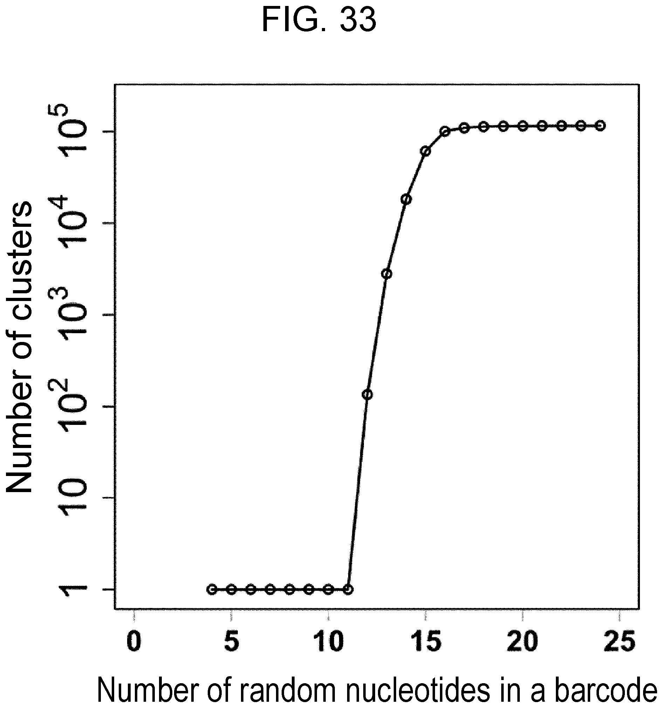

[Figure 33] Figure 33 shows dependence of the number of clusters (unique barcodes) on the random number of nucleotides designed for barcodes. The results of Sequence Run 1 are shown.

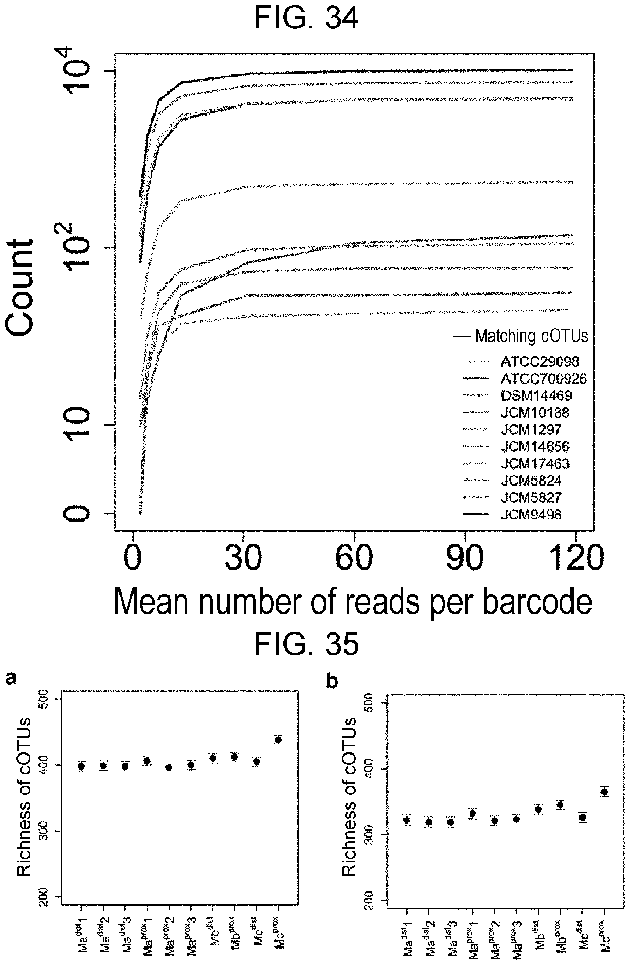

[Figure 34] Figure 34 shows dependence of the number of cOTUs allowed to match 10 lines of San sequences for the mean value of reads per unique barcode. The data of Mock-b are shown.

[Figure 35] Figure 35 shows the richness of cOTUs. In Figure 35a, 6,075 cells were subsampled for each sample. In Figure 35b, 3,000 cells were subsampled. Ma, Mb, and Mc, mice; dist and prox, positions; 1a, 2, and 3, technical replicates; error bar, standard error.

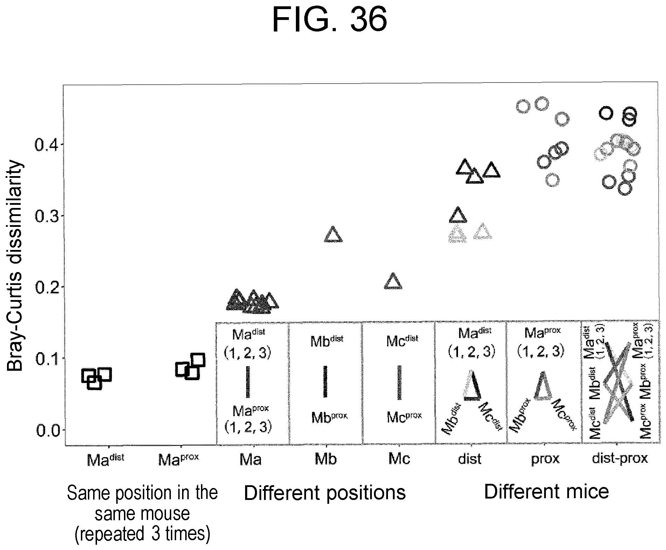

[Figure 36] Figure 36 shows Bray-Curtis dissimilarity of the microbiota between the samples measured using the proportional abundance of cOTUs. Ma, Mb, and Mc, mice; dist and prox, positions; 1, 2, and 3, technical replicates.

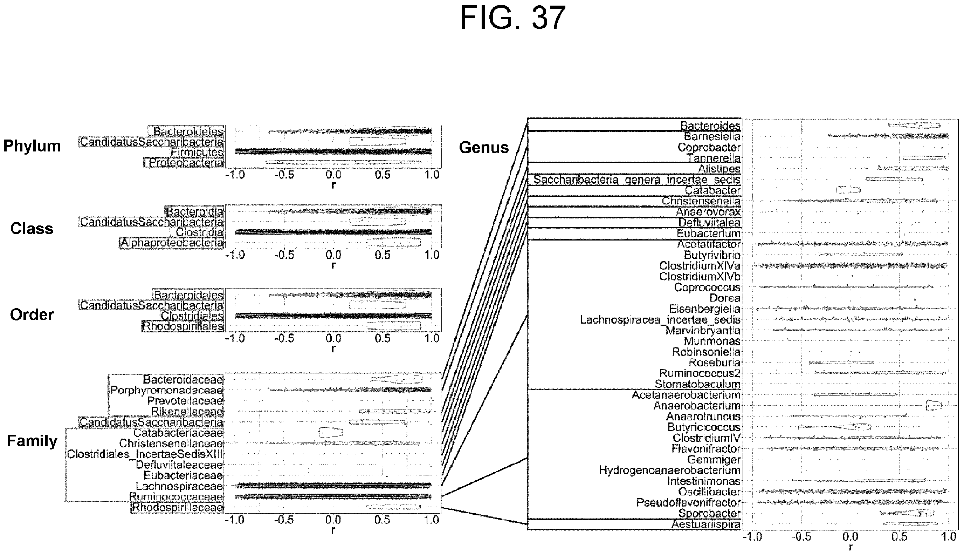

[Figure 37] Figure 37 shows a distribution (violin plot) of r of cOTU pairs (dots) from the same taxon. The color box surrounding the names at levels from phylum to family represents the ownership thereof.

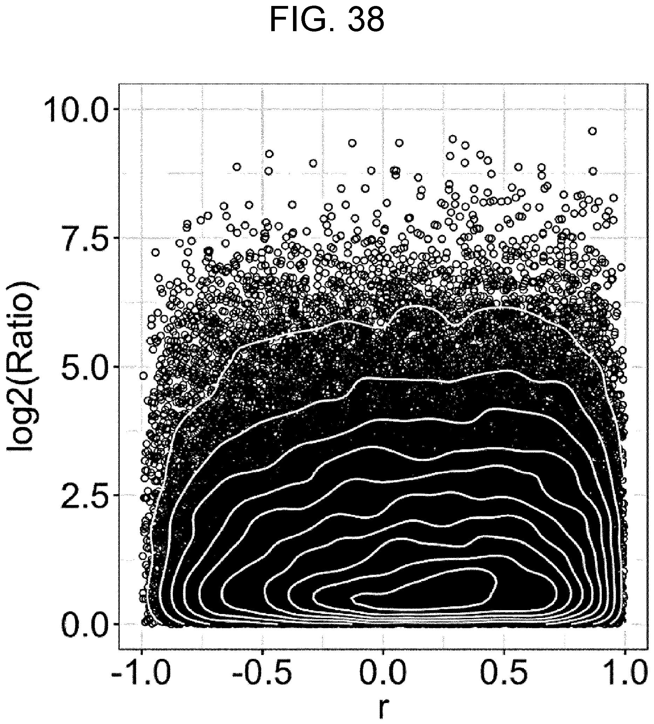

[Figure 38] Figure 38 shows dependence of the ratio of the mean concentrations between cOTUs on r. Dot, a cOTU pair; ratio, a value obtained by dividing a higher concentration by a lower concentration in the pair; yellow line, quantitative contour line (10% interval).

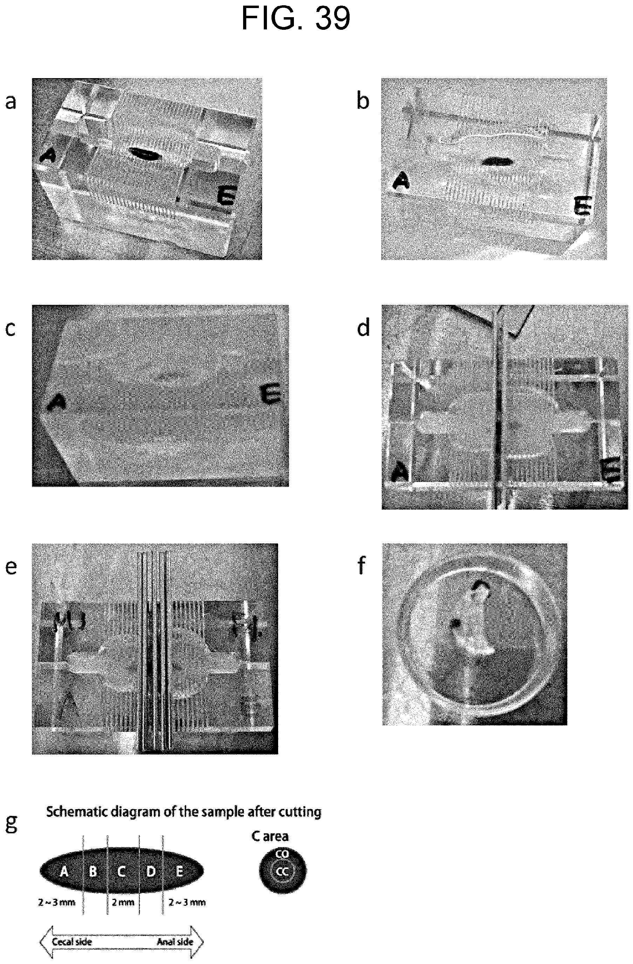

[Figure 39] Figure 39 shows how the murine large intestine sample was subdivided using a brain slicer. Panels a to f show how a murine large intestine sample was placed on a brain slicer (Panel a), embedded (Panel b), frozen (Panel c), and sliced (Panels d and e) to obtain a sliced (subdivided) sample (Panel f). Panel g illustrates division into regions from the cecum (cecal side) towards the anus (anal side). Area C was further subdivided into the central portion and the peripheral portion (Panel g).

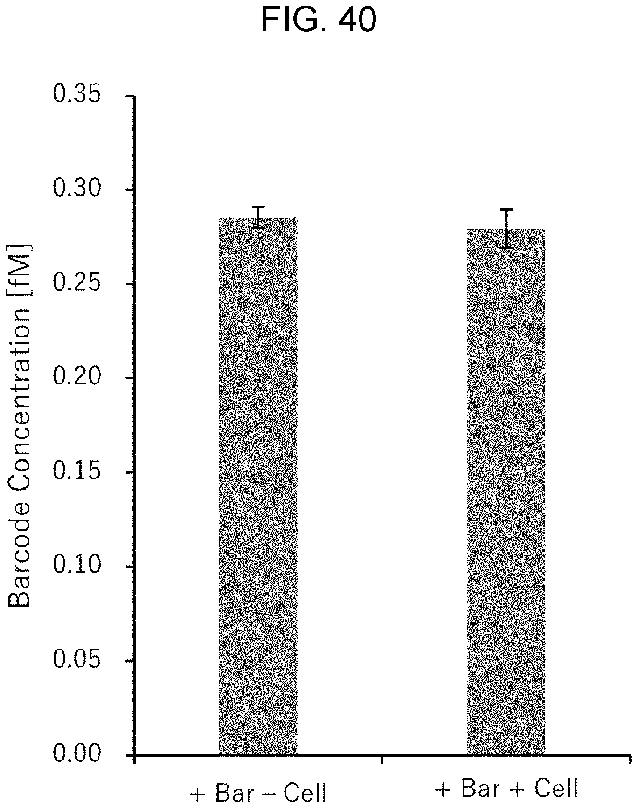

[Figure 40] Figure 40 shows concentrations of barcode sequences in each sample. "-Cell" indicates the results in the absence of cells, and "+Cell" indicates the results in the presence of cells. The error bar represents a standard deviation (n = 4).

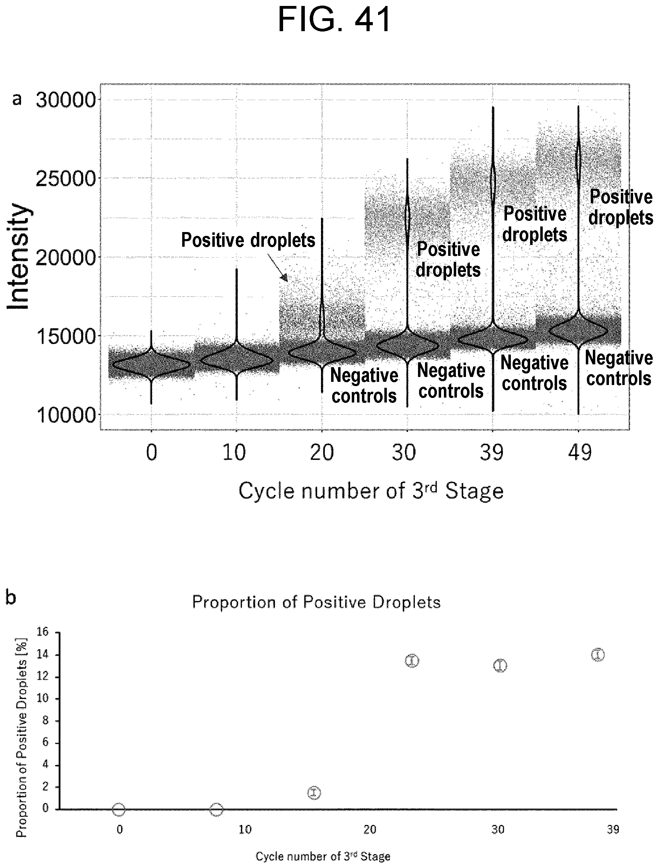

[Figure 41] Figure 41 shows the relationships of the number of cycles in the third stage of ddPCR with the fluorescence intensity of droplets (Panel a) and with the proportion of positive droplets in all droplets (Panel b). The error bar represents a standard deviation (n = 4) .

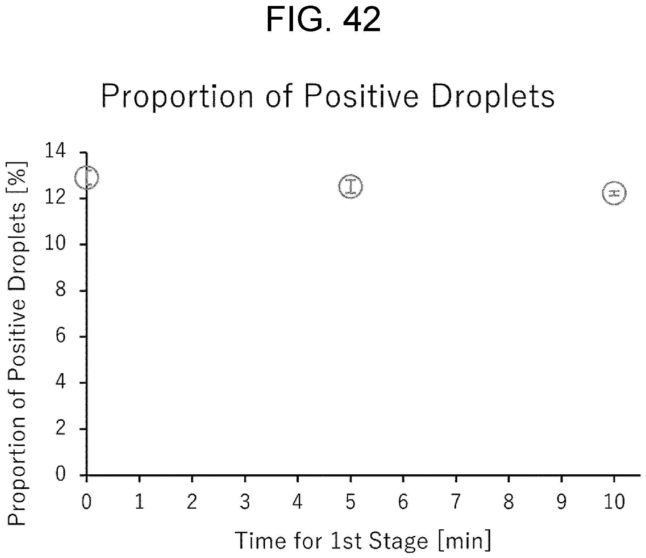

[Figure 42] Figure 42 shows the relationship between the reaction time at the third stage of ddPCR and the proportion of positive droplets in all droplets. The error bar represents a standard deviation (n = 4).

Description of Embodiments

In the present specification, the term "subject" refers to a living organism, that is, an animal or a plant. Examples of subjects can include vertebrates: for example, mammals, fish, birds, amphibians, and reptiles, such as, for example, primates such as humans, chimpanzees, gorillas, orangutans, monkeys, marmosets, and bonobos and four-legged animals such as pigs, rats, mice, cows, sheep, goats, horses, cats, and dogs (e.g., carnivores, cloven-hoofed animals, odd-toed ungulates, and rodents).

In the present specification, the term "cell" refers to a cell of a living organism and can be a cell of a bacterium, protozoa, chromista, animal, plant, and fungus. In the present specification, the term "singulated cells" means cells that exist in a form of being separated into individual cells. Therefore, a solution containing singulated cells means a solution containing one or more cells each of which exists separately. The solution containing singulated cells is preferably a solution in which all or most contained cells exist in a form of being separated into individual cells, but may contain a cell aggregate comprising two or more adhered cells as long as singulated cells are contained.

In the present specification, the term "cell population" is a composition comprising a plurality of cells. The cell population generally comprises cells of a plurality of types, and each type can include a plurality of cells. The form of the composition can be a liquid or a solid.

In the present specification, the term "microbiota" means a population of microorganisms. Naturally, various microbiotas exist. For example, microbiotas exist in soil, water (ocean, river, swamp, pond), air, and the epidermis, body hair, oral cavity, nasal cavity, gastrointestinal tract (e.g., the esophagus, stomach, small intestine, large intestine, cecum), and reproductive organ of animals; and outer skin and root of plants. The microbiota in an animal reflects or affects the health condition of the animal. A microbiota can comprise 10 or more types, 20 or more types, 30 or more types, 40 or more types, 50 or more types, 60 or more types, 70 or more types, 80 or more types, 90 or more types, or 100 or more types of microorganisms. A microbiota can include unknown microorganisms. Unknown microorganisms can be 10% or more, 20% or more, 30% or more, or 40% or more of the microorganism types included in a microbiota.

In the present specification, the term "cellular barcode" means a nucleic acid having a unique nucleotide sequence allocated for each cell. Each cell can be linked to a cellular barcode having a different nucleotide sequence (i.e., a nucleotide sequence unique to the cell). Therefore, the number of cellular barcodes can represent the number of cells. Thus, the number of cells, which has been conventionally measured in quantitative manner, can be measured by converting numbers of nucleotide sequences to qualitatively assessable numbers. A sufficient number of different cellular barcodes can be prepared for the total number of cells present.

In the present specification, the term "isolation" means separating a target substance from others. Isolation can include concentration or purification of the target substance after isolation.

In the present specification, the "amplification product" means a nucleic acid obtained by amplification of a gene (e.g., polymerase chain reaction (PCR)). In PCR, two primers are designed to flank a DNA site to be amplified, and the portion flanked by the two primers is amplified by allowing to react with DNA polymerase under a predetermined condition. A primer can be a nucleic acid in the form of a single chain having a sequence that hybridizes with the DNA site to be amplified, but an additional nucleotide sequence (e.g., an adapter, an index sequence unique to the sample, a restriction enzyme recognition site) may be linked to the nucleic acid at the 5' end thereof.

In the present specification, the term "paralog" means two genes arising from gene multiplication on the genome. In the present specification, the term "ortholog" means genes that exist in different organisms and have a homologous function.

According to the present invention, a method for treating a cell population, the method comprising

(A) obtaining a droplet population from a cell dispersion comprising an isolated cell population, the droplet population comprising aqueous droplets, at least some of which each comprise one cell and one-molecule cellular barcode

is provided.

According to the present invention,
a droplet population comprising aqueous droplets, at least some of which each comprise one cell and one-molecule cellular barcode is provided. In this embodiment, cells can be cells constituting an isolated cell population (e.g., a microbiota).

The cell dispersion of the above (A) can be obtained by dispersing cells included in the isolated cell population in an aqueous solution. Cells can be dispersed in a solution utilizing a shear stress of water caused by water flow, for example, by shaking, pipetting, or the like. The term "to disperse" means to disassemble a cell aggregate composed of a plurality of cells in an aqueous solution in order to obtain a plurality of single cells and, preferably, to suspend single cells in the aqueous solution. The method of the present invention can comprise dispersing cells included in an isolated cell population in an aqueous solution.

In one embodiment, a cell population can be a microbiota. In this embodiment, a natural microbiota can be preferably used as the microbiota. Examples of microbiotas include soil, water (ocean, rivers, swamps, pond), air, and the epidermis, body hair, oral cavity, nasal cavity, gastrointestinal tract (e.g., esophagus, stomach, small intestine, large intestine, cecum), and reproductive organs of animals. Microbiotas present in the outer skin and roots of plants can also be used. For example, a microbiota in the gastrointestinal tract can be used. For example, such a microbiota can be a microbiota in the oral cavity, a microbiota in the esophagus, a microbiota in the stomach, a microbiota in the duodenum, a microbiota in the small intestine (e.g., the jejunum or the ileum), a microbiota in the cecum, or a microbiotas in the large intestine (e.g., the ascending colon, transverse colon, descending colon, sigmoid colon, rectum). A natural microbiota is preferably analyzed without being cultured, but analyzing after culturing the natural microbiota can be acceptable. In one preferred embodiment, a microbiota includes unknown microorganisms. In one preferable subject, the types of unknown microorganisms can account for 10% or more, 20% or more, 30% or more, or 40% or more of microorganism types included in a microbiota. In one embodiment, a cell population can include extracellular DNA. Extracellular DNA can include predetermined genes. Extracellular DNA may be removed before the cell population is treated. Extracellular DNA can be removed by filtration or centrifugation as described later. Extracellular DNA may be included in a cell population to be treated.

A cell population is isolated by obtaining a cell population. Isolation of a cell population may further comprise separating the obtained cell population from one or more components that are not cells. The cell population can be separated from one or more components that are not cells by filtration or centrifugation. The filtration can be performed by using, for example, a filter having a pore size of submicrometers (e.g., 0.22 $\mu$m), and the cell population can be collected from residues on the filter.

In the present invention, cells included in an isolated cell population can be dispersed in an aqueous solution before droplets are prepared. Here, the term "to disperse" means to allow individual cells to exist separately. Dispersion can be achieved by disintegrating a cell aggregate by pipetting without destroying cells. Aqueous solutions are not particularly limited as long as cells are not destroyed, and water, physiological saline, and the like can be used. The isolated cell population can be dispersed in pure water, physiological saline, a reaction mixture for gene amplification, or the like.

In one embodiment, droplets can be prepared in oil. Therefore, in this embodiment, the droplet population obtained in (A) comprises aqueous droplets (water droplets) in oil. In other words, the droplet population obtained in (A) can be water-in-oil droplet type particles (a population of aqueous droplets dispersed in oil) .

The particle size of the above-mentioned aqueous droplets can range from 10 $\mu$m to 100 $\mu$m as the lower limit and from 50 $\mu$m to 1,000 $\mu$m as the upper limit. The particle size of aqueous droplets can range, for example, from 10 $\mu$m to 1,000 $\mu$m, for example, 20 $\mu$m to 900 $\mu$m, 30 $\mu$m to 800 $\mu$m, 40 $\mu$m to 700 $\mu$m, 50 $\mu$m to 600 $\mu$m, 50 $\mu$m to 500 $\mu$m, 50 $\mu$m to 400 $\mu$m, 50 $\mu$m to 300$\mu$m, 50 $\mu$m to 200 $\mu$m, or 50 $\mu$m to 150 $\mu$m, or, for example, approximately 100 $\mu$m. Such a droplet population can be suitably prepared by those skilled in the art using, for example, a microfluid device. Such a droplet population can also be prepared using a commercially available droplet generator. As the commercially available droplet generator, for example, QX200 Droplet Generator of BIO-RAD Laboratories, Inc. can be used.

According to the method for treating a cell population of the present invention, a droplet population comprising aqueous droplets which include aqueous droplets comprising one cell and one molecule of a cellular barcode (e.g., DNA) having one type of a nucleotide sequence unique to the cell can be obtained. More specifically, in the method for treating a cell population of the present invention, for example, a droplet population comprising aqueous droplets which comprise one cell and a single type of a cellular barcode unique to each cell can be obtained by mixing an aqueous solution containing a plurality of dispersed cells and an aqueous solution containing cellular barcodes having a nucleotide sequence different for each one molecule in oil.

According to the method for treating a cell population of the present invention, another cell is included in aqueous droplets comprising a cellular barcode having another one type of a nucleotide sequence unique to the cell. The cell can be included in 50% or less, 40% or less, 35% or less, 30% or less, 25% or less, or 20% or less (e.g., 20%) of all droplets. The probability of a plurality of cells being contained in one droplet can be reduced by doing this. When it is assumed that cells are contained in 20% of droplets, in theory, the number of cells contained in, for example, 90% or more of cell-containing droplets is 1. Furthermore, cellular barcodes can also be contained in 50% or less, 40% or less, 35% or less, 30% or less, 25% or less, or 20% or less (e.g., 20%) of all droplets. By doing this, the number of cellular barcodes contained in, for example, 90% or more of cellular barcode-containing droplets can be 1. By doing this, droplets containing one cell and one molecule of a cellular barcode can be obtained, and the droplets can account for 1% to 10%, 2% to 6%, 3% to 5%, or, for example, approximately 4% of all droplets. In one embodiment, the proportion of cell-containing droplets to all droplets can be made 30% or less (preferably, approximately 20%), and the proportion of cellular barcode-containing droplets to all droplets can be made 30% or less (preferably, approximately 20%). Thus, by reducing the proportion of droplets containing a cell and a cellular barcode to all droplets, the possibility that two or more cells are mixed into one droplet and the possibility that two or more molecules of cellular barcodes are mixed into one droplet can be reduced or eliminated. Of note, the existence of droplets containing only one or neither of a cell and a cellular barcode does not affect the subsequent steps after sequencing a product of linking of a predetermined gene and a cellular barcode in a cell.

In the obtained droplet population, the proportion of droplets containing two or more cells and one cellular barcode can be, for example, 0.5% or less, 0.4% or less, or 0.3% or less and can be, for example, 0.3% to 0.5%. In the obtained droplet population, the proportion of droplets containing one cell and two or more cellular barcodes can be, for example, 0.5% or less, 0.4% or less, or 0.3% or less and can be, for example, 0.3% to 0.5%. In the obtained droplet population, the proportion of droplets containing two or more cells and two or more cellular barcode can be, for example, 0.05% or less, 0.04% or less, or 0.03% or less and can be, for example, 0.03% to 0.05%. Here, a smaller number of droplets containing two or more cells or cellular barcodes are more preferred, but occurrence of such droplets is acceptable.

Aqueous droplets may further contain primers and reagents for gene amplification in addition to one cell and one molecule of a cellular barcode. Since cells are destroyed during the gene amplification reaction, the reagents do not need to include a surfactant. Additionally, aqueous droplets can be an aqueous solution suitable for a gene amplification reaction (e.g., a gene amplification reaction solution).

Any oils can be used as long as they are stable and inactive under an environment of a gene amplification reaction (60°C to 100°C). Examples of such oils include mineral oil (e.g., light oil), silicone oil, fluoride oil, and other commercially available oils, and combinations thereof, but are not limited to these examples.

Under such a condition, a droplet population comprising aqueous droplets, at least some of which each comprise one of the obtained cells and one molecule of a cellular barcode can be obtained from an aqueous solution containing cells, cellular barcodes, primers, and reagents for gene amplification. More specifically, a gene amplification reaction mixture containing cells, cellular barcodes, primers, and reagents for gene amplification can be prepared to obtain a droplet population from this solution as described above.

According to the present invention,
a method for determining (or analyzing) a gene sequence included in a cell population, comprising

(A) obtaining a droplet population from a cell dispersion comprising an isolated cell population, the droplet population comprising aqueous droplets, at least some of which each comprise one cell and one-molecule cellular barcode, and

(B) obtaining an amplification product of the cellular barcode and an amplification product of a predetermined gene in each obtained droplet, further obtaining a linked product of nucleotide sequences of the cellular barcode and all or some of the predetermined genes, and sequencing the obtained linked product to determine the nucleotide sequences of the predetermined genes and the nucleotide sequences of the cellular barcodes

(hereinafter referred to as the sequencing method of the present invention) is also provided.

When droplets are formed, components required for gene amplification can be introduced into each droplet beforehand by mixing necessary components for PCR, excluding a template, such as a primer set for amplification of the cellular barcode and amplification of the predetermined gene in the cell, dNTPs, and thermostable DNA polymerase in a solution (e.g., a cellular barcode solution). Then, a liquid containing a droplet population is transferred into a tube for PCR, and a DNA amplification reaction can be induced in each droplet by PCR. The amplification product of the predetermined gene in the cell and the amplification product of the cellular barcode can be obtained in each droplet by gene amplification in each droplet. Amplification can include amplification cycles of, for example, 25 cycles, preferably, 30 or more cycles. Subsequently, the amplification product of the predetermined gene in the cell and the amplification product of the cellular barcode can be linked in each droplet (for example, see Figure 5). Linking can be performed in a step of an amplification reaction (e.g., a PCR reaction) by, for example, designing one of primers for the cellular barcode and one of primers for the predetermined gene so that they have complementary sequence portions that are hybridizable with each other (for example, see SEQ ID NOS: 4 and 5 in Figure 5). By doing this, one type of a cellular barcode can be attached to each molecule of the amplification product of the predetermined gene derived from one cell.

A cellular barcode has a nucleotide sequence unique to the cell in the center thereof (however, nucleotide sequences with a specific position can be designed to be the same sequences among the sequences) and can have a nucleotide sequence at either end to hybridize with an amplification primer. In one embodiment, the nucleotide sequence to hybridize with an amplification primer can be a common sequence among cellular barcodes. The primers for amplification of the cellular barcode can have nucleotide sequences hybridizable with an adapter sequence for sequencing and one end of the cellular barcode under a gene amplification environment. A primer for amplification of the cellular barcode can further have an index sequence for identifying the type of a sample. The other primer for amplification of the cellular barcode can have a linker sequence for linking to the predetermined gene and a nucleotide sequence hybridizable with the other end of the cellular barcode under a gene amplification environment.

A primer for amplification of the predetermined gene can have a nucleotide sequence to hybridize with a linker sequence contained in a primer for amplification of the cellular barcode and a nucleotide sequence to hybridize with a nucleotide sequence of the predetermined gene at the amplification site under a gene amplification environment. The other primer for amplification of the predetermined gene can contain a nucleotide sequence to hybridize with a nucleotide sequence of the predetermined gene at the amplification site under a gene amplification environment and a sequencing adapter sequence. The other primer for amplification of the predetermined gene can further have an index sequence unique to the sample to identify the type of the sample.

The amplification product of the cellular barcode and the amplification product of the predetermined gene have the same linker sequence. Therefore, an amplification product of a linked product of the amplification product of the cellular barcode and the amplification product of the predetermined gene can be obtained during the gene amplification.

A sequencing adapter sequence can contain a sequence for bridging PCR before sequencing at both ends. The sequencing adapter sequence can contain a site of binding to a sequencing primer. The sequencing adapter sequence can contain an index sequence unique to the sample to identify the type of the sample. The bridging PCR is a technique to hybridize DNA subjected to sequencing which has a sequence hybridizable with each of two types of solid-phase oligo DNA at either end and amplifying the DNA on a solid-phase surface by PCR in this state.

Therefore, the present invention also provides a droplet population including aqueous droplets which comprise the amplification product of a predetermined gene derived from one cell, wherein one type of a cellular barcode unique to the cell is linked to each molecule of the predetermined gene. In this droplet population, each droplet comprises the predetermined gene derived from one different cell and one type of a cellular barcode unique to the cell (that is, a different cellular barcode is contained in each droplet).

For each one molecule of the predetermined gene derived from one cell, a linked product linked to one type of a cellular barcode unique to the cell can comprise a sequencing adapter sequence, a cellular barcode sequence, a linker sequence, all or some of the nucleotide sequences of the predetermined genes, and a sequencing adapter sequence in this order, as described above. This linked product may further contain an index sequence having a nucleotide sequence unique to the sample. The index sequence can be flanked by any two of the sequencing adapter sequence, the cellular barcode sequence, the linker sequence, all or some of the nucleotide sequences of the predetermined genes, and the sequencing adapter sequence. The index sequence may be contained instead of a sequencing adapter sequence or in addition to the sequencing adapter sequence.

In the present invention, a linked product of each molecule of the amplification product of the predetermined gene derived from one cell and the amplification product of one type of a cellular barcode unique to each cell can be prepared. Here, the predetermined gene is preferably of one type, but not limited to one type and can be of a plurality of types. The cellular barcode is preferably of one type for each cell.

In the present invention, the determined nucleotide sequence of the predetermined gene and the nucleotide sequence of the cellular barcode are linked and managed for a certain linked product. It is estimated based on this link that the predetermined genes to which the identical cellular barcode is linked are derived from the identical cell. Therefore, the sequencing method of the present invention can further comprise obtaining a combination of nucleotide sequences comprising the determined nucleotide sequence of the predetermined gene and the nucleotide sequence of the cellular barcode for each linked product.

Further, the sequencing method of the present invention can further comprise estimating that the predetermined genes to which the identical cellular barcode is linked are derived from the identical cell.

In one embodiment of the present invention, the predetermined gene is an endogenous gene of a microorganism and can be preferably a gene widely shared by various species evolutionally, such as, for example, a housekeeping gene. The housekeeping gene is a gene which is essential for energy metabolism and cell function and expressed or may be expressed in every cell. Housekeeping genes are not particularly limited, but examples thereof include ribosomal RNA (rRNA for example, 16S rRNA and 23S rRNA), ribosomal intergenic transcribed spacer (ITS), which exist between 16S rRNA and 23S rRNA, putative ABC transporter (abcZ), adenylate kinase (adk), shikimate dehydrogenase (aroE), glucose-6-phosphate dehydrogenase (gdh), single-function peptidoglycan transglycosylase (mtg), putative dehydrogenase subunit (pdhC), phosphoglucomutase (pgm), regulator of pilin synthesis(pilA), proline iminopeptidase (pip), polyphosphate kinase (ppk), and 3-phosphoserine aminotransferase (serC) (refer to Maiden et al., PNAS, Vol.95, 3140-3145, 1998). The sequences of these genes can be used in analyses of microbiotas. Further, 18S rRNA can also be used in analyses of fungi. If two or more types of genes are predetermined, an amplification reaction is performed using suitable primers and reaction conditions, so that each gene is linked to the cellular barcode. Since a cell population is analyzed based on the nucleotide sequence of the predetermined gene in the method of the present invention, it is advantageous to use a gene found in as many cells as possible as the predetermined gene. In one embodiment of the present invention, the predetermined gene can be the gene coding for 16S rRNA. The nucleotide sequence of the predetermined gene can be a full-length or partial sequence of the gene. For example, in the case of 16S rRNA, the sequence to be determined does not have to be a full length and may be a portion thereof. The V3 region and the V4 region can be used as a portion of 16S rRNA.

In the present invention, it is sufficient to use only one type of a gene (or a group of homologous genes) as the predetermined gene, and two or more types of different genes (or a group of two or more genes that are non-homologous to each other) do not need to be used. However, the predetermined gene may be two or more types of different genes (or a group of two or more genes that are non-homologous to each other).

In the sequencing method of the present invention, sequencing can be performed by destroying droplets and mixing solutions contained in all droplets. In the sequencing method of the present invention, sequencing can be performed using methods known to those skilled in the art. For example, sequencing can be performed in parallel using a next-generation sequencer (e.g., MiSeq and HiSeq of Illumina, Inc.). By using such a sequencer that decodes sequences in parallel, several tens of thousands to several hundreds of millions of gene fragments can be analyzed rapidly. In this case, those skilled in the art can add a sequencing adapter to the linked product, if required for sequencing, and this step is known to those skilled in the art.

The sequencing method of the present invention may further comprise collecting DNA in solutions before sequencing. DNA can be collected by collecting the aqueous phase separately contained in each droplet. For example, DNA can be collected by adding the obtained droplet population to an organic solvent (e.g., chloroform) and preferably further an aqueous solution (for example, buffer solutions, e.g., a Tris buffer solution containing a divalent metal ion chelator (e.g., $Ca^{2+}$ chelator and $Mg^{2+}$ chelator, for example, ethylenediamine tetraacetic acid (EDTA)) (i.e., Tris-EDTA buffer solution or TE solution)), stirring the mixture well, separating the aqueous phase and the organic phase, and collecting the aqueous phase. By doing this, target DNA (i.e., the linked product) discretely present in each compartment of a droplet in water-in-oil droplet type particles can be collected into the aqueous solution. In the aqueous solution thus obtained, all linked products derived from the contained droplets are mixed in the solution (a solution not divided into compartments by oil) (in other words, a state in which linked products discretely present in each droplet compartment exist in one solution compartment). Since nucleotide sequences of a large number of gene fragments can be decoded in parallel in sequencing as described above, a solution in which a large number of DNA are mixed is suitable for sequencing.

Additionally, the sequencing method of the present invention may further comprise purifying DNA before sequencing. DNA can be purified by gel filtration of the aqueous solution obtained by the above-described collection step. Gel filtration can be performed by techniques usually used to separate the DNA amplification product and other components in the solution (e.g., an unlinked barcode amplification product, primers not used for amplification, others) using gel filtration columns or the like. For example, gel filtration columns for DNA purification can be used as gel filtration columns. Additionally, the sequencing method of the present invention may further comprise purifying DNA contained in a solution by using columns or beads coated with a carboxyl group. Dehydrated DNA can be adsorbed specifically to the columns or beads coated with a carboxyl group via a salt, and then can remove DNA from the columns by hydration. For example, Agencourt AMPure XP (Beckman Coulter, Inc.) or the like can be used as the beads coated with a carboxyl group.

Further, when an amplification reaction of DNA is performed in the step of DNA amplification using tagged primers (e.g., biotinylated primers), which have a tag, a tag (e.g., biotin) has linked to the DNA amplification product. Such a tagged DNA amplification product can be concentrated or removed using columns or beads to which a molecule binding to the tag (e.g., tag-binding molecules such as avidin, streptavidin, and NeutrAvidin) is linked. In particular, when a linked product of the cellular barcode and the predetermined gene is obtained, the sequencing method of the present invention can preferably comprise removing cellular barcodes and predetermined genes that have failed to be linked. Specifically, one primer for amplification of a cellular barcode and one primer for amplification of a predetermined gene can be designed so that they have a tag and a complementary sequences. Specifically, a tag can be attached to only each of the primers designed to have complementary sequences. By doing this, as shown in Figure 5, the amplification product of the cellular barcode and the amplification product of the predetermined gene can be linked to each other in a region corresponding to the tagged primer portion. When the obtained linked product is further amplified by gene amplification, the amplification product of the linked product does not contain a tag, whereas an amplification product of the products that have failed to be linked has a tag derived from the primer at an end thereof. Thus, in one embodiment, in the above (B), one of two primers for amplification of a cellular barcode which has a sequence complementary to one of two primers for amplification of a predetermined gene (this one primer has a tag molecule) has a tag molecule. The tag molecule is lost from the linked product of the cellular barcode and the predetermined gene during amplification of the cellular barcode and the predetermined gene, and the tag molecule is to remain in only amplification products that have failed to be linked. Therefore, the amplification products that have failed to be linked can be removed by affinity using columns and beads to which a tag-binding molecule binds, and the amplification product of the linked product can be thereby purified in higher purity.

Therefore, in the sequencing method of the present invention,

the amplification product of the cellular barcode has a first region derived from a first primer, the amplification product of the predetermined gene has a second region derived from a second primer, the first region and the second region have complementary sequence portions hybridizable with each other, the first primer and second primer each have one or more tag molecules linked thereto, and the tag molecule is not contained in the linked product in the (B), and the sequencing method of the present invention may further comprise
removing amplification products having a tag molecule from the linked products collected in the aqueous solution, using columns or beads that carry a molecule having an affinity for the tag molecule in the (B). By doing this, amplification products having a tag molecule that have failed to be linked can be separated from the intended linked products.

The sequencing method of the present invention may comprise eliminating a nucleotide sequence region with low sequencing quality. Sequencing quality can be assessed by, for example, using a quality score based on the Phred algorithm (e.g., Phred quality score such as, for example, Q score ($Q = -10 \log_{10}(e)$, wherein e is an estimate of the probability of the basecall being incorrect)) (see Ewing et al., Genome Res., 8(3): 175-185, 1998 and Ewing and Green, Genome Res., 8(3): 186-194, 1998). As widely performed by those skilled in the art to reduce decoding errors in sequencing, sequences with a quality score being a certain threshold or lower can be excluded from the analysis. For example, sequences with a Q score of 20 or lower, 15 or lower, or 10 or lower can be excluded from the analysis.

The sequencing method of the present invention may further comprise

(C-1) clustering the determined nucleotide sequences based on nucleotide sequence of the cellular barcode to obtain a plurality of first clusters.

In the above (C-1), the "determined nucleotide sequences" can be a combination of nucleotide sequences comprising the determined nucleotide sequence of the predetermined gene and the nucleotide sequence of the cellular barcode.

Clustering the determined nucleotide sequences based on the determined nucleotide sequence of the cellular barcode can include not only clustering the nucleotide sequences of the cellular barcodes according to whether they are completely identical sequences, but also clustering sequences having some difference into the same cluster. The reason why sequences having some difference are clustered into the same cluster is that, in experiments, errors occur during the amplification reaction or sequencing of cellular barcodes, and the decoded nucleotide sequences can become different sequences from the original nucleotide sequences. However, errors that occur during the amplification reaction or sequencing are empirically well known. It is effective to cluster sequences having some difference into the same cluster not to distinguish identical sequences as different sequences because of the errors corresponding to such errors.

For example, when clustering is performed according to a criterion that the determined nucleotide sequences of the cellular barcodes are completely identical (distance 0), the nucleotide sequences derived from one cell are clustered correctly into one cluster if neither amplification error nor sequencing error exists. Therefore, there is no problem in such a case. In contrast, if any amplification error or sequencing error exists, nucleotide sequences derived from one cell can be incorrectly clustered into two or more clusters as those derived from different cells, when clustering is performed according to the criterion that the determined nucleotide sequences of the cellular barcodes are completely identical (distance 0).

In theory, with a criterion that sequences having an addition, elimination, deletion, or insertion (in particular, indel) of n nucleotides are also clustered into the identical cluster (distance n; n can be a natural number of 1 to 5), nucleotide sequences derived from one cell are to be correctly clustered into one cluster even if an addition, elimination, deletion, or insertion (in particular, indel) of up to n nucleotides arises from an amplification error or a sequencing error. Here, those skilled in the art can suitably select n based on the error rate in the amplification reaction or the sequencing error rate. When n is set to be large, the cellular barcodes can be designed to be always different for each cell by more than n nucleotides. In one embodiment of the present invention, n can be set as 1. In another embodiment of the present invention, n can be set as 2. In yet another embodiment of the present invention, n can be set as 3. Even if an addition, elimination, deletion, or insertion (in particular, indel) of up to n nucleotides occurs, the cellular barcode from which a nucleotide sequence having such an error is derived can be determined by designing the determined nucleotide sequences of the cellular barcodes so that the sequences are very different for each cell. Clustering can be expected to have an effect of reducing such impacts of experimental errors. Clustering can be performed with reference to WO 2018/235938, which is incorporated into the present specification as a whole.

Given that the cellular barcode is a sequence unique to each cell, in theory, a linked product including the identical cellular barcode should be linked to only the predetermined gene derived from the identical cell. Therefore, the predetermined genes derived from the identical cell can be determined by clustering the determined nucleotide sequences (including amplification products of the cellular barcodes and the predetermined genes) based on the nucleotide sequence of the identical cellular barcode. If only one predetermined gene exists in a cell, in theory, only one sequence is detected for the predetermined gene in the first cluster obtained in the above (C-1). In contrast, if a plurality of predetermined genes exists in a cell, in theory, the first cluster obtained in the above (C-1) can include two or more sequences (paralogs) for the predetermined gene. Therefore, in the method for analyzing a cell population of the present invention further comprising the above (C-1), the existence of cells having a multiplication(copy, paralog, or the like) of the predetermined gene can be detected in the cell population.

Further, in the above description, it can be estimated in the method of the present invention that the number of cells is, in theory, equal to the number of cellular barcode types or the number of clusters obtained based on the nucleotide sequence of the cellular barcode. Therefore, there is an advantage that the multiplication of the predetermined gene in one cell would not affect the accuracy of the number of cells to be calculated.

Therefore, the method for determining the gene sequences included in the cell population of the present invention may further comprise

(D-1) estimating the number of cells included in the cell population or the number of cells having a specific predetermined gene from the obtained number of the first clusters.

Further, in the above description, the determined nucleotide sequences were clustered based on the nucleotide sequence of the cellular barcode to obtain a plurality of first clusters. In contrast, in the following embodiments, the method for analyzing a cell population of the present invention can comprise clustering the nucleotide sequences determined based on the determined nucleotide sequence of the predetermined gene.

Specifically, the method for analyzing a cell population of the present invention may further comprise

(C-2) clustering the nucleotide sequences determined based on the determined nucleotide sequence of the predetermined gene to obtain a plurality of second clusters.

Clustering nucleotide sequences determined based on the nucleotide sequence of the predetermined gene to obtain a plurality of second clusters can comprise not only clustering sequences according to whether they are completely identical, but also clustering sequences having some difference into the same cluster. The reason for clustering sequences having some difference into the same cluster is that errors can occur in sequences during the amplification reaction or sequencing of the cellular barcode in experiments.

For example, when clustering is performed according to a criterion that the determined nucleotide sequences of the predetermined gene are completely identical (distance 0), the nucleotide sequences derived from one cell are clustered correctly into one cluster if neither amplification error nor sequencing error exists. Meanwhile, in theory, with a criterion that sequences having an addition, elimination, deletion, or insertion (in particular, indel) of n nucleotides are

14

also clustered into the identical cluster (distance n; n can be a natural number of 1 to 5), nucleotide sequences derived from one type of a gene are to be clustered into the same cluster even if the addition, elimination, deletion, or insertion (in particular, indel) of up to n nucleotides arises from an amplification error or a sequencing error. In one embodiment of the present invention, n can be set as 1. In another embodiment of the present invention, n can be set as 2. In yet another embodiment of the present invention, n can be set as 3. Here, n can be suitably selected by those skilled in the art. The number of the obtained clusters corresponds to the number of the types of the predetermined genes.

The sequences of predetermined genes are not understood for all microorganisms. However, in the sequencing method of the present invention, the cell population may include unknown microorganisms. This is because the unknown microorganisms can be treated as different microorganisms from known microorganisms as long as the unknown microorganisms have a predetermined gene having a nucleotide sequence that can distinguish them from other microorganisms.

By the way, if the nucleotide sequence of the predetermined gene in an unknown microorganism is different from the sequence of a known predetermined gene only by a distance of n or less, it is possible in the above-mentioned method that the unknown gene and the known gene may be clustered into the same cluster and may be estimated to be derived from the identical gene even if they have essentially different nucleotide sequences.

Therefore, the above (C-2) can further comprise a further step:

(C-2α) determining the most abundant nucleotide and determining the second most abundant nucleotide at one position of different nucleotide sequences when different nucleotide sequences of the predetermined genes are included in one cluster, and clustering nucleotide sequences having the most abundant nucleotide and nucleotide sequences having the second most abundant nucleotide into separate clusters when the ratio (Ratio2nd) of the number of nucleotide sequences (i.e., the number of reads) having the second most abundant nucleotide to the number of nucleotide sequence (i.e., the number of reads) having the most abundant nucleotide at the position is a predetermined value or higher. By doing this, different sequences derived from an essentially different gene among nucleotide sequences classified into the identical cluster can be treated as different sequences. By doing this, the frequency of assessing a different gene as identical by Step (C-2) can be reduced.

Step (C-2α) can be continued until Ratio2nd, a difference in nucleotide sequences, becomes lower than a predetermined value for all nucleotide sequences. The predetermined value can be a number of, for example, 0.6 or higher, 0.65 or higher, 0.7 or higher, 0.75 or higher, or 0.8 or higher. This is because, if the nucleotide sequence really exists, it should be contained in a plurality of cells and would be detected with a certain proportion. Meanwhile, since errors occur in low frequency, errors and originally existing sequences can be distinguished by this assessment.

In Step (C-2α), the above-mentioned number of reads may be weighted by the quality score for the nucleotide sequence of the predetermined gene. The quality score can be a score determined based on, for example, the Phred algorithm, for example, a Phred quality score, or, for example, Q score. When the quality score is lower than the predetermined value, the number of reads may be weighted with a low value (for example, 0). When the quality score is higher than the predetermined value, the number of reads may be weighted with a high value (for example, depending on the value of the score). This is as described in Step 3.2 in the Examples.

By this step, a nucleotide sequence having the most abundant nucleotide is designated as a "representative nucleotide sequence" (RepSeq) of the cluster.

When a shift of nucleotides is found for different RepSeqs (in other words, two nucleotide sequences match when the nucleotide sequences are shifted), a RepSeq found in more first clusters is designated as Mother, and a RepSeq found in less first clusters is designated as Shift. When the shifted nucleotide sequences are eliminated, it can be assumed that the clusters have the nucleotide sequence of Mother. At this time, the count (number of reads) of the shifted nucleotide sequences can be added to the number of reads in the Mother RepSeq. This is done as described in Step 5 in the Examples.

Step (C-2α) may further comprise excluding nucleotide sequences detected only with a single read as an error.

If the same sequence is detected in a plurality of first clusters, it is possible that the sequence has truly existed. Therefore, the precision of determining the nucleotide sequence is increased by performing Step (C-1) and Step (C-2) in combination. Additionally, whether a plurality of predetermined genes have existed in one cell can be determined by performing Step (C-1) and Step (C-2) in combination.

By doing this, the present invention can

(D-2) estimate the number of types of cells included in a cell population (how many types of cells are included in a cell population) from the number of the obtained second clusters.

Therefore, the method for determining gene sequences included in a cell population of the present invention may further comprise

(C-3) clustering the determined nucleotide sequences based on the determined nucleotide sequence of the cellular barcode to obtain a plurality of first clusters, and clustering the determined nucleotide sequences based on the determined nucleotide sequence of the predetermined gene to obtain a plurality of second clusters.

The details and effects of the clustering step described herein are as described in the above (C-1) and (C-2).

In the above (C-3), a second cluster may be formed for each first cluster, or a first cluster may be formed for each second cluster.

The method for determining gene sequences included in a cell population of the present invention may further comprise

(D-3) determining a first cluster into which the nucleotide sequence of the predetermined gene is classified from the nucleotide sequence of the cellular barcode linked to nucleotide sequence of the predetermined gene classified into at least one second cluster based on information about a combination of the obtained nucleotide sequence of the cellular barcode and the nucleotide sequence of the predetermined gene, and estimating the number of cells classified into the second cluster from the number of the first clusters into which the cellular barcode is classified.

Here, the nucleotide sequences of the predetermined genes were classified: out of two nucleotide sequences with a distance of n (e.g., two nucleotide sequences having a difference of one loss or deletion (i.e., 1-indel) in the central portion of the sequence), a nucleotide sequence classified into more first clusters is designated as Mother (that is, a nucleotide sequence detected in more cells is designated as Mother), and the other one classified into less first clusters is designated as 1-Indel. The number of first clusters in which the number of reads in Mother is more than the number of reads in 1-Indel ($No_{Mother}$) and the number of first clusters in which the number of reads in Mother is less than the number of reads in 1-Indel ($No_{1-Indel}$) are compared. If $No_{Mother}$ is larger than $No_{1-Indel}$, the pair of Mother and 1-Indel can be kept. Further, if the ratio of $No_{1-Indel}$ to the number of first clusters including both Mother and 1-Indel is smaller than a predetermined value (e.g., ($No_{1-Indel}$ - 3) /$No_{1-Indel}$), the pair of Mother and 1-Indel can be kept. After a 1-indel is deleted from the remaining pairs of Mother and 1-Indel, the number of reads in 1-Indel can be added to the number of reads in Mother. Further, if two different Mothers exist for the same 1-Indel, the number of reads can be added to Mother found in more first clusters. Further, if there is a first cluster in which 1-Indel alone is detected without Mother, the number of reads in 1-Indel can be assumed as the number of reads in Mother in the cluster. This is done as described in Step 7 in the Examples.

Further, one amplification product may be linked to another amplification product during the process of gene amplification, and the resulting occurrence of a chimeric molecule can be a problem. Although the Examples show that the frequency of generation of chimeric molecules is clearly very low in the method of the present invention, the method of the present invention can further comprise identifying the chimeric molecules. Chimeric molecules can be identified as follows. For example, if the ratio (N_d/Total_N) of the number of first clusters including only chimeric molecules and not including Parents (N_d) to the number of first clusters including a chimeric molecules (Total_N) is a certain value or lower which is lower than 1, these chimeric molecules are considered to have arisen from errors and can be excluded from RepSeqs. This is done as described in Step 8 in the Examples.

In addition to the above-described (C-2), the method of the present invention may further comprise creating a cell-based operational taxonomic unit (cOTU). The numbers and types of microorganisms included in a cell population are often unknown. Further, when unknown microorganisms exist, analyses of gene sequences in the cell population would be insufficient with information about known gene sequences registered in databases alone. In particular, when an operational taxonomic unit (OTU) is formed based on the nucleotide sequence of a predetermined gene, and one microorganism species has n multiplications for the predetermined gene, an error occurs because the count of the microorganism species would be n times more than the correct count. Additionally, if one of two different microorganism species has nucleotide sequences A and B, and the other one has A and C, and an operational taxonomic unit (OTU) is formed based on the nucleotide sequence, three OTUs corresponding to A, B, and C would be formed, and the number of cells would have an error by the count of A. Accordingly, in (C-4), cOTUs are formed from the information of RepSeqs to reduce the above-mentioned error in the count obtained when cells having a gene multiplication are included in the cell population. Of note, in theory, cOTU is a classification unit for microorganisms classified by the nucleotide sequence of the predetermined gene and a technical means for more detailed classification of microorganisms that was able to be classified only into high-order taxa so far. This technique is particularly useful for analyzing a cell population including microorganisms that have not been classified in details or unidentified microorganisms. If classification is possible, it would be advantageous because differences between cell populations can be compared based on the classification.

A cOTU can be formed as follows. Specifically, as in conventional methods, one second cluster can be assumed as one cOTU. In consideration that two or more second clusters are included in one cell, however, the present invention can further comprise classifying a plurality of second clusters linked to the identical cellular barcode into one cOTU.

In other words, in addition to the above-described (C-3), the method of the present invention may further comprise, for example,

(C-4) classifying second clusters into an identical cell-based operational taxonomic unit (cOTU, i.e., an identical cell type) when sequences classified into an identical first cluster are classified into different second clusters.

This formation of cOTUs may further comprise excluding experimental errors (for example, when two cells are contained in one droplet and analyzed, the nucleotide sequences of the predetermined gene derived from two cells are thereby detected in one first cluster).

If two nucleotide sequences of the predetermined gene linked to one cellular barcode sequence exist, the probability that two cells get mixed in one droplet, in theory, follows the Poisson distribution. Since the above-mentioned error type A appears to be an error that depends on the cell concentration during the preparation of droplets, it is considered that the error frequency can be reduced by reducing the cell concentration during preparation of droplets (a concentration at which cells are contained in 20% of droplets was used in the Examples). Additionally, the probability that two nucleotide sequences exist in different cells but are contained in one droplet during the operation, in theory, follows the Poisson distribution.

If a plurality of RepSeqs labeled with one cellular barcode (RepSeqs may be sequences after elimination of various errors in the above-described steps, which is preferred) exist, all these RepSeqs are picked up. The number of droplets containing two RepSeqs is expressed as (Overlap). The probability that two RepSeqs derived from different cells are contained in one droplet (Poission_Overlap) can be expressed as $(A \times B \times \mu)$/total number of droplets, wherein the total number of cells is the total number of droplets containing the cellular barcode, A is the number of droplets containing one RepSeq, B is the number of droplets containing the other RepSeq, and $\mu$ is an integrated parameter for detection efficiency in droplets that can include PCR amplification efficiency, sequencing depth effect, and the like. Here, the expression,

(Poission_Overlap) = $(A \times B \times \mu)$/total number of droplets, can be converted to
$\log_{10}$(Poission_Overlap) = $\log_{10}\{(A \times B \times \mu)$/total number of droplets$\}$.

Further, the above expression can be converted to $\log_{10}$(Poission_Overlap) = $\log_{10}(A \times B)$ - $\log_{10}$ (total number of droplets/$\mu$).

Here, A and B can be measured experimentally, $\log_{10}$ (total number of droplets/$\mu$) can be set as a certain constant for each experiment. Therefore, when $\log_{10}$ (total number of droplets/$\mu$) is assumed as a constant OD, the above expression can be converted to

$$\log_{10}(\texttt{Poission\_Overlap}) = \log_{10}(A \times B) - OD.$$

This can be linearly approximated with y = x - OD. $\log_{10}$ (Poission_Overlap) can be calculated by assuming various integers for A and B. If the value of $\log_{10}$ (Overlap) in reality is outside the confidence interval of the calculated $\log_{10}$(Poission_Overlap), it can be estimated that two nucleotide sequences were contained in one cell. Additionally, if two nucleotide sequences are within the confidence interval of $\log_{10}$ (Poission_Overlap), it can be estimated that they were contained in different cells. As a confidence interval, for example, a one-sided confidence interval (for example, it can be a confidence interval of 95% or higher, 98% or higher, 99% or higher, or 99.9%, or higher) can be used. Thus, if it cannot be explained statistically by the Poisson distribution, it can be estimated that two nucleotide sequences were contained in one cell. Or, if it can be explained statistically by the Poisson distribution, it can be estimated that two nucleotide sequences existed in different cells.

Further, in theory, the results of RepSeqs for the same microorganism are considered the same in different samples. Therefore, even if it is reproduced in different samples, a plurality of different cell population samples can be measured to obtain the ratio of the number of samples for which the value of $\log_{10}$ (Overlap) to the number of samples including two RepSeqs is outside the confidence interval of $\log_{10}$ (Poission_Overlap). If this ratio is larger than a certain value (for example, a certain value can be a number of 0.4 or more), it can be estimated that two RepSeqs are derived from one cell.

Further, two RepSeqs classified into the same first cluster are found to exist in the identical cell, and these two RepSeqs can be therefore classified into a cOTU. Thus, the second cluster can be re-classified as cOTU.

Or, if the predetermined gene is 16s rRNA, a predicted taxon having the highest score can be created by classification using the RDP classifier or machine learning using a training set of 16s rRNA in the RDP classification, and this taxon can be used as a cOTU. Of note, the RDP classifier is a tool for determining a microorganism species from the nucleotide sequence of 16S rRNA developed by the Ribosomal Database Project.

Further, the method of the present invention may further comprise correcting (or standardizing) the total number of cells calculated by the method of the present invention using the total number of cells estimated from the count obtained by optical microscopy or the like. The precision of predicting the number of cells (e.g., the number of cells in a specific cluster or the number of cells in a specific cOTU) calculated by the method of the present invention can be improved by correcting (or standardizing) the total number of cells.

The method of the present invention can be used for a comparison of two different cell populations. Further, the method of the present invention can further comprise

(E) for each of a first cell population and a second cell population that is different from the first cell population, estimating (i) the number of cOTUs and/or (ii) the number of cells included in a specific cOTU included in the cell population, and comparing (i) the number of cOTUs and/or (ii) the number of cells included in the specific cOTU estimated for the first cell population with (i) the number of cOTUs and/or (ii) the number of cells included in the specific cOTU estimated for the second cell population.

In the above-described (E), the numbers of cells in the cell populations to be compared can be made equal beforehand. In the above-described (E), the characteristics of each cell population can be described in view of cOTUs by comparing the number of cOTUs and the number of cells included in each cOTU between two different cell populations.

Two cell populations can be, for example, cell populations isolated from an identical site of an identical subject at different timepoints, can be cell populations isolated from different sites of an identical subject at an identical timepoint, or can be cell populations isolated from an identical site of different subjects at an identical timepoint.

When cell populations isolated from an identical site of an identical subject at different timepoints are compared by the above-described (E), differences associated with the sampling times (e.g., changes with time in a health condition, differences in a health condition before and after a treatment, onset or progression of a disease or a condition) are to be described in view of cOTUs. Further, when cell populations isolated from different sites of an identical subject at an identical timepoint are compared by the above-described (E), differences associated with the sampling sites (e.g., differences of microbiota for each organ) are to be described in view of cOTUs. Further, cell populations isolated from an identical site of different subjects at an identical timepoint are compared by the above-described (E), differences associated with the subjects (e.g., health condition, sex, region, race) are to be described in view of cOTUs.

The method of the present invention may further comprise

(F) comparing (i) the number of cOTUs and (ii') the number of cells included in a specific cOTU which are estimated for the first cell population with (i) the number of cOTUs and (ii') the number of cells included in a specific cOTU which are estimated for the second cell population.

In the above-described (F), a correlation between the number of cOTUs estimated for the first cell population and the number of cOTUs estimated for the second population can be determined.

In the above-described (F), one or more specific cOTUs estimated for the first cell population and one or more cOTUs estimated for the second cell population which correspond to the one or more cOTUs can be compared. Here, whether a cOTU estimated from one cell population corresponds to a cOTU estimated from another cell population can be checked by confirming whether all nucleotide sequences (or nucleotide sequences after correction of errors) included in the cOTU are identical. In the above-described (F), in particular, whether an increase or decrease in the number of cells included in each cOTU correlates positively or negatively or does not correlate (weakly correlate) with an increase or decrease in the number of cells included in other cOTUs can be determined. By doing this, the network between cOTUs can be estimated.

Or, in the field of synecology, cell populations (these cell populations include a plurality of cOTU taxa, and the number of cells has been determined for each cOTU taxon) can be compared using various indices which serve as indices for similarity between groups. For example, the similarity between the first cell population and the second cell population can be obtained as the root mean square of a difference in the number of cells included in each cOTU (c.f., Euclidean distance). Further, the similarity between the first cell population and the second cell population can also be obtained as the sum of absolute values of a difference in the number of cells included in each cOTU (c.f., Manhattan distance). The cell populations are shown to be more dissimilar as these numerical values are larger. If the cell populations are completely identical, the numerical value is 0. Bray-Curtis dissimilarity (index) is a standardized Manhattan distance. When the cell composition of the first cell population is assumed as $(X_{11}, ..., X_{1n})$, and the cell composition of the second cell population is assumed as $(X_{21}, ..., X_{2n})$, the Bray-Curtis index can be obtained by the following expression:

[Expression 1]

$$Bray\text{-}Curtis\ dissimilarity\ (index) = \frac{\sum_{i=1}^{n}|X_{i1} - X_{i2}|}{\sum_{i=1}^{n}|X_{i1} + X_{i2}|}$$

The Bray-Curtis index is 1 when two groups are completely different and 0 when they completely match. Thus, the Bray-Curtis index may be referred to as dissimilarity because this index has been designed to become larger when the groups are more different. The Bray-Curtis index can be calculated using a statistical processing program (e.g., R package vegan function such as, for example, vedist function). In addition, similarity can be assessed using assessment indices commonly used in the field of synecology, such as Morishita index, Jaccard index, and Chao index. The standard deviation and the confidence interval of the estimated similarity can be assessed by the bootstrap method and the like.

The method of the present invention can further comprise

(G) performing hierarchical clustering for cOTUs.

Hierarchical clustering can be performed by methods known to those skilled in the art based on, for example, the intensity of correlation between cOTUs (e.g., Spearman correlation coefficient r). Hierarchical clustering may be performed by methods known to those skilled in the art based on the distance between cOTUs calculated from r. The distance can be calculated by, for example, 1 - minimum (|r'|) [r' ∈ (r - OCI, r + OCI)], wherein OCI represents a one-sided 90% confidence interval of each r. The results of hierarchical clustering can be expressed as evolutionary trees. This can be performed by, for example, R package hclust or Pheatmap. Further, a network of cOTUs with a Pearson correlation coefficient r being a threshold or higher (e.g., 0.5 or higher, 0.6 or higher) can be illustrated using a package igraph. Thus, correlations between cOTUs can be illustrated based on the relationship of cOTUs in a plurality of cell populations.

When cOTUs correspond to known microorganisms, correlations between known microorganisms can be characterized. Even if cOTUs correspond to unknown microorganisms, correlations between cOTUs can be characterized. When one cOTU corresponds to one known microorganism, a correlation between the known microorganism and another microorganism (this another microorganism may be unknown or known) can be characterized. Further, when two correlating cOTUs correspond to two known microorganisms, they can be used to find a new correlation between two known microorganisms and the like. Thus, when n correlating cOTUs correspond to n types of known microorganisms, new correlations between n types of known microorganisms can be found. Thus, the method of the present invention can be used to characterize correlations between microorganisms by investigating a plurality of cell populations (e.g., a plurality of microbiota). The health condition of a subject may correlate with the microbiota in the subject. Therefore, by further investigating a correlation between the health condition of a subject and one cOTU, the health condition of the subject can be predicted from a cOTU corresponding to an unknown microorganism even if the cOTU itself is the unknown microorganism (it should be noted that the cOTUs themselves are common among different samples). Further, in addition to the correlation between the health condition of the subject and one cOTU, the precision of predicting the health condition of the subject from cOTUs can be improved by further investigating a correlation of the cOTU with another correlating cOTU.

Thus, while previous analyses have been based on the precondition that one microorganism has one predetermined gene, the present invention provides a method for describing a microorganism using a new group concept of cOTU even if one microorganism has a plurality of predetermined genes. Further, the number of cells included in each cOTU can be accurately counted by counting the number of cells using a cellular barcode qualitatively for each cOTU. When the cell population to be analyzed is analyzed by the method of the present invention, the type of cOTUs included therein and the number of cells included in each cOTU can be obtained. A cell population including unknown microorganisms can also be analyzed in further detail using information of the unknown microorganisms by analyzing the obtained cell population, types of cOTUs, and the number of cells included each cOTU.

Further, the present invention has an advantage that analytical precision is not deteriorated even if the gene copy number varies depending on the cell. In other words, the gene copy number in a cell may vary in an identical microorganisms. In such a case, the gene copy number might affect the cell count in conventional methods. Since the number of cells is counted qualitatively using the cellular barcode in the method of the present invention, cells can be counted without being affected by gene copy numbers in the cell. Some microorganisms release substances that affect the environment (e.g., toxic substances and growth factors). Measuring the number of cells accurately enables more accurate estimation of the amount of the released substances and can pave the way for developing a mathematical modeling based on the volume of the released substances.

In the method of the present invention, the gene to be sequenced may be one specific type of a gene or a plurality of types of genes. In the method of the present invention, the gene to be sequenced does not need to be the whole genome.

Furthermore, in conventional methods, for example, nucleotide sequences of all the genes coding for 16S rRNA included in the cell population are sequenced in the analysis of 16S rRNA, and the obtained nucleotide sequences are classified based on a threshold. For example, 97% is selected as a threshold for identity, and analyses are performed assuming that genes with identity of 97% or higher are the same gene. In such analyses, however, microorganisms that should belong to biologically different taxa by nature, such as different species, different genera, or different families, were to be recognized as one group. However, the method of the present invention can determine whether a new 16S rRNA is truly new or arises from an experimental error. For example, an identical sequence found in a plurality of cells can be a sequence that exists by nature, and it can be confirmed using a cellular barcode. Thus, the method of the present invention can be an assessment method that is not affected by the similarity of nucleotide sequences when the nucleotide sequences are different.

Examples

In the Examples, a mock microbiota composed of known bacteria at known concentrations (herein referred to as "a mock cell population") was prepared to verify the assay system, and then an actual microbiota (here the cecal microbiota) was investigated.

[Method]

Preparation of a mock cell population

A mock cell population comprising human intestinal bacterial strains (ATCC29098, ATCC700926, DSM14469, JCM1297, JCM5824, JCM5827, JCM9498, JCM10188, JCM14656, and JCM17463) was prepared.[9] Table 1 lists the names, supply sources, media, and culture conditions of these strains. Cultured bacteria were preserved in the original medium with 10% glycerol or in phosphatebuffered saline (PBS) at -80°C until experiments (Table 1). After cultured, JCM14656 and DSM14469 were centrifuged and washed once with PBS. JCM10188 was cultured on GAM agar (Nissui), and bacterial colonies were collected and vortexed at 3,200 rpm for 1 minute to suspend the bacterial cells in PBS (VORTEX GENE 2; Scientific Industries).
Ten strains were diluted with PBS and mixed at prespecified concentrations in a class II biosafety cabinet (Table 1). Following each step of dilution and mixing, the mixture was vortexed at 3,200 rpm for 1 minute. This mixture of 10 strains is referred to as a "mock cell population." The mock cell population was preserved at - 80°C until experiments.

[Table 1]

Table 1: Names, supply sources, media, and culture conditions of bacterial strains

| Bacterial name | Strain ID | Supply source | Medium | Culture condition | Wash | Preservation | Concentration* | Phylum | Gram positive/ negative** |
|---|---|---|---|---|---|---|---|---|---|
| *Collinsella aerofaciens* | JCM 10188 | RIKEN BRC | GAM Agar | Anaerobic condition | - | ## | $7.8 \pm 2.7 \times 10^2$ | Actinobacteria | + |
| *Bacteroides caccae* | JCM9498 | RIKEN BRC | GAM | Anaerobic condition | - | # | $3.7 \pm 1.1 \times 10^4$ | Bacteroidetes | - |
| *Bacteroides ovatus* | JCM5824 | RIKEN BRC | GAM | Anaerobic condition | - | # | $3.3 \pm 1.3 \times 10^2$ | Bacteroidetes | - |
| *Bacteroides thetaiotaomicron* | JCM5827 | RIKEN BRC | GAM | Anaerobic condition | - | # | $3.8 \pm 1.1 \times 10^4$ | Bacteroidetes | - |
| *Blautia hydrogenotrophica* | JCM 14656 | RIKEN BRC | GAM | Anaerobic condition | PBS | ## | $1.3 \pm 0.5 \times 10^2$ | Firmicutes | + |
| *Clostridium symbiosum* | JCM1297 | RIKEN BRC | GAM | Anaerobic condition | - | # | $5.5 \pm 2.3 \times 10^2$ | Firmicutes | + |
| *Eubacterium rectale* | JCM 17463 | RIKEN BRC | GAM | Anaerobic condition | - | # | $7.2 \pm 2.9 \times 10^3$ | Firmicutes | + |
| *Marvinbryantia formatexigens* | DSM 14469 | DSMZ | PYG | Anaerobic condition | PBS | ## | $1.6 \pm 0.7 \times 10^2$ | Firmicutes | + |
| *Desulfovibrio piger* | ATCC29098 | ATCC | ATCC medium 1249 | Anaerobic condition | - | # | $1.6 \pm 0.5 \times 10^3$ | Proteobacteria | - |
| *Escherichia coli* | ATCC700926 | ATCC | LB | Aerobic condition | - | # | $1.0 \pm 0.2 \times 10^4$ | Proteobacteria | - |

The symbols in the above table are as follows:

* Concentration of added cells that was measured by microscopic imaging for formation of the mock cell population (mean ± S.D., n = 5, cells/μL)
** +, Gram-positive; -, Gram-negative
# Preserved in a medium with 10% glycerol at -80°C.
## Preserved in phosphate buffer saline (PBS) at -80°C. GAM, Gifu Anaerobic Medium (Nissui).
GAM Agar, Modified GAM Agar (Nissui)
LB, Luria-Bertani (Nacalai Tesque).
PYG, Peptone Yeast Glucose, DSMZ medium 104.
ATCC medium 1249, Modified Baar's medium for sulfate reducers.

Measuring concentrations of bacteria by microscopic imaging

The concentration of each strain was measured by fluorescence imaging under a microscope. Fluorescences-stained bacteria were measured using polystyrene microspheres (Bacteria Counting Kit; Thermo Fisher Scientific). Bacterial cells were heated at 70°C for 5 minutes and stained using propidium iodide (Thermo Fisher Scientific). The volume was calculated based on the microsphere concentration measured using a bacteria cell counting chamber (SomaLogic, Inc.). Five independent measurements were performed for each bacterial strain. The mean concentration and the standard deviation (as an error bar) of these five measurements were used to calculate the concentration of each bacterial strain in the mock cell population.

[Table 2]

**Table 2: Designing cell counts and absolute concentrations for the mock cell population**

| Lineage ID | cOTU ID | Number of cells (a) | Number of cells (b) | Number of cells (c) | Mean | Standard deviation | Absolute concentration (cells/μL) | Standard deviation |
|---|---|---|---|---|---|---|---|---|
| ATCC700926 | COTU-MK-01 | 4815: | 4918 | 4855 | 4863 | 52 | 1.63E+04 | 2E+02 |
| JCM9498 | CCTU MK-02 | 9639 | 10181 | 10353 | 10058 | 372 | 3.36E+04 | 1.2E+03 |
| JCM5827 | COTU-MK-03 | 7712 | 7461 | 7459 | 7544 | 145 | 2.52E+04 | 5E+02 |
| JCM17463 | COTU-MK-04 | 5185 | 4773 | 4796 | 4918 | 232 | 1.64E+04 | 8E+02 |
| ATCC29098 | COTU-MK-05 | 517 | 555 | 477 | 516 | 39 | 1.73E+03 | 1.3E+02 |
| JCM 10188 | COTU-MK-06 | 132; | 138 | 125 | 132 | 6 | 4.40E+02 | 2.2E+01 |
| DSM14469 | COTU-MK-07 | 21 | 20 | 14 | 18 | 4 | 6.1E+01 | 1.3E+01 |
| JCM1297 | COTU-MK-08 | 78 | 111 | 108 | 99 | 18 | 3.3E+02 | 6E+01 |
| JCM5824 | COTU-MK-09 | 73 | 60 | 44 | 59 | 15 | 2.0E+02 | 5E+01 |
| JCM14656 | COTU-MK-10 | 39 | 31 | 34 | 35 | 4 | 1.2E+02 | 1E+01 |

a, b, and c represent three repeated samplings.

The absolute concentration was obtained by normalizing a raw count value determined by sequencing by the total concentration (94,400 cells/μL) of the mock cell population measured by droplet digital PCR (ddPCR).

Sanger sequencing of 16S rRNA gene

The 16S rRNA gene of each strain was amplified using 2 × KAPA HiFi HotStart ReadyMix (Roche) and primers F1-full-Fw and F3-full-Rv (Table 3). Subsequently, the amplified 16S rRNA gene was cloned in a pCR-Blunt II-TOPO vector and amplified with *E. coli* using Zero Blunt TOPO PCR Cloning Kit (Thermo Fisher Scientific). Subsequently, the 16S rRNA gene was amplified from each single colony of *E. coli* using T7-Promoter and SP6-Promoter as primers (Table 3). Finally, the V3-V4 region of the 16S rRNA gene amplified from each colony was sequenced by Sanger sequencing (Fasmac Co., Ltd.) using the F2-Rv primer (Table 3).

[Table 3]

Table 3: Sequences of primers and barcodes

| Primer name | Nucleotide sequence |
|---|---|
| 341F | 5'-CCTACGGGNGGCWGCAG-3' |
| 805R | 5'-GACTACHVGGGTATCTAATCC-3' |
| P7-R2P-341F | 5'-CAAGCAGAAGACGGCATACGAGATGTGACTGGAGTTCCTTGGCACCCGAG AATTCCACCTACGGGNGGCWGCAG-3' |
| Biotin-link-805R | 5'-/5Biosg/GCTCCTGCGTTCGGATCGTAGTCGGAC/iBiodT/ACHVGGGTATCTAA TCC-3' |
| Biotin-Link-barcode-F | 5'-/5Biosg/CGACTACGATCCGAACGCAGGAGCTCAGCC/iBiodT/CGACAGTCCAG TG-3' |
| P5-index-RIP-barcode-R | 5'-AATGATACGGCGACCACCGAGATCTACACXXXXXXXXXACACTCTTTCCCTA CACGACGCTCTTCCGATCT-3' (X: index sequence) |
| NoBiotin-Link-barcode-F | 5'-CGACTACGATCCGAACGCAGGAGCTCAGCCTCGACAGTCCAGTG-3' |
| F1-Fw | 5'-AGRGTTTGATYMTGGCTCAG-3' |
| F1-Rv | 5'-CTGGCACGDAGTTAGCC-3' |
| F1-full-Fw | 5'-CAAGCAGAAGACGGCATACGAGATGTGACTGGAGTTCCTTGGCACCCGAG AATTCCAAGRGTTTGATYMTGGCTCAG-3' |
| F3-full-Rv | 5'-GCTCCTGCGTTCGGATCGTAGTCG TACGGYTACCTTGTTACGACTT-3' |
| T7-Promoter | 5'-TAATACGACTCACTATAG-3' |
| SP6-Promoter | 5'-ATTTAGGTGACACTATAG-3' |
| F2-Rv | 5'-CTTGTGCGGGCCCCCGTCAATTC-3' |
| Barcode-1 | 5'-TCAGCCTCGACAGTCCAGTGACNNNNTNNNNGNNNNANNNNCNNNNNNNN AGATCGGAAGAGCGTCGTGTAGGGAAAGAGTGT-3' |
| Barcode-2 | 5'-TCAGCCTCGACAGTCCAGTGTGNNNNANNNNCNNNNTNNNNGNNNNNNNN AGATCGGAAGAGCGTCGTGTAGGGAAAGAGTGT-3' |
| Barcode-3 | 5'-TCAGCCTCGACAGTCCAGTGGANNNNCNNNNANNNNGNNNNTNNNNNNNN AGATCGGAAGAGCGTCGTGTAGGGAAAGAGTGT-3' |
| Barcode-4 | 5'-TCAGCCTCGACAGTCCAGTGCTNNNNGNNNNTNNNNCNNNNANNNNNNNN AGATCGGAAGAGCGTCGTGTAGGGAAAGAGTGT-3' |
| P1_qPCR_Fw | 5'-AATGATACGGCGCACCACCGA-3' |
| P2_qPCR_Rv | 5'-CAAGCAGAAGACGGCATACGA-3' |
| StdTarget1 | 5'-GAGTTCCTTGGCACCCGAGAATTCCA-174N-3' |
| StdTarget2 | 5'-/SPhos/-176N-CGACTACGATCCGAACGCAGGAGC-3' |

(continued)

| Primer name | Nucleotide sequence |
|---|---|
| RandomBar_std1 | 5'-TTCCCTACACGACGCTCTTCCGATCTNNNNNNNNCNNNNTNNNNANNNNG NNNNCTCACTGGACTGTCGAGGCTGAGCTCCTGCGTTCGGATCGTAGTC-3' |
| RandomBar_std2 | 5'-TTCCCTACACGACGCTCTTCCGATCTNNNNNNNNGNNNNANNNNTNNNNA NNNTGCACTGGACTGTCGAGGCTGAGCTCCTGCGTTCGGATCGTAGTC-3' |
| RandomBar_std3 | 5'-TTCCCTACACGACGCTCTTCCGATCTNNNNNNNNTNNNNCNNNNGNNNNC NNNNGACACTGGACTGTCGAGGCTGAGCTCCTGCGTTCGGATCGTAGTC-3' |
| RandomBar_std4 | 5'-TTCCCTACACGACGCTCTTCCGATCTNNNNNNNNANNNNGNNNNCNNNNT NNNNACCACTGGACTGTCGAGGCTGAGCTCCTGCGTTCGGATCGTAGTC-3' |
| Index_NSE501 | 5'-AATGATACGGCGACCACCGAGATCTACACTAGATCGCACACTCTTTCCCTA CACGACGCTCTTCCGATCT-3' |
| Index_NSE502 | 5'-AATGATACGGCGACCACCGAGATCTACACCTCTCTATACACTCTTTCCCTAC ACGACGCTCTTCCGATCT-3' |
| Index_NSE505 | 5'-AATGATACGGCGACCACCGAGATCTACACGTAAGGAGACACTCTTTCCCTA CACGACGCTCTTCCGATCT-3' |
| Index_NSE506 | 5'-AATGATACGGCGACCACCGAGATCTACACACTGCATAACACTCTTTCCCTA CACGACGCTCTTCCGATCT-3' |
| std_R2 | 5'-CAAGCAGAAGACGGCATACGAGATGTGACTGGAGTTCCTTGGCACCCGAG AATTCCA-3' |
| Il_primer | 5'-CTGAGCTCCTGCGTTCGGATCGTAGTCG-3' |
| CONV-341F | 5'-TCGTCGGCAGCGTCAGATGTGTATAAGAGACAGCCTACGGGNGGCWGCA G-3' |
| CONV-806R | 5'-GTCTCGTGGGCTCGGAGATGTGTATAAGAGACAGGACTACHVGGGTATCTA ATCC-3' |

Method of sequencing 16S rRNA

In brief, bacteria in the mock cell population were suspended in PBS and exposed sequentially to lysozyme, achromopeptidase, and proteinase K for cytolysis. Subsequently, DNA was collected by phenol-chloroform extraction. The V3-V4 region of the 16S rRNA gene was amplified using region-specific primers containing Illumina Adapter Overhang Nucleotide Sequences (CONV-341F and CONV-805R in Table 3). The amplification product was purified using AMPure XP magnetic beads (Beckman Coulter) and indexed using Nextera XT Index Kit v2 (Illumina). After purification using AMPure XP, the pooled libraries were analyzed qualitatively and quantitatively using TapeStation (Agilent) and KAPA Library Quantification Kit for Illumina (Kapa Biosystems). A denatured library spiked with 20% PhiX Control v3 (Illumina) was sequenced using a MiSeq platform (Illumina, 2 × 300 bp paired-end reads). The quality of the sequence data was checked, and sequences were trimmed using Trimmomatic version 0.38.[47] OTUs were clustered using mothur version 1.35.1[48] with an identity threshold of 97%. The most abundant sequence in each OTU was selected as the representative sequence of the OTU (Figure 1b).

Preparation of mice

All treatments of mice were conducted in accordance with the protocol approved by the Animal Experiments Committee of Institute of Physical and Chemical Research and the institute's ethical guideline. Mice which maintained the condition were as follows: Six-week-old male C57BL6/J mice were purchased from CLEA Japan and maintained in the same cage at Institute of Physical and Chemical Research for 3 days by feeding CE-2 feed (CLEA Japan) before sampling.

Collection of murine cecal content

The murine cecum was removed out of the body by an operation within 10 minutes after cervical dislocation under anesthesia with sevoflurane. The cecal content from different sites (Figure 2a) was sliced with sterilized scissors for sampling. The sampling step was performed in a class II biosafety cabinet within 10 minutes after the operation. A sample from each site of each mouse was collected into a DNA LoBind Tube (Eppendorf). For controls, two empty test tubes were used. The weight of the sample was measured (ranged from 8.57 to 19.82 mg for all samples) immediately after collection into the DNA LoBind Tube. Subsequently, each sample was dispersed in added PBS (50 μL per mg), and the dispersion was vortexed at 3,200 rpm for 1 minute to mix. The suspended sample was preserved at 4°C until subsequent experiments.

Filtration of extracellular DNA

The murine cecal sample was diluted in 1 mL of PBS per mg of the cecal content, and then the mixture was vortexed at 3,200 rpm for 1 minute. For a control, PBS was added to an empty tube. Subsequently, 400 μL of the diluted sample was filtered by centrifugation (10,000 g, 10 minutes, 4°C) using Ultrafree-MC Centrifugal Filter (Merck) having a pore size of 0.22 μm. A volume of 400 μL of fresh PBS was added to the sample remaining on the membrane, the sample was suspended by pipetting, and the total volume was transferred to a fresh DNA LoBind Tube. Subsequently, the sample suspension was vortexed at 3,200 rpm for 1 minute. Extracellular DNA contained in the sample suspension and the fluid that passed through the filter were preserved at 4°C until subsequent measurements. Of note, the appropriateness of DNA isolation using a 0.22-μm filter was confirmed because the amount of extracellular DNA in the fluid that passed through the filter was virtually equal, with no cells detected in the fluid that passed through the filter, and the amounts of bacteria collected from the top of the filter after filtration were equal as compared with use of a filter having a pore size of 0.1 μm, and the amount of bacteria collected from the filter correlated to the number of bacteria obtained by digital PCR (see Figure 31).

BarBIQ method

Measuring the total concentration

The total concentration of cellular or extracellular 16S rRNA genes was measured by Droplet Digital™ PCR (ddPCR) (Bio-Rad) using primers F1-Fw and F1-Rv (Table 3). The concentration of four equimolar mixed cellular barcode templates (Table 3; each template containing 24 random nucleotides was designed according to our previous publication,[25] and the number of random nucleotides was sufficient to distinguish individual cells measured in a single MiSeq sequencing operation) was also measured by ddPCR using primers NoBiotin-Link-barcode-F and P5-index-R1P-barcode-R (Table 3). ddPCR was performed in accordance with the user manual of QX200™ ddPCR™ EvaGreen (trademark) Supermix (Bio-Rad).

One step droplet amplification

To prepare a sequence library, a total of approximately 240,000 cells (or 20,000 copies of extracellular 16S rRNA gene) were mixed with 960 μL of a solution containing equimolar mixed cellular barcodes, primers (400 nM P7-R2P-341F, 400 nM P5-index-R1P-R, 10 nM Biotin-link-805R, and 10 nM Biotin-Link-F), ddPCR™ Supermix for Probes (No dUTP) (Bio-Rad), 128 units of Platinum Taq (Invitrogen), and 100 nM NTP. The mixture solution was vortexed at 3,200 rpm for 1 minute and then encapsulated into droplets by a Bio-Rad droplet generator, and 30 μL of the mixture solution and 80 μL of Droplet Generation Oil for Probe (Bio-Rad) were loaded in each channel of the DG8™ Cartridge (32 channels were used for each sample). To measure the mock cell population, approximately 600,000 cells were mixed with 2,400 μL of a solution containing approximately 600,000 copies of the cellular barcodes, 320 units of Platinum Taq, and primers, dNTPs, and ddPCR™ Supermix for Probes (No dUTP); subsequently, the mixture solution was vortexed and then encapsulated into droplets using 80 channels per sample. A library for MiSeq sequencing was generated by one-step PCR in droplets (at 95°C for 5 minutes; six cycles at 94°C for 45 seconds and at 60°C for 150 seconds; 49 cycles at 94°C for 25 seconds and 60°C for 80 seconds; at 98°C for 10 minutes).

Collection and purification of the library

The library generated by the droplet amplification technique was collected using chloroform, 80 μL of TE buffer (Invitrogen) and 280 μL of chloroform (Sigma) were mixed with droplets collected from each DG8™ Cartridge (eight wells), then the mixture was pipetted 10 times and vortexed until aqueous and organic phases were separated; and, after centrifugation (at 21,900 g for 10 minutes), an aqueous-phase solution containing the library was extracted. Subsequently, off-target DNA molecules such as unlinked barcode amplification products, remaining primers, and byproducts in the collected solution were removed by bead purification using AMPure XP and gel purification using 2% E-Gel™ EX Agarose Gels (Thermo Fisher Scientific Inc.). Subsequently, biotinylated and unbound 16S rRNA amplification products were removed using streptavidin magnetic beads (NEB), and unbound 16S rRNA amplification products were biotinylated with a primer Biotin-link-805R (Figure 5).[28] The purification steps using AMPure XP, gel, and streptavidin beads were performed twice each. Finally, the purified library was concentrated using DNA Clean and Concentrator Kit (Zymo Research). The quality of the library was examined using Agilent 2100 Bioanalyzer, and the concentration was measured by qPCR (KAPA SYBR Fast qPCR kit; KAPA Biosystems) using primers P1_qPCR_Fw and P2_qPCR_Rv (Table 3). The detailed protocol for the purification steps using AMPure XP, gel, and streptavidin beads was followed in accordance with the user manual for each product.

MiSeq sequencing

Pair-end sequencing was performed for the library of the samples on the MiSeq platform (MiSeq Reagent Kit v3, 600 cycles; Illumina), allocating 30 cycles for Read 1, 295 cycles for Index 1, eight cycles for Index 2, and 295 cycles for Read 2 (Figure 5). The primer for sequencing Illumina Index 1 was replaced with a custom primer designated as I1_primer (Table 3), to read the 16S rRNA sequences instead of indices. To maintain heterogeneity of the sequences for sequencing, a separately prepared spike-in control was sequenced together with the samples (Figures 18 and 19). More specifically, the total concentration of bacteria, extracellular DNA, or cellular barcodes was measured by Droplet Digital™ PCR (ddPCR) in accordance with the instruction of QX200TM ddPCR EvaGreen™ Supermix (Bio-Rad). For samples of bacteria and extracellular DNA, F1-Fw and F1-Rv, which were primers targeting the V1-V2 region of the 16S rRNA gene, or 341F and 805R, which are primers targeting the V3-V4 region of the 16S rRNA gene, were used (Table 3). For the cellular barcodes, primers Biotin-Link-barcode-F and P5-index-R1P-barcode-R (containing an index GTACTGAC) were used (Table 3). QX200TM ddPCR™ EvaGreen™ Supermix, 1 $\mu$M primers, 1 $\mu$M dNTPs, and the samples (subjected to multiple dilutions and vortexed at 3,200 rpm for 1 minute) were mixed to make a volume of 30 pL, and the mixture was pipetted to mix. Subsequently, the mixture solution was encapsulated into droplets using Droplet Generation Oil for EvaGreen (Bio-Rad), DG8™ Cartridge (Bio-Rad), and Droplet Generator (Bio-Rad). Droplet PCR was performed with the following steps: the initial denaturation at 95°C for 5 minutes; denaturation at 95°C for 45 seconds, annealing and elongation with six cycles at 60°C for 150 seconds; denaturation at 95°C for 25 seconds, and annealing and elongation with 39 cycles at 60°C for 80 seconds (primers F1-Fw and F1-Rv) or denaturation at 95°C for 25 seconds and annealing and elongation with 34 cycles at 60°C for 80 seconds (primers 341F and 805R); and signal stabilization at 4°C for 5 minutes and at 90°C for 5 minutes. Subsequently, the fluorescence intensity of droplets was measured using QX200 Droplet Reader (Bio-Rad), and the numbers of positive and negative droplets were determined based on a threshold which is the trough of the bimodal distribution of the intensity obtained using a software QuantaSoft (Bio-Rad) (Figure 18a). Finally, the concentration of the sample was calculated based on the ratio of positive and negative droplets and the dilution rate of the sample.

The sum of the concentrations of cells and extracellular DNA was obtained for an identical sample (a cecal sample obtained from a male C57BL6/J mouse) using both primer sets, F1-Fw/F1-Rv and 341F/805R, and it was confirmed that the concentrations measured using these primer sets were consistent. For the following reasons, primers F1-Fw and F1-Rv were used to measure the concentration of the bacterial sample by BarBIQ.

For this comparison, the ratio of positive and negative droplets was determined by Gaussian fitting because of the clearly ill-defined separation between the distributions of positive and negative droplets when 341F and 805R were used (Figure 18b). The ratio was fitted to the peak of the intensity distribution by a function normalmixEM in the R package mixtools using four Gaussian distributions (Figure 18c). In brief, fitting by two Gaussian distributions, of which one is for positive droplets and the other is for negative droplets, can be sufficient. However, the data clearly showed that there were two or more Gaussian distributions. Therefore, the intensity distributions by different numbers of Gaussian distributions were fitted. The proportion of positive droplets was found to be stable when four or more Gaussian distributions (six or less were tried) were used (Figure 18d). This suggests that four Gaussian distributions is sufficient to explain the intensity distributions. To calculate the proportion of positive droplets, a Gaussian distribution fitted to positive droplets was assumed when the mean of these Gaussian distributions was larger than the apparent trough of the intensity bimodal distribution, and it was opposite for negative droplets. Finally, the proportions of positive droplets were compared between the results obtained using two primer sets, and it was found that basically these proportions did not differ for either the bacterial cell sample or the extracellular DNA sample (Figure 18e). Since separation of positive droplets and negative droplets using primers F1-Fw and F1-Rv was much clearer than when 341F and 805R were used (Figures 18a and 18b), the primers F1-Fw and F1-Rv were selected for BarBIQ.

Adjustment of bacterial concentration and barcode concentration during preparation of droplets

Bacteria were used at a concentration of 250 cells/$\mu$L to generate droplets. Given that the volume of one droplet was approximately 0.8 nL, approximately 20% of droplets were to contain bacteria with this concentration. Following the Poisson distribution, 90% or more of droplets containing bacteria contain only one bacterium, and others contain two or more bacteria under this condition.

In theory, BarBIQ normalizes the proportional concentration of each cOTU determined by sequencing using the total concentration to obtain the absolute concentration of the cOTU, and different cellular barcode concentrations do not change the proportional concentration of each cOTU. Therefore, the concentration of the cellular barcode does not affect the concentration measurement in BarBIQ. However, the cellular barcode with a higher concentration generates more junk amplicons, which may affect identification of the 16S rRNA sequence. Meanwhile, the cellular barcode with a lower concentration would reduce the efficiency of detecting bacteria. We used cellular barcodes in the range from 100 to 250 molecules/$\mu$L for BarBIQ measurement. As a result, 8% to 20% of droplets contained barcodes. Since only droplets containing both cells and barcodes are sequenced, it was predicted that 3% to 11% of droplets would be eventually sequenced.

The detection rates of bacteria cells at these concentrations ranged from 3% to 11%. Approximately three-fold differences in the detection rates were observed for different samples even when cellular barcodes were used at the same concentration, and the differences seem to be attributed to instability at lower concentrations of the cellular barcode molecules. Since the cOTU count determined by sequencing showed a favorable correlation between repeated experiments for showing different cell detection rates, it was indicated that, basically, the detection rate did not affect measurement of proportional concentrations of all detected cOTUs (Figure 15).

Spike-in control for BarBIQ sequencing

As often performed using PhiX in sequencing of amplification products,[54] the designed spike-in control was mixed with the library and sequenced at the same time to avoid disproportionate nucleotide types in sequencing. A schematic view of preparation of the spike-in control is provided in Figure 20. First, StdTarget1 and StdTarget2, two single-stranded DNA (ssDNA) containing 174 and 176 random nucleotides, respectively, were linked by T4 RNA ligase (NEB) overnight at a concentration of 400 nM, and then a denaturation step using enzymes was performed at 65°C for 15 minutes. Subsequently, four different random barcode templates were prepared by elongation using four different primers containing random barcodes (RandomBar_std1, RandomBar_std2, RandomBar_std3, and RandomBar_std4; Figure 16 and Table 3) separately designed from the linked products of StdTarget 1 and StdTarget 2, followed by an annealing step at 90°C to room temperature over 15 minutes, and elongation was performed using Klenow polymerase (NEB). After column purification, four different indexed primers (Index_NSE501 for RandomBar_std2, Index_NSE502 for RandomBar_std3, Index_NSE505 for RandomBar_std4, and Index_NSE506 for RandomBar_std1; Figure 16 and Table 3) were used, a common primer std_R2 was used for the other end, and four different DNA products were amplified from the elongated templates prepared at the final step. A product comprising approximately 600 nucleotide pairs was purified by gel electrophoresis. PCR was performed further two times using primers P1_qPCR_Fw and P2_qPCR_Rv to amplify more products. Products from each round of PCR were purified by gel electrophoresis. The spike-in control was prepared by mixing these four different products in equal ratios based on the concentrations measured by qPCR using primers P1_qPCR_Fw and P2_qPCR_Rv.

Given that, when the mean number of reads per unique barcode exceeds 60, each number of cOTUs are saturated, it was confirmed that the sequencing depth in all the sequencing experiments was sufficient for digital counting (Figures 28 and 34).

Data processing pipeline

A pipeline for identifying the bar sequence and cOTU (cell type) and processing the data obtained by sequencing to quantify each cOTU was developed. The primary strategy of the pipeline is shown in Figure 6, and details of each step were as described in WO 2018/235938A and as follows. As a general rule, the reads from MiSeq were first clustered using the cellular barcode (Read R1).[25] Subsequently, 16S rRNA sequences (Reads I1 and R2) linked to the same cellular barcode were further clustered based on the identity of these sequences. A representative sequence (RepSeq) for each group of clustered 16S rRNA sequences was generated based on both the number of reads and the sequencing quality for each sequence type. Conceivable erroneous RepSeq sequences were removed at a plurality of steps, depending on both the number of reads for each RepSeq and the number of RepSeqs for each RepSeq sequence type, (refer to WO 2018/235938A and see Figure 6). A unique RepSeq sequence type was designated as Bar sequence. Subsequently, Bar sequences were clustered into a cOTU based on their co-detection frequency in the same droplet. If two or more Bar sequences were detected in the same droplet frequently, they were considered to be a plurality of 16S rRNA genes derived from the same bacterium and clustered into a single cOTU. Subsequently, the number of cells for each cOTU was counted by the number of unique cellular barcodes (i.e., barcode cluster). The absolute cell concentration of each cOTU was determined by normalizing cells counted by the sequencing of the cOTU using the total concentration of the sample measured by ddPCR. Further, cOTUs contaminated during sampling and/or measurement were identified using the control.

The most part of the pipeline was written in Perl (version 5.22.1), and others were performed by a software such as R (version 3.5.1), nucleotide sequence clusterizer (version 0.0.7),[25] or bwa (version 0.7.15).[49] The Perl modules and the R packages used for this pipeline are listed in Table 4.

[Table 4]

Table 4: Perl modules and R packages used for data analyses

| Name | Type | Citations |
| --- | --- | --- |
| ape | R package | Paradis E. & Schliep K. 2019. ape 5.0: an environment for modern phylogenetic and evolutionary analyses in R. Bioinformatics, 35, 3, 526-528. |
| dplyr | R package | Hadley Wickham, Romain Fran<U+00E7>ois, Lionel Henry and Kirill M<U+00FC>ller (2018). dplyr: A Grammar of Data Manipulation. R package version 0.7.6. https://CRAN.R-project.org/package=dplyr |
| ggplot2 | R package | H. Wickham. ggplot2: Elegant Graphics for Data Analysis. Springer-Verlag New York, 2016. |
| gridExtra | R package | Baptiste Auguie (2017), gridExtra: Miscellaneous Functions for "Grid" Graphics. R package version 2.3. https://CRAN.R-project.org/package=gridExtra |
| igraph | R package | Csardi G, Nepusz T: The igraph software package for complex network research, InterJournal, Complex Systems 1695, 2006, http://igraph.org |
| Matrix | R package | Bates DM, Maechler M. Package 'Matrix'. R package version 1.2-12. 2017 |
| pheatmap | R package | Raivo Kolde (2019), pheatmap: Pretty Heatmaps. R package version 1.0.12. https://CRAN.R-project.org/package=pheatmap |
| plotrix | R package | Lemon, J. (2006) Plotrix: a package in the red light district of R. R-News, 6(4): 8-12. |
| RColorBrewer | R package | Erich Neuwirth (2014), RColorBrewer: ColorBrewer Palettes, R package version 1.1-2. https://CRAN.R-project.org/package=RColorBrewer |
| scales | R package | Hadley Wickham (2018), scales: Scale Functions for Visualization. R package version 1.0.0. https://CRAN.R-project.org/package=scales |
| smacof | R package | Jan de Leeuw, Patrick Mair (2009). Multidimensional Scaling Using Majorization: SMACOF in R. Journal of Statistical Software, 31(3), 1-30. URL http://www.jstatsoft.org/v31/i03/ |
| stats | R package | R Core Team (2018). R: A language and environment for statistical computing. R Foundation for Statistical Computing, Vienna, Austria. URL https://www.R-project.org/. |
| vegan | R package | Jari Oksanen, F. Guillaume Blanchet, Michael Friendly, Roeland Kindt, Pierre Legendre, Dan McGlinn, Peter R. Minchin, R. B. O'Hara, Gavin L. Simpson, Peter Solymos, M. Henry H. Stevens, Eduard Szoecs and Helene Wagner (2019). vegan: Community Ecology Package. R package version 2.5-4. https://CRAN.R-project.org/package=vegan |
| IPC::System::Simple | Perl module | Paul Fenwick |
| Bio::SeqIO | Perl module | Christopher Fields |
| Bio::Seq | Perl module | Christopher Fields |
| Text::Levenshtein::XS | Perl module | Nick Logan |

(continued)

| Name | Type | Citations |
|---|---|---|
| List::Util | Perl module | Graham Barr, Paul Evans |
| Statistics::Basic | Perl module | Paul Miller |
| Excel::Writer:: XLSX | Perl module | John McNamara |
| Math::round | Perl module | Geoffrey Rommel |

Details of BarBIQ data processing

In our sequencing, R1 (30 nucleotides) was a cellular barcode, I1 (295 nucleotides) and R2 (nucleotides) were 16S rRNA sequences, and I2 (8 nucleotides) was an index for labeling each sample uniquely. All three sequencing operations are summarized in Table 4.

Step 1: Clustering based on cellular barcode

The reads of the cellular barcode (R1) were clustered based on the sequence as reported previously (WO 2018/235938A), excluding the initial deletion of low-quality reads. First, as widely performed,[47] low-quality R1 reads (the mean score < 15) containing at least one window consisting of four consecutive nucleotides were excluded. The proportions of reads of Sequence Runs 1, 2, and 3 were 0.23%, 0.05%, and 0.06%, respectively, and they were excluded by this process. Subsequently, an R1 read that matches the last four fixed nucleotides of the designed cellular barcode was selected for the next step. All R1 reads having distance 2 parameters derived from an identical Sequence Run containing both the sample and the spike-in control were clustered using a software, a nucleotide sequence clusterizer.[25] Reads which had a different index but were clustered into the same cluster were excluded. The obtained cluster was designated as BCluster. Each read had two 16S rRNA sequences (I1 and R2) and a cellular barcode (R1) (Figure 6).

Step 2: Trimming based on low-quality ends and primer portions of reads I1 and R2

At this stage, the ends of all reads were trimmed at specific positions based on the quality of reads and primer portions thereof. The quality of read nucleotides in MiSeq sequencing is generally reduced at the ends of a read, resulting in more errors at the ends.[50] Since maintaining the same read length is necessary at the next stage of data processing, we applied uniform thresholds and trimmed the ends of all reads with one sequencing operation. The trimming position of sequence runs were determined based on the average quality of all reads. The rule of selecting trimming positions was as follows: when the starting average quality of two sequential positions was lower than 25 (incidental changes in the quality during sequencing can be avoided using the average quality at two sequential positions), the position was selected from the head of the read as the first position. Trimming positions 231 (I1) and 194 (R2) were used for Sequence Run 1, 294 (I1) and 267 (R2) were used for Sequence Run 2, and 271 (I1) and 237 (R2) were used for Sequence Run 3. Further, the primer portions of each read were directly trimmed depending on the lengths of designed primers: 21 nucleotides for I1 and 17 nucleotides for R2.

Step 3: Clustering by 16S rRNA sequences (I1 and R2)

At this stage, two substeps of clustering reads in each BCluster were performed based on 16S rRNA sequences (I1 and R2).

Step 3.1: Clustering by substitution distance

In step 3.1, the reads I1 and R2 were clustered by a parameter of distance 3 based on the substitution distance between the reads using a nucleotide sequence clusterizer software, and the reads I1 and R2 having the same MiSeq ID were by physically linked and considered as a single read.

Step 3.2: Clustering based on a single position in a read

Since very similar sequences that might be a true 16S rRNA sequence, not an erroneous sequence, were integrated in Step 3.1, an additional clustering step was used. For each subcluster generated by Step 3.1, reads were re-clustered based on a specific position of the read (all reads were aligned according to a first nucleotide). The logic diagram of this step is shown in Figure 20. For each read position, the number of reads containing each kind of nucleotides (A, T, C, and G) was counted, and the ratio (designated as Ratio2nd) of the number of reads containing the second most abundant nucleotide to the number of reads containing the most abundant nucleotide was calculated. Further, the count of each read was weighted by the quality score of the nucleotide sequence at this position. The rule was as follows: the count was weighted by 0 if the score was lower than 15 and by a score obtained by dividing the score by 41 if the score was 15 or higher. Then, the highest Ratio2nd was selected among all positions and compared with the threshold of 0.75. If the Ratio2nd was 0.75 or higher, reads containing the second most abundant nucleotide were separated into a new subcluster from the original subcluster. Then, the reads in the newly generated subclusters for both sequences were reclustered by the same strategy, and clustering was repeated until the Ratio2nd became lower than 0.75 at all positions in all subclusters. The last subcluster was designated as SCluster (Figure 6). The efficiency of amplifying 16S rRNA sequences (a plurality of 16S rRNA sequences from the same bacterium) in droplets was often biased. Therefore, although the Ratio2nd in this case may be lower than 0.75, both sequences are true 16S rRNA sequences. Fortunately, the amplification bias in these different types of sequences (e.g., A and B) derived from an identical bacterium occurred at random. For example, Sequence A had more reads at times, and Sequence B had more reads at other times. Therefore, both these two sequence types may have been identified from different droplets, and the amplification bias did not affect cell counting. However, the number of droplets in which both sequence types were detected was reduced when the threshold of 0.75 was used, compared with cases where a lower threshold was used. This may affect the step used to identify two sequences from the same bacterium (see Step 12). Meanwhile, when a threshold lower than 0.75 is used, a subcluster containing erroneous sequences alone can be generated, resulting in a problem in the next step. Therefore, the 16S rRNA sequences were identified using the threshold of 0.75. However, when both sequences derived from the same bacterium were detected in the same droplet, the sequences were detected using another threshold of 0.1. The data generated by the threshold of 0.1 were used only for detecting a plurality of 16S rRNA sequences derived from the same bacterium, and only the 16S rRNA sequences identified using the threshold of 0.75 were used. Of note, since only substitution errors were considered in Step 3, all sequences with insertion errors or deletion errors were clustered as SClusters, and adverse reactions of this pipeline were resolved in the next step.

Step 4: Preparation of representative sequence (RepSeq) for each SCluster

For each SCluster, representative sequences (RepSeqs) for both the reads I1 and R2 were generated based on both the sequence quality score of each nucleotide and the proportion of a nucleotide of each type. To calculate the proportion of a nucleotide of each type, the number of reads for a nucleotide of each type was weighted by the quality score. The rule was as follows: the number of reads was weighted by 0 if the quality score was lower than 15 and by a score obtained by dividing the score by 41 if the score was 15 or higher. For each position, the most abundant nucleotide type was used as a representative nucleotide (Figure 6). Since a RepSeq generated from an SCluster of a single read has a high risk of errors, RepSeqs of a single read were also removed at this stage. The number of reads in each SCluster was used at the next stage as important information to distinguish correct RepSeqs from erroneous RepSeqs containing errors.

Step 5: Removal of shifted RepSeq

At this stage, RepSeqs having an error type due to insertion or deletion (indel) which occurred in the head portion (21 nucleotides in I1 and 17 nucleotides in R2) of the read excluded as a primer portion in Step 2 were removed. For example, on the assumption that a BCluster x contains reads of the 16S rRNA sequences, when some of them have two deletions in the head portion, two types of reads (RepSeqs i and j (two deletions in the reads)) occur after excising the primer portions, and RepSeq j should have shifted by two nucleotides from left towards right compared with RepSeq i (Figure 21). A RepSeq having this error type was designated as a <u>shifted</u>

<u>RepSeq.</u>

The logic diagram of Step 5 is shown in Figure 21. The strategy was as follows. a) Pairs of all RepSeq types conceivable for each BCluster were found. One RepSeq type was a sequence shifted from another RepSeq type, and only pairs of RepSeq types having a shift of less than eight nucleotides were selected. b) For each pair of the shifted RepSeq types (A and B), a RepSeq type identified in more BClusters was designated as Mother, and the other was considered as Shift wherever possible. This is because, in general, there are less erroneous sequences than correct sequences. c) For each pair of Mother and Shift, the number of BClusters having more reads of Mother than Shift ($No_{Mother}$) and the number of BClusters in the opposite case ($No_{Shift}$) were counted. Only BClusters including both Mother and Shift were used. d) Subsequently, since there are less erroneous sequences than correct sequences, Mother and Shift were preserved when $No_{Mother}$ is larger than $No_{Shift}$. e) If corresponding Shift exists in a BCluster including Mother, the Shift in this BCluster was deleted, the number of reads in this Shift was added to the that of Mother (the total number of reads for Mother was used at the next step). If a Shift existed in BCluster not including Mother, Mother was replaced with the Shift (if two or more Mothers existed for an identical Shift, the Mother identified in more BClusters was to be selected), and the number of reads in the Shift was to be used as the number of reads for the replaced Mother. According to this rule, the Shift was removed based on the pairs of Mother and Shift preserved in d). The RepSeqs I1 and R2 were independently processed.

Step 6: Linking of RepSeqs I1 and R2

In this step, the RepSeq I1 and the RepSeq R2 were linked based on overlapped sequences at the ends thereof. The lengths of the 16S rRNA gene in the V3-V4 region depended on the Silva database (version 123.1) and were distributed in a range from almost (> 99.9%) 400 to 500 bp (Figure 22). Therefore, sequencing of 295 nucleotides of reads for both I1 and R2 can basically achieve 90 or more overlapped nucleotides between the ends of each read pair of I1 and R2. However, because of the low sequencing quality at the ends of each read (see Step 2), the only sequences that can be used for data processing were approximately 294 nucleotides in I1 and approximately 267 nucleotides in R2 based on the best experience in the performed sequence runs. However, 60 or more overlapped nucleotides can still be detected. Therefore, to obtain the full length of the 16S rRNA gene in the V3-V4 region, overlapped sequences between the RepSeq I1 and the RepSeq R2 were found, and a linking step was performed using them as a single RepSeq. However, because of the poor sequencing quality, only 231 nucleotides in I1 and 194 nucleotides in R2 were used for Sequence Run 1. Therefore, no overlapped sequences were detected, and the RepSeqs I1 and R2 were not linked.

In general, several nucleotides at the ends of both the RepSeqs I1 and R2 can be the same incidentally. Therefore, it was considered that this was because an overlap of five or more identical nucleotides at the ends of both the RepSeq I1 and the RepSeq R2 was used as a threshold to avoid false overlaps. In theory, the possibility of incidental overlaps is $(1/4)^b$, wherein b is the number of overlapped nucleotides, and the possibility of accidental overlapping of five nucleotides is $(1/4)^5 \leq 0.00098$.

Further, in the data of the mock cell population and M0, all incidental overlaps were < 5 nucleotides (overlaps could not be found because short reads were used).

A difference due to substitutions between the RepSeq I1 the RepSeq and R2 might occur, although rarely, in the overlapped portion because the quality at the end portions of reads was relatively low. Therefore, another step was applied to remove these errors. The strategy for this step was as follows. a) After the above-described linking step, RepSeqs that had not been linked were found. b) Subsequently, each RepSeq was compared with other RepSeqs in an identical BCluster (directly compared with the respective RepSeqs I1 and R2), and a RepSeq having a one-nucleotide difference was found. c) RepSeqs with a one-nucleotide difference were linked, RepSeqs that had not been linked were deleted, and the reads were added to the linked RepSeqs.

Step 7: Removal of RepSeq having one insertion or deletion (1-indel)

In this step, RepSeqs having an error type arising from one insertion or deletion (1-indel) error in the main portion of the read (not in the head portion of the read (i.e., the primer portion; see Step 5)) were removed. Since clustering in Step 3 was based only on substitution as described above, all erroneous reads containing an indel were separated to prepare individual RepSeqs. In general, an indel error very rarely occurs around the center of the read in sequencing (Schirmer M et al., BMC Bioinformatics 2016; 17:125). Therefore, at the stage, not only a 1-indel, but also two-nucleotide indel having substitution and a 1-indel were considered in Step 9 (described later).

The logic diagram of Step 7 is shown in Figure 23. The strategy is as follows. a) All pairs of RepSeq types that had a 1-indel difference were found in each BCluster. b) Since there are generally less erroneous sequences than correct sequences, the RepSeq type identified in more BClusters was designated as Mother for each pair of RepSeq types having a 1-indel (A and B), and the other was considered as 1-Indel. c) For each pair of Mother and 1-Indel, the number of BClusters having more reads of Mother than those of 1-Indel ($No_{Mother}$) and the number of BClusters in the opposite case ($No_{1-Indel}$) were counted. Only BClusters including both Mother and 1-Indel were used. d) We preserved a pair of Mother and 1-Indel only when $No_{Mother}$ is larger than $No_{1-Indel}$. This is because there are generally less erroneous reads than correct reads. e) The ratio (Rs) of the number of BClusters including possible 1-indels and likely Mothers and the number of all BClusters including possible 1-indels ($No_{1-Indel}$) was calculated, and a conditional statement Rs $\leq$ ($No_{1-Indel}$-

3)/No$_{1\text{-Indel}}$ was true, possible pairs of Mother and 1-Indel were selected. f) Based on the selected pairs Mother and 1-Indel, if a 1-indel exists in Mother and a BCluster, the 1-indel was deleted from this BCluster, and the number of 1-Indel reads was added to that of Mother (the total number of reads was used for Mother in the next step). If 1-Indel exists in BClusters not including Mother, the number of reads of 1-Indel was replaced with that for Mother (if there were two or more Mothers for the same 1-Indel, Mother identified in more BClusters was selected) and used as the number of reads for Mother with which the number of reads of 1-Indel was replaced.

Step 8: Removal of chimera

At this stage, chimeric RepSeqs having an error type arising from chimeric amplification were removed. Chimera occurs during PCR at all times, which makes the product more complex. In particular, chimeric RepSeqs occur very frequently during the measurement of 16S rRNA amplification products.[27]

The logic diagram for removing chimeras is provided in Figure 24. The strategy was as follows. a) In each BCluster, chimeras of RepSeq types (A, B, and C) in all possible orders were examined. The head portion of A was the same as the head portion of B, the other portion of A (containing B) was identical to the end portion of C, the number of reads in A was not the largest among the three, A was considered chimeric, and B and C were considered parents of this chimera. b) For each of the identified chimeras, the number of BClusters including chimeras (Total_No) and the number of BClusters including only chimeras but not including parents (No_d) were counted. c) Conditional statements Ratio_d(= N_d/Total_No) $\leq$ 0.1 and Ratio_d $\leq$ 1/Total_No were true, and candidates of chimeras were excluded from RepSeqs.

Only 1% to 5% were chimeric in BarBIQ, and this proportion is much lower than those by the conventional methods (approximately 70%).[27] It was therefore shown that chimeras were removed by this step. The reason why almost no chimeras were generated by BarBIQ is that a barcode and a sequencing adapter were attached to the 16S rRNA gene derived from a single bacterium by one-step amplification in a separated space (that is, a droplet), and this means that the 16S rRNA amplification products derived from different bacteria were not mixed. This approach was not performed even in the latest studies on high throughput sequencing of the 16S rRNA gene using droplets and barcodes (Borgstrom E et al., Nat Commun 2015; 6: 7173 and Sheth RU et al., Nat Biotechnol 2019; 37(8): 877-883).

Step 9: Removal of rare erroneous RepSeqs

In this step, RepSeqs having high-level errors such as errors of one indel and one substitution (designated as Case A), one indel and two substitutions (designated as Case B), or two indels (designated as Case C) were removed. As described above, only indel errors can occur by our clustering method in Step 3, high-level errors discussed here include indels. Meanwhile, more complex errors occur very rarely and are removed in Step 10.

The logic diagram of Step 9 is shown in Figure 25. The strategy is as follows. a) All possible pairs of RepSeq types in each BCluster having any of the above-described differences (Cases A, B, and C) were identified. b) The numbers of RepSeq reads for each identified pair were compared. If the ratio of the numbers of reads between RepSeqs (small and large) was lower than a threshold of 0.2, RepSeqs having a small number of reads were excluded, and the number of reads was added to other pairs.

Step 10: Removal of low-count RepSeqs

After most errors were removed in the above-described steps, unknown RepSeqs (different from San sequence) still remained in data of the mock cell population. However, all these RepSeqs existed in a small number. Accordingly, the number of BClusters was counted by type of the remaining RepSeqs. Since the variation due to the low count was large, the mean count obtained by repeating the sampling for each RepSeq type (sequencing of the identical sample by a different sampling) was used. For each repeat, the count of each RepSeq type was normalized by the total count of all RepSeq types using the highest total count among all repeats. Subsequently, the mean count of each RepSeq type was calculated from all repeated experiments. Sampling was performed for the mock cell population three times, and the mean count was obtained from three repeated experiments. Finally, if the mean count was lower than 2, the RepSeq type was excluded.

After this step, in the data of the mock cell population excluding the type of RepSeqs which matched the San sequence, all the remaining RepSeq types can be rationally explained by one-nucleotide errors (see Step 11) arising from PCR or contamination (see Step 14).

We also conducted a study using only one repeat or two repeats and found that a threshold of < 6 (one repeat) or a threshold of < 3 (two repeats) functioned for the mock cell population data. However, since one sampling and two samplings are likely to have a higher risk than three samplings because of randomness, < 10 and < 5 were used for one sampling and two samplings, respectively, as the thresholds for the cecal sample.

Step 11: Removal of RepSeqs having a one-nucleotide error

At this stage, one-nucleotide errors of RepSeq types that are considered to have arisen from PCR were removed. To characterize this RepSeq, first, the remaining RepSeq types having a one-nucleotide or zero-nucleotide difference from each San sequence were classified into groups (low-count RepSeq types were maintained for this analysis; see Step 10). Subsequently, the distribution of the mean counts of all RepSeq types in each group was plotted (Figure 26a), and the ratio (highest ratio) of the highest mean count of RepSeq types having a one-nucleotide difference to the mean count of RepSeq types matching the San-sequence in the identical group was calculated (Figure 26b). We found two categories (Categories 1 and 2 in Figure 26b): Category 1 was a group with 1,000 or higher counts of San-sequence-matching RepSeq types, and Category 2 was a group with less than 1,000 counts of San-sequence-matching RepSeq types. In Category 1, the highest mean number of RepSeq types having a one-nucleotide difference was larger than 2, and the highest ratios for these RepSeq types were consistent between the different groups. It was concluded that these RepSeq types having a one-nucleotide difference probably had errors arising from PCR. This is because the ratio of the number of the actual 16S rRNA sequence to the number of other actual 16S rRNA sequences usually varies for each type of the 16S rRNA sequence. Accordingly, a step of removing these RepSeq types was applied using a threshold that the ratio of the count of RepSeq types having a one-nucleotide difference to the count of San-sequence-matching RepSeq types is lower than 1/400 (Figure 26b). If only one repeat measurement was performed, a threshold of 1/100 was used for the data. In Category 2, the highest mean count of RepSeq types having a one-nucleotide difference was < 2, which was similar between the different groups, indicating a possibility that these results may have been due to the low concentration of these 16S rRNA sequence. Errors occurred at random, and all erroneous sequences did not match. Since there was a high risk of low-count RepSeqs, RepSeqs were excluded in Step 10. In the mock cell population data, it was confirmed that, if the ratio of the numbers of counts of two RepSeq types showing only a one-nucleotide difference was 1/50 or higher, both the RepSeq types matched the San sequence. However, no RepSeqs with this ratio being in the range from 1/400 to 1/50 were found. Further, one odd RepSeq type was detected in our data of the mock cell population. This sequence was examined, and it was found that the sequence matched the center of the San sequences JCM5824-A and JCM5824-B and was much shorter than the full length of the V3-V4 region of JCM5824-A/B. The 6-mer at the center of JCM5824-A/B was the same as the 3' end of the forward primer used to amplify the 16S rRNA gene. Given that this odd sequence was always detected simultaneously with the full-length V3-V4 region of JCM5824-A and/or JCM5824-B in an identical droplet, and the count was always very rare (2, 4, and 1 in three repeats), it was interpreted that this odd RepSeq type was a non-specific amplification product of the 16S rRNA gene from JCM5824. However, since a short amplification product of this type was not found in the cecal specimen, no step for detecting these short amplification products was included in our final pipeline. After all the above-described steps, the remaining RepSeq types (unique RepSeqs) were designated as BarBIQ identifying sequences (Bar sequences) and labelled with respective ID numbers.

Step 12: Clustering Bar sequences into a cOTU

Making the best of the great advantage of BarBIQ, a plurality of 16S rRNA sequences were identified from the same bacterium based on cellular barcodes at this stage.

For this purpose, two possibilities should be considered. One is a possibility that different bacteria might be mixed in an identical droplet, and the other is a possibility that only one of different sequences can be detected because of the amplification bias against different sequences from the identical bacterial cell. The first possibility depends on the Poisson distribution and is very rare because bacteria were used at a low concentration to generate droplets. The second possibility is not affected by the concentration of bacteria. It was found that these two possibilities could be distinguished by experimentally setting the ratio of the number of bacteria to the number of droplets as 20%.

To distinguish these two possibilities, the authors checked all possible pairs of Bar sequences. For each pair (labelled as BS_A and BS_B), the number of droplets containing both pairs (designated as Overlap), the number of droplets containing only BS_A (designated as A), and the number of droplets containing only BS_B (designated as B) were counted. These counts are based on the data processed using a parameter 0.1 in the above-described Step 3.2.

In theory, if one pair of Bar sequences are derived from different bacteria, the number of droplets in which both the Bar sequences are detected should follow the Poisson distribution, and the estimated number of droplets detected at the same time (designated as Poission_Overlap) can be calculated as follows:

```
Poission_Overlap = (A × B × μ)/total number of droplets,
```

wherein the total number of droplets is the total number of droplets containing cellular barcodes; $\mu$ is a constant and an integrated parameter for detection efficiency in droplets that can include PCR amplification efficiency, sequencing depth effect, and the like. Meanwhile, if the Bar sequences are derived from an identical bacterium, the number of droplets in which both the Bar sequences are detected would not follow the Poisson distribution.

Subsequently, the parameter was divided into two terms using $\log_{10}$ conversion:

$$\log_{10}(\text{Poission\_Overlap}) = \log_{10}(A \times B) - \log_{10}(\text{total number of droplets}/\mu)$$

While the parameters, A and B, in the first term can be obtained from data, the parameters in the second term, the total number of droplets and $\mu$, cannot be measured individually. It was assumed that $\mu$ was the same for different Bar sequence pairs, and that logic (total number of droplets/p) was constant for all Bar sequence pairs in each experiment. This term was designated as an operational droplet (OD). Subsequently, the OD was estimated by inserting a running median of $\log_{10}(\text{Poission\_Overlap})$ for $\log_{10}(A \times B)$ using a model y = x - OD. In general, in our data, most Bar sequence pairs were derived from different bacteria, and their measured Overlap was similar to theoretical Poission_Overlap. Therefore, the running median of $\log_{10}(\text{Poission\_Overlap})$ was imitated using the running median of $\log_{10}(\text{Overlap})$ (here, a running median is a group consisting of median values in a region of window a of a specific size, median values further obtained by shifting the region by overlap b of a specific size, and further median values obtained by repeating this operation, and a is > b). The running median of $\log_{10}(\text{Overlap})$ was obtained with a window of 0.4 and an overlap of 0.2 based on $\log_{10}(A \times B)$, and only medians exceeding 0 were used (red, white circles in Figure 27a).

After OD was obtained by fitting, data were replotted using $\log_{10}(\text{Overlap})$ against $\log_{10}(A \times B)$ + OD (Figure 27b). In actual fact, this was a relationship between $\log_{10}(\text{Overlap})$ and $\log_{10}(\text{Poission\_Overlap})$. Therefore, data of $\log_{10}(\text{Overlap})$ for which the pair was derived from different bacteria should be on a linear line y = x. However, data were widely distributed because of noises.

Subsequently, simulation was performed to estimate possible distributions of $\log_{10}(\text{Poission\_Overlap})$ for different values of $\log_{10}(A \times B)$ + OD. First, it was confirmed that distributions of $\log_{10}(\text{Poission\_Overlap})$ are slightly different when the values of $\log_{10}(A \times B)$ + OD for different values of A, B, and OD were the same, that the distribution became widest when A is equal to B, and that the distributions of $\log_{10}(\text{Poission\_Overlap})$ was different when the values of $\log_{10}(A \times B)$ + OD were different. Accordingly, simulation was repeated 500,000 times for the distribution of $\log_{10}(\text{Poission\_Overlap})$ for possible values of each of A and B in a range from 1 to 1,500 (A = B, integers) and a fixed value of OD = $\log_{10}(5,000)$. Here, when A is equal to B, the Poisson distribution is considered to be widest. In this case, since it can be estimated that sequence pairs that do not follow the Poisson distribution are more likely to be sequences obtained from different droplets, the simulation was performed with A = B here. The same distribution of higher approximate simulation values was used for the $\log_{10}(A \times B)$ + OD vales between two simulations. Then, a one-sided confidence interval of each distribution was calculated as 0.999 (green line in Figure 27b).

In the data of the mock cell population, all $\log_{10}(\text{Overlap})$ values of Bar sequence pairs derived from an identical bacterium were larger than the upper limit 0.999 of the one-sided confidence interval (UP999), but the values of pairs derived from different bacteria were equal to or smaller than UP999 of the one-sided confidence interval (Figure 27b; It should be noted that $\log_{10}(\text{Overlap})$ values of some Bar sequence pairs were lower than the lower limit 0.999 of the one-sided confidence interval because detection efficiency was not stable, but these values do not affect this purpose). These data indicated that, since the values of $\log_{10}(\text{Overlap})$ of pairs derived from an identical bacterium were significantly larger than $\log_{10}(\text{Poission\_Overlap})$ when 20% was used as the ratio of the number of bacteria to the number of droplets, the pairs derived from an identical bacterium could be easily distinguished by UP999.

Subsequently, the data of M0 were analyzed using the same method as used for measurement of the cecal sample. No clear gaps were observed in the vicinity of UP999 in the plot of $\log_{10}(\text{Overlap})$ against $\log_{10}(A \times B)$ + OD (Figure 27c). To find a favorable threshold for the cecal sample, the mapping information of each Bar sequence based on a public database Silva was used. Since the names of most Bar sequences cannot be determined based on the database at a species level, occasionally even at higher levels, we focused only on Bar sequences mapped to different names. In each of repeated experiments of sampling in the M0 data, the $\log_{10}(\text{Overlap})$ values of several Bar sequence pairs mapped to different names in the database were larger than UP999 (black circle in Figure 27c). Subsequently, when these Bar sequence pairs were examined in two other samplings, all the $\log_{10}(\text{Overlap})$ values were lower than UP999. Since 20,000 or more Bar sequence pairs were examined in one measurement, and it was rational that they were outside the one-sided confidence interval, 0.999, these cases may have occurred statistically by accident.

To avoid statistically rare cases, whether these two Bar sequences were derived from the same bacterium was determined using a plurality of repeated experiments. In theory, the results of Bar sequences from the same bacterium should be the same in different samples. Therefore, all samples can be used as repeats for this purpose. Subsequently, using all cell samples derived from the cecum of mice Ma, Mb, and Mc, the ratio of the number of samples in which the $\log_{10}(\text{Overlap})$ values of Bar sequence pairs were shown to be larger than UP999 to the total number of samples in which both Bar sequences were detected was analyzed. This ratio is referred to as Ratio_Positive. The ratio, rather than the number of samples, is used because some Bar sequences are detected in only some samples, the number of samples that can be used for each Bar sequence pair can vary. To secure reliability, only Bar sequence pairs detected in at least two samples were used. Further, some samples were found to have poor OD fitting, only samples with a standard error of OD by fitting being lower than 0.08 were selected. All Bar sequence pairs mapped to different names based on mapping names of Bar sequences had a low Ratio_Positive (Figure 27d), and the distribution was exponentially attenuated, indicating that such sequences occur with a low possibility. Therefore, Bar sequence pairs derived from an identical bacterium were identified using a threshold of Ratio_Positive > 0.5.

Subsequently, all Bar sequences were classified into groups based on the identified Bar sequence pairs derived from an identical bacterium. Each group can have one Bar sequence or a plurality of Bar sequences. We designated these groups as cell-based operational taxonomic units (cOTUs). The strategy for this classification was to classify the Bar sequences into one group if the Bar sequence and at least one of the plurality of Bar sequence are derived from the same bacterium. Some Bar sequence pairs in some cOTUs could not be detected by the above-described process. This may be because detection efficiency was low when a droplet contains two or more sequences.

Step 13: Counting the number of cells in each cOTU

When RepSeqs detected in an identical BCluster belonged to an identical cOTU, a single cell was assumed. Subsequently, the number of cells in each cOTU was calculated based on cellular barcodes (number of BClusters). The data processed with a parameter of 0.75 in Step 3.2 were used to calculate the number of cells.

Step 14: Removal of cOTUs contaminated with foreign substances

At this stage, cOTUs contaminated with foreign substances were removed based on the control. To identify cOTUs contaminated with foreign substances, measurement was performed for a similar time period (several days) under the same condition using a control sample M0 of the mock cell population or an empty test tube control of the cecal samples of mice Ma, Mb, and Mc.

The strategy for detecting cOTUs contaminated with foreign substances was as follows. The number of BClusters in a cOTU identified in the sample was counted for each control, and the count of each cOTU in the control was compared with the count of identical cOTUs in the sample. In an experiment of the mock cell population, the counts were also normalized by the estimated total number of droplets because a different number of droplets were used to prepare the libraries of the mock cell population and the M0 sample. In another experiment, standardization of the counts was not applied because the control was an empty tube, and the same number of droplets was used in all experiments.

Three different categories (I, II, and III) were found for the mock cell population (Figure 28). (I) The count of cOTUs in the control (i.e., M0) was much larger than that in the sample (i.e., the mock cell population), and the cOTU sequence did not match the San sequence. (II) The number of cOTUs was comparable between the sample and the control (their mean $\pm$ SD were overlapped), and the cOTU sequences did not match San sequence either. (III) The number of cOTUs in the sample was much larger than that in the control, and the cOTU sequence matched San sequence. Category I could not be explained as a chimera because bacteria cross-mixed in from the control to the sample or mixed in from the environment were the same as bacteria in the control (because we used an actual sample as the control for measurement of the mock cell population), and, further, this cOTU sequence was very different from the Bar sequence ($\leq$ 86% identity) that matched the San sequence in the sample. This result indicates that this cOTU does not have an error arising from a chimera. Category II can be explained as foreign substances mixed in from the environment for both the sample and the control. This is because their numbers were similar in the different samples and the control. We removed cOTUs from the samples for both Category I and Category II. This is because they were probably foreign substances mixed in from the environment or other samples. In Category III, bacteria mixed in from the sample to the control or incidentally mixed in from the environment were the same bacteria in the sample although there was a possibility that foreign substances were cross-mixed in. In this case, the value obtained by subtracting the count of this cOTU in the control from the count of this cOTU in the sample was used as the final number of cells in this cOTU in the sample. There was a possibility of cross-mixing of foreign substances only when the concentration of cOTUs in the sample was high, and these counts in the sample contaminated with foreign substances were very rare.

Since only one measurement was performed for each sample for data of mice Ma, Mb, and Mc, the square root of the count was used as an error bar based on the Poisson sampling noise instead of a repeat SD.

For the data of mice Ma, Mb, and Mc, two empty test tubes were used as controls. In this case, the two test tubes were experimental repeats rather than sampling of repeats, and do not follow the Poisson distribution. Further, to avoid an accident due to a small number of repeats, $3.27 \times$ SD was used as an error bar against the controls. Further, if $3.27 \times$ SD is smaller than 10% of the mean count, 10% of the mean was used as an error bar. The rule of removing cOTUs contaminated with foreign substances from these samples was as follows. If the count in the control + the error bar was higher than the count in the sample - the error bar, this cOTU was removed from the sample. If the count in the control + the error bar was lower than the count in the sample - the error bar, the count in the sample - the count in the control was used as the final count of the cOTUs in the sample.

The number of cells in the cOTU contaminated with foreign substances was approximately 0.5% of the number of all cells detected in measurement of the mock cell population, and approximately 4% in measurements of the cell samples from Ma, Mb, and Mc.

Step 15: Calculation of cell concentrations

The absolute cell concentration in each cOTU was calculated by normalizing by the count obtained in Step 13 using the total concentration measured by droplet digital PCR.

Comparison with 16S rRNA gene databases

The sequence identity between the 16S rRNA genes closest (i.e., highest identity) to the Bar sequence identified in three different public databases, GreenGene (release 13_5),[10] Ribosomal Database Project (release 11.5),[11] and Silva (release 131.1)[12] was calculated using the NCBI blast (version 2.7.1).[51]

Taxonomic prediction by an RDP classifier

Classification of the identified cOTUs from the phylum to the genus was predicted based on their Bar sequences by a RDP classifier using the bootstrap cutoff of 50%.[36] The RDP classifier was trained using the 16S rRNA training set[11] (https://rdp.cme.msu.edu/classifier/classifier.jsp). A predicted taxon having the highest score was selected for the cOTU including a plurality of Bar sequences.

Bray-Curtis dissimilarity

The bray-Curtis dissimilarity between the samples of each pair based on the cell concentrations was calculated using the vegdist function of vegan R package. Subsequent analyses were performed using R (version 3.5.1) and JupyterLab (version 0.34.9).

Estimation of technical noises

Noises in the cOTU in the technical replicates of a sample Ma$^{dist}$ measured by BarBIQ were examined mainly from the sampling noises by comparing the mock noises obtained from the Poisson distribution and the technical noises of the cOTU. To eliminate a bias from the different total numbers of detected cells in the technical replicates, the number of cells for each replicate was standardized for the minimum total number of cells in the replicate by subsampling using dilution of a function in the vegan R package. The noises in the cOTU were quantified by CV$^2$. Here, the CV represents a change in the coefficient calculated based on the normalized numbers of cells in cOTUs in three technical replicates.[52,53] The Poisson noise simulated for each cOTU was calculated based on three numbers (to imitate three technical replicates) randomly generated from the Poisson distribution, which is the mean number of cells in a given cOTU in the sample, to perform two simulations (1 and 2). Subsequently, a residual error of CV$^2$ after correction of the theoretical mean was calculated for each cOTU.[52,53]

$$R_{mc} = \log_{10}(CV^2) - \log_{10}(CV_{Poisson}^2)$$

Here, the CV$_{Poisson}$ is a theoretical CV for a predetermined cOTU based on the Poisson distribution. The distribution of all R$^{mc}$ in the sample of Ma$^{dist}$ matches the distribution of the simulation, indicating that technical noises in the BarBIQ measurement were mainly due to sampling (Figures 7c and 7d).

Estimation of confidence intervals of mouse-dependent CVs

For each cOTU, 95% confidence intervals of CVs of the cell concentrations at distal and proximal positions in three mice (Ma$^{dist}$1, Mb$^{dist}$, and Mc$^{dist}$ or Ma$^{prox}$1, Mb$^{prox}$, and Mc$^{prox}$) were estimated by simulation. The simulation process was repeated 1,000 times, and the CV was obtained from three simulated cell concentrations for the given cOTU for each time. Each simulated concentration was obtained by a random number generated by the Poisson distribution. The mean was the number of sequenced cells in the given cOTU in the sample (i.e., one of Ma$^{dist}$1, Mb$^{dist}$, Mc$^{dist}$,

or Ma$^{prox}$1, Mb$^{prox}$, Mc$^{prox}$) and was then normalized using the estimated total concentration of this sample. This estimated total concentration was randomly generated from the normal distribution with the mean being the measured flora concentration of this sample and the standard deviation being of 10.1% of the mean (10.1% was the maximum standard deviation (10.1%) standardized by the mean of five independent experiments of repeated filtering (Figure 18)). A 95% confidence interval of each CV was obtained from the distribution of CVs from 1,000 simulations.

Corrected bacterial network

Hierarchical clustering was performed by a function hclust in a statistical package (a heat map was generated using the Pheatmap package). The distance used for clustering is defined as 1 - minimum (|r'|) [r' $\in$ (r - OCI, r + OCI)], wherein OCI means a 90% one-sided confidence interval of each r. Evolutionary trees of hierarchical clustering were obtained by the complete linking method. Specifically, a Pearson correlation coefficient r between all included cOTUs was obtained. Then, a distance between one microorganism and another microorganism was determined based on the above-mentioned expression, and cOTUs were clustered based on the distance. The distance between possible cOTU pairs within a branch after clustering was lower than the height of the branch. The OCI of each r was obtained by simulation. The simulation process was repeated 1,000 times, the cell concentration in each cOTU was randomly generated for each time for the samples Ma$^{dist}$1, Ma$^{prox}$1, Mb$^{dist}$, Mb$^{prox}$, Mc$^{dist}$, and Mc$^{prox}$ (this process is the same as the above-described simulation of the confidence interval of the CV) to calculate Pearson's r for each cOTU pair. Then, the OCI was obtained from the distribution of simulators simulated 1,000 times.

A network of cOTUs for each SCBG obtained using a threshold of 0.6 was visualized by a force-directed layout[39] using the igraph of the package, and the layout of nodes (i.e., cOTUs) in the network was created using r which is larger than 0.9, and all r between cOTUs were displayed with color gradients using the RColorBrewer package.

The network of SCBGs based on the correlation between SCBGs for each possible pair of SCBGs ($R_{inter}$) was visualized by a force-directed layout using the igraph package. The layout of SCBGs was determined based on $R_{inter}$ that is larger than 0.7, and all $R_{inter}$ between SCBGs were drawn by lines with color gradients using the RColorBrewer package. Kruskal-Wallis test for a comparison of the means of $R_{inter}$ was performed using the function Kruskal.test in the R package stats.

Example 1: Attaching indices (indexing) to single bacterial cells included in microbiota, allocating single RNA barcodes (Barcoding), and counting the numbers of cell units and molecules by sequence decoding

Interactions between a microbiota and a host are associated with homeostasis and many diseases of the host.[13-16] To further understand the mechanism of interactions between the microbiota and the host in an integrated manner, it is important not only to study the microbiota, but also to link compositional analyses of the microbiota to other analyses, such as metabolomics and/or transcriptomics, for both the microbiota and the host.[5] For this purpose, measurement of concentrations based on a commonly usable unit, for example, the number of cells per weight and/or the number of molecules per volume is required. However, it has been difficult to measure the microbiota composition at a cell level with current techniques.[6-8] Furthermore, a microbiota comprises an enormous number of bacteria of a large number of bacterial species.[17] Therefore, a high throughput cell quantification method having a high taxonomic resolution ability is demanded.

High throughput methods based on sequencing of 16S rRNA gene amplification products using next-generation sequencing techniques have contributed to studies of the diversity of bacteria in a given cell population over many years.[22,23] However, since conventional methods amplify the 16S rRNA gene from a purified bulk bacterial genome and measure the number of amplified molecules, they basically have the following limitations.

1) Since different species have a different copy number of the 16S rRNA gene on the genome, and the copy number is unknown for most species, it is difficult to measure the number of cells and compare the numbers of cells of different species;
2) Identification of 16S rRNA sequences is not accurate because of errors occurring during sequencing and amplification, resulting in a low taxonomic resolution ability.

In fact, sequencing errors were corrected using molecular barcodes,[24-26] but amplification errors due to a chimera generated mainly during sequence amplification have not been sufficiently removed.[27]

To overcome these limitations of the conventional methods, a cell quantification method associated with accurate identification of the 16S rRNA genes and BarBIQ (Figures 1a and 29) was developed. First, a sample was prepared in a buffer solution, and the aggregates were broken by vortex. Subsequently, the bacterial sample was mixed with a solution containing cellular barcodes,[25,26] primers, and reagents for DNA amplification, and the mixture was encapsulated into droplets with a size of 100 $\mu$m. The ratio of single cells and single cellular barcodes (i.e., DNA molecules) was adjusted based on the concentrations of barcodes and bacteria and the Poisson distribution, so that approximately 4% of droplets would have both single cells and single cellular barcodes. For subsequent sequencings, the amplified barcode and sequencing adapter were linked to the 16S rRNA gene amplified in the droplet by single-step amplification (approximately 450 nucleotides in the V3-V4 region)[28] (Figure 5). After amplification, droplets were cut, the library (linked amplicons) was purified, and both the cellular barcodes and the 16S rRNA sequences of the individual amplified molecules were sequenced using a high throughput sequencer MiSeq. We analyzed the sequenced molecules (i.e., reads) for each sequence type of the barcode (i.e., cell), identified the type of each cell based on the 16S rRNA sequence, and counted the number of cells for each cell type (Figure 6). This analysis also functioned for bacteria having a plurality of 16S rRNA sequence types on the genome. This is because the same cellular barcode was linked to a plurality of amplified 16S rRNA sequences derived from an identical cell. Finally, the cell concentration for each cell type in a sample was obtained by normalizing the number of sequenced cells using the total concentration in the identical sample measured by droplet digital PCR (see the section of the "BarBIQ method").

The essential difference between BarBIQ and conventional methods is the unit for defining the composition of a microbiota. In conventional methods, the unit is an operational taxonomic unit (OTU), and this essentially represents a group of similar obtained 16S rRNA sequences by clustering based on the identity of sequences obtained from bulk sampling.[30] However, BarBIQ uses cell types classified based on the 16S rRNA sequence identified from each barcoded cell. To distinguish the cell-based method of the present inventors and conventional methods using OTUs, the classification unit obtained herein was designated as "cell-based operational taxonomic unit (cOTU)."

First, it was demonstrated that BarBIQ acted on the mock cell population including 10 types of cultured human intestinal bacterial strains (Table 1). It was found that each of 16 sequences (Bar sequences) derived from the mock cell population including two pairs of Bar sequences identified by BarBIQ had a one-nucleotide difference (Figure 1b).

All 16 Bar sequences were found to be identical to one of 16S rRNA sequences identified by Sanger sequencing of 10 cultured strains (San sequences) (Figure 1b). It should be noted that some San sequences were not found by BarBIQ and detected from only one or two cells by Sanger sequencing. Subsequently, 10 cOTUs were identified from the 16 Bar sequences based on the cellular barcodes, and each of them corresponded to one of the 10 strains (Figures 1b and 20 to 28). In contrast, when the identical mock cell populations were measured by a usual method, only two of 12 types of representative OUT sequences were found to be identical to one of San sequences (Figure 1b). Therefore, BarBIQ had one-nucleotide precision and resolution ability for identifying 16S rRNA sequences. Here, it was concluded that the conventional method performed here was infeasible.

Subsequently, the concentration of each cOTU in the mock cell population ($[C]_{BarBIQ}$) (per volume) was measured by BarBIQ. It was confirmed that the concentration measured by BarBIQ matched the cell concentration measured by microscopic imaging ($[C]_{Microscopy}$, Figure 1c) . The Pearson moment correlation coefficient r (Pearson's r) between two measured values was 0.98. The mean ratio ($[C]_{BarBIQ}/[C]_{Microscopy}$) was 0.88, which was determined by applying it to a logarithmic scale using a fixed gradient 1 ($R^2$ = 0.95). This result indicated that BarBIQ had accurately measured the cell concentration of each bacterium (cOTU) in the mock cell population.

Subsequently, we applied BarBIQ to the microbiota derived from the murine cecum. The cecum functions as a microorganism fermentation container[31] and often selected as a sampling site for studies of microbiota-related diseases.[32,33] As recently reported,[34] we removed extracellular DNA from the cecal specimen because extracellular bacterial DNA might affect the quantification of the intestinal microbiota.

In three co-accommodated male C57BL6/J mice (Ma, Mb, and Mc), a microbiota was investigated at two positions, distal (dist) and proximal (prox) positions of the coloncecum and small intestine-cecum joints (Figure 2a). Measurement was performed for the samples collected at both sites in Ma (three repeated experiments each were performed at a distal site, Ma$^{dist}$1, Ma$^{dist}$2, and Ma$^{dist}$3, and at a proximal site, Ma$^{prox}$1, Ma$^{prox}$2, and Ma$^{prox}$3) and for other samples (Mb$^{dist}$, Mb$^{prox}$, Mc$^{dist}$, and Mc$^{prox}$). A total of $1.3 \times 10^5$ bacteria cells were counted, and 604 cOTUs including 730 Bar sequences were identified. Surprisingly, 230 identified Bar sequences (32% of the 730 sequences) have not yet been registered in the widely used three different public databases (GreenGene,[10] Ribosomal Database Project,[11] and Silva[12]), and their identity to the closest registered sequence was 86.9% to 99.9% (Figure 2b). Since BarBIQ was found to have a single-nucleotide precision by the mock cell population, it was concluded that BarBIQ could identify unknown 16S rRNA sequences.

Subsequently, the cell concentration was quantified for each cOTU identified in the sample as described above. First, it was confirmed that technical replicates of the identical sample had high reproducibility (Pearson's r $\geq$ 0.982; Figure 15), and that noises for quantification including the filtration step were mainly due to sampling (Figure 7b to 7d). Subsequently, the cell concentrations were compared between the samples, using 240 cOTUs detected in all samples. We found that the concentrations of 10 to 97 cOTUs (Figures 2d, 2e, and 16a to 16c) of each sample pair (i.e., different positions and/or different mice) were different. The differences were larger than the sampling noises, and the change factor was larger than 2 (dots which were outside the confidence interval and whose change factor was two or more times larger in Figures 2d, 2e, and 16; i.e., cOTUs considered to have a different concentration), but the concentrations of other 143 to 230 cOTUs (60% to 96% of the 240 cOTUs) were consistent (dots in the range of the confidence interval or with a change factor being less than two times in Figures 3d, 3e, and 16a to 16c). For example, when differences between healthy mice and diseased mice are to be described, consistent identification of bacteria would be crucial.

To quantify the overall difference in each sample pair, Bray-Curtis dissimilarity (diversity of $\beta$ based on abundance)[35] analysis was performed based on the cell concentrations in 240 cOTUs (Figure 2f). Consistent with the above-described observation results, the differences between different samples (between samples from different sites and between samples from different mice in Figure 2f) were significantly larger than the differences between the repeated experiments in Ma (see Ma$^{dist}$ and Ma$^{prox}$ in Figure 2f). Furthermore, heterogeneity between samples at a proximal position or a different position (circular symbols in Figure 2d) from different mice was higher than those at different positions from the same mouse or at a distal position from different mice (triangular symbols in Figure 2d). These results indicated that the quantitative analysis of comprehensive cell-based differences in microbiotas showed that microbiotas from distal and proximal positions in the same mouse or different mice were different overall.

Further, both the position-dependent (dependent on the positions of the sample collection site (i.e., distal and proximal positions)) and mouse-dependent differences in concentrations were investigated for each of the 240 cOTUs. First, three repeated experiments were performed for each position in Ma to statistically compare cOTU concentrations between different positions of the same mouse. Of these, the concentrations of 13 cOTUs (5% of the 240 cOTUs) were significantly different (FDR < 0.05 and change factor > 2) (Figure 8), and the maximum change factor (based on the mean concentration among repeated experiments) was 4.1.

Subsequently, consistency of the cell concentrations in three mice was quantified by calculating the coefficient of variation (CV, CV obtained by dividing the standard deviation of cell concentrations for three mice by their mean value) for cOTUs at each site (Figure 3a). The authors compared CVs between distal and proximal positions for each cOTU (Figures 9a and 9b) and found that CVs of the majority of cOTUs did not vary depending on the simulated confidence interval. Interestingly, consistency of cOTUs of an identical genus (i.e., CV) often varied (Figures 9a and 9b) (it should be noted that classification was predicted by the Bar sequence of each cOTU using RDP classifier[36] because information from the public databases was limited). For example, the CV of cOTUs of the genus Clostridium XIVa ranged from 0.05 to 1.70 at the two sites (Figure 3b). Interestingly, it has been reported that one species belonging to this genus produces a short chain fatty acid such as butylate,[37] which is the main function of the cecum.[31] This finding indicates that quantifying cells at more detailed levels than a genus level, in particular, quantifying cells at a cOTU level is required to further understand the physiological roles of bacteria.[37,38]

To understand the relationships between cells, bacterial networks were explored based on correlations between cOTU pairs. Correlating bacterial networks associated with transition of conditions in humans have been shown over several years. However, the network analyses so far have been performed basically based on OTUs at the genus level or higher levels, in other words, based on OTUs, not cOTUs. In this example, using the measured concentrations of six samples (Ma$^{dist}$1, Ma$^{prox}$1, Mb$^{dist}$, Mb$^{prox}$, Mc$^{dist}$, and Mc$^{prox}$), correlations based on cell concentrations were characterized by calculating Pearson's r for each pair of 296 commonly detected cOTUs on a logarithmic scale (Figure 4a). No cOTUs having a high correlation with the majority of cOTUs were found, but some cOTUs strongly correlated with some other cOTUs (Figure 10).

Accordingly, we performed hierarchical clustering based on distances of all possible cOTU pairs and found groups of bacteria in which all cOTUs were strongly correlated (strongly correlated bacterial groups; SCBGs) using Iris (Figure 4b). To identify SCBGs including both positively and negatively correlated cOTUs, |r|, not r, was used, which means that the SCBG defined herein is a "relationship group." To secure the reliability of identification of SCBGs, simulated errors were considered to calculate Iris. When the threshold of 0.6, which is expressed as a dotted line on the evolutionary tree in Figure 4b, was used, a total of 31 SCBGs were found. SCBG was defined as a branch which was lower than the threshold and included three or more cOTUs (Figures 4b and 17). The obtained SCBGs were characterized. The number of cOTUs in an SCBG varied from 3 to 19 (Figure 11c), and more than half (16/31) of SCBGs included positively and negatively correlated cOTUs (Figures 4c and 12a to 12f). The mean abundances of cOTUs in each SCBG were widely distributed (Figures 4c and 11d), and the highest difference in abundance between the SCBGs was 230 times, which was observed in SCBG12 (Figure 11d). As in Figure 3c, taxonomic prediction (Figure 4c) of each cOTG was different species (Figures 4c and 13). These findings indicated that a strong relation was possible even if bacteria belonged to different taxa or their abundances were different. The number of SCBGs and the number of cOTUs in each SCBG change as a function of the threshold (Figures 11a and 11b), and the threshold of 0.6 used herein seems to be a transition point for the number of SCBGs (Figure 11a). To define SCBGs, a different threshold may be selected so that specific characteristics can be found in a given sample.

To assess characteristics of a bacterial microbiota at the entire network level, correlations between SCBGs were investigated using all possible SCBG pairs. The correlation $R_{inter}$ between two SCBGs was defined as the mean of |r|s calculated for all possible cOTU pairs where two cOTUs in a pair are from different SCBGs (Figure 4d). First, it was confirmed that all $R_{inter}$ were basically lower than the $R_{inner}$, which is a correlation within the SCBGs defined as the mean of |r|s calculated for all possible cOTU pairs in each SCBG (Figure 14a). Subsequently, each SCBG was found to show a relatively high correlation with a small number of SCBGs. Finally, the mean of $R_{inter}$ between an SCBG and all other SCBGs was calculated for each SCBG, and the mean of all 31 SCBGs was found not to have a significant difference using Kruskal-Wallis test (chi-square value = 30, df = 29, p value = 0.41) (Figure 14b). These findings indicated that the average characteristics of all SCBGs in the overall network were not clear. Analyses of the entire bacterial networks, which compare the bacterial network of a disease model with that of a healthy mouse, are considered important, for example, to find characteristics of a disease-related landmark in a microbiota.

Further, the following investigation was performed.

Experiment 1. Subdivision of large intestine sample

The large intestine was subdivided, and an analysis of each subdivided fragment was attempted in a state in which position information could be identified. Specifically, immediately after a mouse was slaughtered, the entire large intestine was removed and spread to make it linear, and the positional relationships of the solid contents in the large intestine were photographed. One solid content in the large intestine was removed in a state wrapped in the intestinal wall using sterilized scissors and sterilized tweezers and placed in the center of the hole of a brain slicer (Muromachi Kikai Co., Ltd., MK-RC-01) with the cecal side on the left (Panel a of Figure 39). At this time, the brain slicer had been marked beforehand so that the cecal side (A) and the anal side (E) could be distinguished.

Subsequently, after sterilization in an autoclave, 3% agarose (Nacalai Tesque, 01157-95)-containing 1 × TAE (Nacalai Tesque, 32666-81) that had been incubated at 50°C was quietly poured (Panel b of Figure 39) and allowed to stand at -20°C for 30 minutes to embed the large intestine content in an agarose gel (Panel c of Figure 39). The brain slicer was removed from a freezer set at -20°C, and a sterilized razor blade (Muromachi Kikai Co., Ltd., TCB-100) was inserted into a groove of the brain slicer positioned approximately 1 mm left from the center of the solid large intestine content and the second groove positioned to the right thereof (Panel d of Figure 39). Since the grooves of the brain slicer used in this experiment had a width of 1 mm, the central portion having a thickness of 2 mm (hereinafter, Area C) was separated from the large intestine content by this operation. Then, a razor blade was inserted into each of the grooves positioned at 2 mm or more and 3 mm or less inside from the left and right ends of the solid large intestine contents (Panel e of Figure 39) to separate the cecal side end portion (hereinafter, Area A) and the anal side end portion (hereinafter, Area E). The widths of Area B (between Area A and Area C) and Area D (between Area C and Area E) varied depending on the length of the entire solid large intestine content. Further, in some intestinal samples, the length of the entire content was short, and either Area B or Area D was missing. Finally, a razor blade was inserted into each of the grooves positioned 1 mm or more outside from the left and right ends of the solid large intestine content. By removing the razor blades from the brain slicer, a section containing the large intestine content in each area was collected in a state in which the razor blade was attached thereto, and the large intestine content in each area was placed in a DNA LoBind Tube (Eppendorf, 0030108051) using sterilized tweezers. After removed from the brain slicer, Area C was placed on a sterilized Petri dish (Panel f of Figure 39), divided into the central portion (hereinafter, Area CC) and the peripheral portion (hereinafter, Area CO) using a sterilized 15-gauge metal needle (Musashi Engineering, Inc., SNA-15G-B), and collected into DNA LoBind Tubes. A series of the above-described operations were also repeated for other solid large intestine content areas.

As a result, the solid large intestine contents could be divided into areas of A, B, CC, CO, D, and E (Panel g of Figure 39) (however, Area B or D might be missing for the above-mentioned reasons).

Experiment 2. Scientific experimental consideration of effects of presence and absence of bacteria on distribution of barcode sequences

Under a condition where bacteria cells were contained or not contained, the concentration of an equimolar mixed four cellular barcode template (hereinafter, equimolar mixed cellular barcodes) was measured by ddPCR. Specifically, first, QX200™ ddPCR™ EvaGreen Supermix (Bio-Rad, #1864034), 1 μM primers (NoBiotin-Link-barcode-F and P5-index-R1P-barcode-R), 0.1 μM dNTPs (New England BioLabs Inc., N0447), Platinum Taq DNA Polymerase (Thermo Fisher Scientific, 10966034), and a sample (equimolar mixed cellular barcodes and bacteria cells collected from the murine cecum, or equimolar mixed cellular barcodes alone) were mixed to make a volume of 30 μL and divided into DG8 Cartridge (Bio-Rad, #1864008). Subsequently, the mixture solution was encapsulated into droplets using Droplet Generation Oil for EvaGreen (Bio-Rad, #1864006) and Droplet Generator (Bio-Rad, #1864002JA).

ddPCR was performed by the following steps:

First stage, at 95°C for 5 minutes;
Second stage, six cycles at 95°C for 45 seconds and at 60°C for 150 seconds;
Third stage, 39 cycles at 95°C for 25 seconds and at 60°C for 80 seconds; and
Fourth stage, at 4°C for 5 minutes and at 90°C for 5 minutes.
Then, the barcode concentrations were measured using QX200 Droplet Reader (Bio-Rad, #1864003JA).

The results showed that no significant differences were found in the measured values of the barcode concentrations under a condition where bacterial cells were contained or not contained (see Figure 40), and that the presence or absence of bacteria cells did not affect the distribution ratio of barcodes to droplets.

Experiment 3.Experiment of changing the number of cycles in ddPCR

This experiment examined whether the number of PCR cycles for preparing a sequence library by the BarBIQ method was sufficient to amplify 16S rRNA sequences in bacteria cells contained in a droplet. Specifically, first, QX200™ ddPCR™EvaGreen Supermix, 1 μM primers (F1-Fw and F1-Rv), 0.1 μM dNTPs, and a sample (bacteria cells collected from the murine cecum) were mixed to make a volume of 30 μL and divided into DG8 Cartridge.

Subsequently, the mixture solution was encapsulated into droplets using Droplet Generation Oil for EvaGreen and Droplet Generator. ddPCR was performed under the same cycle condition as in the above Experiment 2, excluding the third stage. In the third stage, the number of cycles was changed to 0, 10, 20, 30, 39, or 49. Then, the fluorescence intensity of droplets was measured using QX200 Droplet Reader, and positive and negative droplets were determined based on a threshold which is the trough of a bimodal distribution of the intensity, using a software QuantaSoft (Bio-Rad, #1864011JA).

The results showed that the intensity distributions of positive droplets and negative droplets were clearly separated (Panel a of Figure 41), and the proportion of positive droplets to all droplets was constant at approximately 14% (Panel b of Figure 41) under a condition where the number of cycles in the third stage was 30 or more, indicating that the number of PCR cycles for preparing a sequence library by the BarBIQ method was sufficient to amplify the 16S rRNA sequences in bacteria cells contained in a droplet.

Experiment 4.Experiment of changing the time for a ddPCR step

This experiment examined whether the initial denaturation time for preparing a sequence library by the BarBIQ method was sufficient to amplify 16S rRNA sequences of bacteria cells contained in a droplet. Specifically, first, QX200™ ddPCR™EvaGreen Supermix, 1 μM primers (F1-Fw and F1-Rv), 0.1 μM dNTPs, and a sample (bacteria cells collected from the murine cecum) were mixed to make a volume of 30 μL and divided into DG8 Cartridge. Subsequently, the mixture solution was encapsulated into droplets using Droplet Generation Oil for EvaGreen and Droplet Generator. ddPCR was performed under the same cycle conditions as in the above Experiment 2, excluding the first stage. In the first stage, the time was changed to 0, 5, or 10 minutes. Then, the fluorescence intensity of droplets was measured using QX200 Droplet Reader, and positive and negative droplets were determined based on a threshold which is the trough of a bimodal distribution of intensity, using a software QuantaSoft.

The results showed that the proportion of positive droplets to all droplets did not change even if the time of the first stage was changed (Figure 42), indicating that the initial denaturation time for preparing a sequence library by the BarBIQ method was sufficient to amplify 16S rRNA sequences of bacteria cells contained in a droplet.

Currently, absolute quantification,[3] accurate measurement,[40] complete gene sequencing,[41] and bacteriabacteria interactions tend to be considered in studies of microbiotas based on 16S rRNA gene amplification products.[42] However, these considerations have not yet been associated with quantification of cells. As far as we know, BarBIQ is the first method that enables high taxonomic resolution quantification of a bacterial microbiota composition at a cell level in a high throughput format. Furthermore, one-nucleotide precision identification of unknown 16S RNA sequences by BarBIQ without using databases is considered useful for other studies. For example, to find a location of a newly found bacterium, fluorescence in situ hybridization (FISH) can be performed by designing a fluorescence probe using a 16S rRNA sequence identified by BarBIQ.

Recently, different meta-omics datasets, such as metagenomics, transcriptomics, proteomics, and metabolomics, were integrated, and further calculation models using these datasets have been proposed as a promising direction for studying the mechanism of microbiota functions.[5] Since bacterial cells not only integrate clearly different meta-omics datasets, but also are fundamental units for their functions in this approach, microbiota should be defined at a cell level. The analyses of microbiotas provided by BarBIQ, which are cell-based and not dependent on taxa, evolve microbiota studies from the current association research to necessary mechanism research.[44]

**Claims**

1. A method for treating a cell population, the method comprising

   (A) obtaining a droplet population from a cell dispersion comprising an isolated cell population, the droplet population comprising aqueous droplets, at least some of which each comprise one cell and one-molecule cellular barcode.

2. A method for analyzing nucleotide sequences of genes included in a cell population, the method comprising

(A) obtaining a droplet population from a cell dispersion comprising an isolated cell population, the droplet population comprising aqueous droplets, at least some of which each comprise one cell and one-molecule cellular barcode; and

(B) obtaining an amplification product of the cellular barcode and an amplification product of a predetermined gene in each obtained droplet, further obtaining a linked product comprising nucleotide sequences of the cellular barcode and all or some of the predetermined gene, and collecting the obtained linked product from the droplets into an aqueous solution and sequencing the obtained linked product to determine the nucleotide sequence of the predetermined gene and the nucleotide sequences of the cellular barcode.

3. The method according to claim 2, wherein, in the (B), the amplification product of the cellular barcode has a first region derived from a first primer, the amplification product of the predetermined gene has a second region derived from a second primer, the first region and the second region have complementary sequence portions hybridizable with each other, the first primer and the second primer each have one or more tag molecules linked thereto, and the tag molecule is not included in the linked product; and

the method further comprising removing the amplification product having a tag molecule from the linked products collected into the aqueous solution using a column or a bead carrying a molecule having an affinity for the tag molecule in the (B).

4. The method according to claim 2 or 3, further comprising
(C-1) clustering the determined nucleotide sequences based on the determined nucleotide sequence of the cellular barcode to obtain a plurality of first clusters.

5. The method according to claim 4, further comprising
(D-1) estimating the number of cells included in the cell population or the number of cells having a specific predetermined gene from the number of the obtained first clusters.

6. The method according to claim 2 or 3, further comprising
(C-2) clustering the determined nucleotide sequences based on the determined nucleotide sequence of the predetermined gene to obtain a plurality of second clusters.

7. The method according to claim 6, further comprising
(D-2) estimating the number of cell types included in the cell population from the number of the obtained second clusters.

8. The method according to claim 2 or 3, further comprising
(C-3) clustering the determined nucleotide sequences based on the determined nucleotide sequence of the cellular barcode to obtain a plurality of first clusters, and clustering the determined nucleotide sequences based on the determined nucleotide sequence of the predetermined gene to obtain a plurality of second clusters.

9. The method according to claim 8, further comprising
(D-3) determining the first cluster, into which the nucleotide sequence of the predetermined gene is classified, from the nucleotide sequence of a cellular barcode linked to the nucleotide sequence of the predetermined gene classified into at least one second cluster based on information on combinations of the obtained nucleotide sequence of the cellular barcode and the obtained nucleotide sequence of the predetermined gene, and estimating the number of cells classified into the second cluster from the number of the first clusters into which the cellular barcode is classified.

10. The method according to claim 8, further comprising
(C-4) when sequences classified into one identical first cluster are classified into different second clusters, classifying the second clusters into one identical cell-based operational taxonomic unit (cOTU).

11. The method according to claim 10, further comprising
(E) estimating (i) the number of cOTUs included in the cell population and/or (ii) the number of cells included in a specific cOTU for each of a first cell population and a second cell population different from the first cell population, and comparing (i) the number of cOTUs and/or (ii) the number of cells included in the specific cOTU estimated for the first cell population with (i) the number of cOTUs and/or (ii) the number of cells included in the specific cOTU estimated for the second cell population.

12. The method according to claim 11, comprising
(F) comparing (i) the number of cOTUs and (ii') the number of cells included in the specific cOTU estimated for the first cell population with (i) the number of cOTUs and (ii') the number of cells included in the specific cOTU estimated for the second cell population.

13. The method according to any one of claims 1 to 12, wherein the cell population is a microbiota.

14. The method according to claim 13, wherein the microbiota is a microbiota in the body or on the body surface.

15. The method according to claim 13, wherein the microbiota is a microbiota in the gastrointestinal tract.

16. The method according to claim 11 or 12, wherein the first cell population and the second cell population are microbiotas obtained from different sites of an identical subject.

17. The method according to claim 11 or 12, wherein the first cell population and the second cell population are microbiotas obtained from an identical site of different subjects.

18. The method according to claim 11 or 12, wherein the first cell population and the second cell population are microbiotas obtained from an identical site of an identical subject at different time points.

19. The method according to any one of claims 1 to 18, wherein the cell population includes unknown cells.

FIG. 1

FIG. 2

# FIG. 3

**a** COTU-CM-0365

Cells/mg

CV:
0.31
0.48

Ma  Mb  Mc

COTU-CM-2018

Cells/mg

CV:
1.69
1.70

Ma  Mb  Mc

**b**

○ Distal    ○ Proximal

CV(SD/Mean)

COTU-CM-

0307 0242 0365 0321 2022 0575 2014 0275 2016 2020 2027 0250 0285 2049 0273 0570 0238 0271 0488 0363 0249 0330 2029 0384 0255 0208 2043 0418 2018

Genus : *Clostridium* XIVa    Family : *Lachnospiraceae*
Order *Clostridiales*    Class : *Clostridia*
Phylum : *Firmicutes*

# FIG. 4

# FIG. 5

Extended Data 1

Single-step amplification in droplets

MiSeq sequence

# FIG. 6

Extended Data Figure 2

Schematic view of data processing

Barcode
Read:R1

16S rRNA sequence
I1                      R2

↓ Cluster by barcode.

↓ Cluster by 16S rRNA.

↓ For each SCluster
(number of reads > 1)

↓ Obtain a representative sequence
(RepSeq).

RepSeq 1-1:
......A......T......G......

↓

↓ For each BCluster

RepSeq 1-1:
RepSeq 1-2:
RepSeq 1-3:
RepSeq 1-4:

↓ Remove RepSeq having a shift.
↓ Link I1 and R2.

RepSeq 1-1:
RepSeq 1-2:
RepSeq 1-3:

↓ Remove RepSeq having one
insertion or deletion.
↓ Remove chimeric RepSeq.
↓ Remove RepSeq having rare errors.

RepSeq 1-1:
RepSeq 1-2:

↓ Count each RepSeq.

RepSeq-type 1: .......... 10
RepSeq-type 2: .......... 10
RepSeq-type 3: .......... 20

↓ Remove low-count RepSeq.
↓ Remove RepSeq having a
one-base error.
↓ Designate RepSeq as Bar sequence.

| RepSeq type | Name | Count |
|---|---|---|
| RepSeq-type 1: | Bar sequence 1 | 10 |
| RepSeq-type 2: | Bar sequence 2 | 10 |
| RepSeq-type 3: | Bar sequence 3 | 20 |

↓ Cluster BISes into cOTU.
↓ Count cells for each cOTU.
↓ Remove cOTUs contaminated with
foreign substances.

| Cell type | Bar sequence | Cell count |
|---|---|---|
| COTU1 | Bar sequence 3 | 20 |
| COTU2 | { Bar sequence 1 | 10 |
| | Bar sequence 2 | |

# FIG. 7

Extended Data Fgure 3

# FIG. 8

Extended Data Figure 4

# FIG. 9a

Extended Data Figure 5

# FIG. 9b

Extended Data Figure 5 continued

# FIG. 10

Extended Data Figure 6

# FIG. 11

Extended Data Figure 7

FIG. 12a

Extended Data Figure 8

FIG. 12b

# FIG. 12c

# FIG. 12d

## Extended Data Figure 8 continued

FIG. 12f

# FIG. 13

Extended Data Figure 9

**Phylum name**
- Proteobacteria
- Firmicutes
- CandidatusSaccharibacteria
- Bacteroidetes

**Class name**
- Deltaproteobacteria
- Betaproteobacteria
- Alphaproteobacteria
- Clostridia
- Bacilli
- CandidatusSaccharibacteria
- Bacteroidia

**Order name**
- Desulfovibrionales
- Burkholderiales
- Rhodospirillales
- Clostridiales
- Lactobacillales
- CandidatusSaccharibacteria
- Bacteroidales

**Family name**
- Desulfovibrionaceae
- Sutterellaceae
- Rhodospirillaceae
- Ruminococcaceae
- Peptostreptococcaceae
- Lachnospiraceae
- Eubacteriaceae
- Defluviitaleaceae
- Clostridiales_IncertaeSedisXIII
- Christensenellaceae
- Catabacteriaceae
- Lactobacillaceae
- CandidatusSaccharibacteria
- Rikenellaceae
- Prevotellaceae
- Porphyromonadaceae
- Bacteroidaceae

# FIG. 14

Extended Data Figure 10

**a**

**b**

# FIG. 15

# FIG. 16c

Different positions in difference mice

# FIG. 17

# FIG. 18

# FIG. 19

# FIG. 20

Step3.2

BCluster 1
BCluster 2
BCluster 3

Step3.1

SCluster 1-1
SCluster 1-2
SCluster i-1
SCluster i-2

Re-assess.

SCluster i-1
1  A
2  A
3  A
4  A
5  A
6  G

SCluster i-1'
7  T
8  T
9  T

Separate reads containing the second most abundant nucleotide.

Each SCluster

$$Ratio2nd = \frac{\text{Number of reads containing the second most abundant nucleotide}}{\text{Number of reads containing the most abundant nucleotide}}$$

If/else  Ratio2nd < 0.75 (threshold)

Other descriptions:
(1) Weighting by quality score
If the score < 15, weight with 0.
If the score ≥ 15, weight with score/41.
(2) Based on the position of reads having the largest Ratio2nd

No

Reads    SCluster i-1
1   A
2   A
3   A
4   A
5   A
6   G
7   T
8   T
9   T
10  T

Position having the largest Ratio2nd

Yes

SCluster 1-1
SCluster 1-2    SCluster 1-2'
SCluster i-1    SCluster i-1'    SCluster i-1"
SCluster i-2

End of step 3.2

# FIG. 21

# Step 5

A pair of RepSeqs one of which may have shifted

BCluster 1
BCluster 2
BCluster 3

Check each BCluster.

BCluster X Identical sequence
RepSeq-x-i AT...............XX...............
RepSeq-x-j ...............CG

Find all pairs of RepSeqs one of which may have shifted less than 8 nucleotides.

A pair of RepSeqs one of which may have shifted
{RepSeq-1-A    {RepSeq-2-A    {RepSeq-3-A    {RepSeq-3-A  •••••
{RepSeq-1-B    {RepSeq-2-B    {RepSeq-3-B    {RepSeq-3-C

Count the number of each of the above RepSeqs:

No[k][i]: Number of BClusters including RepSeq-k-i

O[k][i>j]: Number of BClusters including both RepSeq-k-i and RepSeq-k-j (only when the number of reads of RepSeq-k-i is larger than that of RepSeq-k-j)

O[k][i<j]: Number of BClusters including both RepSeq-k-i and RepSeq-k-j (only when the number of reads of RepSeq-k-j is larger than that of RepSeq-k-i)

A pair of RepSeqs one of which may have shifted
| RepSeq-1-A | RepSeq-2-A | RepSeq-3-A | RepSeq-3-A |
| RepSeq-1-B | RepSeq-2-B | RepSeq-3-B | RepSeq-3-C |
| No[1][A] | No[2][A] | No[3][A] | No[3][A] |
| No[1][B] | No[2][B] | No[3][B] | No[3][C]  ••••••• |
| O[1][A>B] | O[2][A>B] | O[3][A>B] | O[3][A>C] |
| O[1][A<B] | O[2][A<B] | O[3][A<B] | O[3][A<C] |

Consider Mother and Shift as follows:
If No[k][i] > No[k][j] and O[k][i>j] > O[k][j>i], RepSeq-k-i is Mother, and RepSeq-k-j is Shift. Otherwise, if No[k][i] < No[k][j] and O[k][i>j] < O[k][j>i], RepSeq-k-j is Mother, and RepSeq-k-i is Shift. If not either of the above, delete the pair.

Pairs of RepSeqs one of which has shifted
{RepSeq-1-A Mother  {RepSeq-3-A  Shift    {RepSeq-3-A  Shift   •••••••
{RepSeq-1-B  Shift   {RepSeq-3-B Mother   {RepSeq-3-C Mother

Consider 3 pairs in all BCluster.

Case 1: Including both Mother and Shift

| BCluster X | |
| RepSeq-1-A | R[1][A] |
| RepSeq-1-B | R[1][B] |
| ⋮ | |

Delete a shift or substitution.

| BCluster X | |
| RepSeq-1-A | R[1][A] + R[1][B] |
| ⋮ | |

Check each BCluster again.

Case 2: Including only Shift

| BCluster Y | |
| RepSeq-1-B | R[1][B] |
| ⋮ | |

| BCluster Y | |
| RepSeq-1-A | R[1][B] |
| ⋮ | |

Case 3: Including Shift having two Mothers

| BCluster Z | |
| RepSeq-3-A | R[3][A] |
| ⋮ | |

If(No[3][C] > No[3][B])

| BCluster Z | |
| RepSeq-3-C | R[3][A] |
| ⋮ | |

**End of step 5**

Note: R[k][i] is the number of reads for RepSeq-k-i.

## FIG. 22

# FIG. 23

## Step 7

### A pair of RepSeqs that may have 1-indel

BCluster 1
BCluster 2
BCluster 3
⋮

Check each BCluster.

BCluster X   1-indel
RepSeq-x-i ........................A◄.......................
RepSeq-x-j ....................   ...................G

Find all pairs.

**Pairs of RepSeqs that may have 1-indel**

{ RepSeq-1-A        { RepSeq-2-A        { RepSeq-3-A        { RepSeq-3-A
  RepSeq-1-B          RepSeq-2-B          RepSeq-3-B          RepSeq-3-C   ......

Count the number of each of the above RepSeqs:

No[k][i]: Number of BClusters including RepSeq-k-i

O[k][i>j]: Number of BClusters including both RepSeq-k-i and RepSeq-k-j (only when the number of reads of RepSeq-k-i is larger than that of RepSeq-k-j)

O[k][i<j]: Number of BClusters including both RepSeq-k-i and RepSeq-k-j (only when the number of reads of RepSeq-k-j is larger than that of RepSeq-k-i)

**Pairs of RepSeqs that may have 1-indel**

{ RepSeq-1-A        { RepSeq-2-A        { RepSeq-3-A        { RepSeq-3-A
  RepSeq-1-B          RepSeq-2-B          RepSeq-3-B          RepSeq-3-C
  No[1][A]            No[2][A]            No[3][A]            No[3][A]
  No[1][B]            No[2][B]            No[3][B]            No[3][C]    ......
  O[1][A>B]           O[2][A>B]           O[3][A>B]           O[3][A>C]
  O[1][A<B]           O[2][A<B]           O[3][A<B]           O[3][A<C]

Consider Mother and 1-Indel as follows:
If No[k][i] > No[k][j] and O[k][i>j] > O[k][j>i],
RepSeq-k-i is Mother, and RepSeq-k-j is 1-Indel.
Otherwise, if No[k][i] < No[k][j] and O[k][i>j] <
O[k][j>i], RepSeq-k-j is Mother, and RepSeq-k-i is
1-Indel. If not either of the above, delete the pair.

Consider a new parameter
after decide the mother and 1-Indel
R=(O[k][i>j]+O[k][j>i])/No[1-Indel]

If R<(No[1-Indel]-1/No[1-Indel])
Remove the pair

**Pairs of RepSeqs one of which has 1-indel**

{ RepSeq-1-A Mother      { RepSeq-3-A 1-Indel      { RepSeq-3-A 1-Indel
  RepSeq-1-B 1-Indel       RepSeq-3-B Mother         RepSeq-3-C Mother   ......

Check each BCluster again.

Case 1: Including both Mother and 1-Indel

| BCluster X | |
|---|---|
| RepSeq-1-A | R[1][A] |
| RepSeq-1-B | R[1][B] |
| ⋮ | |

Consider 3 pairs in all BClusters.

| BCluster X | |
|---|---|
| RepSeq-1-A | R[1][A] + R[1][B] |
| ⋮ | |

End of step 7

Case 2: Including only 1-Indel

| BCluster Y | |
|---|---|
| RepSeq-1-B | R[1][B] |
| ⋮ | |

Delete 1-indel or substitution.

| BCluster Y | |
|---|---|
| RepSeq-1-A | R[1][B] |
| ⋮ | |

Case 3: Including 1-Indel having two Mothers

| BCluster Z | |
|---|---|
| RepSeq-3-A | R[3][A] |
| ⋮ | |

If(No[3][C] > No[3][B])

| BCluster Z | |
|---|---|
| RepSeq-3-C | R[3][A] |
| ⋮ | |

Note: R[k][i] is the number of reads for RepSeq-k-i.

# FIG. 24

## Step 8

A set of three RepSeqs one of which may be chimeric

BCluster 1
BCluster 2
BCluster 3

Check each BCluster.

BCluster X                    Chimera
RepSeq-x-i ·································
RepSeq-x-j ·································    Parent
RepSeq-x-k ·································

If the chimeric sequence does not have the largest number of reads, record all three in the set.

Sets of three RepSeqs that may be chimeric
RepSeq-1-P1   RepSeq-2-P1   RepSeq-3-P1   RepSeq-4-P1
RepSeq-1-P2   RepSeq-2-P2   RepSeq-3-P2   RepSeq-4-P2    ······
RepSeq-1-C    RepSeq-2-C    RepSeq-3-C    RepSeq-4-C

Count the numbers of each of the above RepSeqs:

NoC[k]: Number of BClusters including only RepSeq-k-c, which is not a parent
Total_No[k]: Number of all BClusters including RepSeq-k-c

Sets of three RepSeqs that may be chimeric
RepSeq-1-P1    RepSeq-2-P1    RepSeq-3-P1    RepSeq-4-P1
RepSeq-1-P2    RepSeq-2-P2    RepSeq-3-P2    RepSeq-4-P2
RepSeq-1-C     RepSeq-2-C     RepSeq-3-C     RepSeq-4-C
No_C[1]        No_C[2]        No_C[3]        No_C[4]       ······
Total_No[1]    Total_No[2]    Total_No[3]    Total_No[4]

Consider new parameters:

$Ratio\_d = No\_C/Total\_No$

Define a chimera as a chimera that is possible only when $Ratio\_d \leq 0.1$ or $Ratio\_d \leq 1/total\ No.$

Chimera
RepSeq-1-C          RepSeq-3-C          RepSeq-4-C        ······

Check each BCluster again.

Delete chimeras in all BClusters.

BCluster X
RepSeq-1-C
⋮

BCluster X
⋮

**End of step 7**

# FIG. 25

## Step 9

FIG. 26

# FIG. 27

# FIG. 28

# FIG. 29

# FIG. 30

# FIG. 31

# FIG. 32

# FIG. 33

Number of clusters vs. Number of random nucleotides in a barcode

# FIG. 34

Count

$10^4$
$10^2$
10
0

— Matching cOTUs
....... ATCC29098
―― ATCC700926
....... DSM14469
........ JCM10188
........ JCM1297
―― JCM14656
........ JCM17463
....... JCM5824
....... JCM5827
―― JCM9498

0    30    60    90    120

Mean number of reads per barcode

# FIG. 35

a

b

# FIG. 36

FIG. 37

# FIG. 38

# FIG. 39

a

b

c

d

e

f

g

Schematic diagram of the sample after cutting

C area

A  B  C  D  E

2~3 mm    2 mm    2~3 mm

Cecal side    Anal side

# FIG. 40

# FIG. 41

# FIG. 42

## Proportion of Positive Droplets

**INTERNATIONAL SEARCH REPORT**

| International application No. |
| --- |
| PCT/JP2021/020338 |

**A. CLASSIFICATION OF SUBJECT MATTER**
Int.Cl. C12Q1/6869(2018.01)i, C12Q1/02(2006.01)i, C12Q1/686(2018.01)i
FI: C12Q1/6869ZZNA, C12Q1/686Z, C12Q1/02

According to International Patent Classification (IPC) or to both national classification and IPC

**B. FIELDS SEARCHED**

Minimum documentation searched (classification system followed by classification symbols)
Int.Cl. C12Q1/6869, C12Q1/02, C12Q1/686

Documentation searched other than minimum documentation to the extent that such documents are included in the fields searched
Published examined utility model applications of Japan     1922–1996
Published unexamined utility model applications of Japan     1971–2021
Registered utility model specifications of Japan     1996–2021
Published registered utility model applications of Japan     1994–2021

Electronic data base consulted during the international search (name of data base and, where practicable, search terms used)
CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP 2018-508198 A (THE REGENTS OF THE UNIVERSITY OF CALIFORNIA) 29 March 2018 (2018-03-29), claims, particularly, claims 96-111, paragraphs [0095]-[0107], [0153], examples, fig. 19 | 1-9, 13-15, 19<br>2-19 |
| X<br>Y | US 2019/0300968 A1 (THE TRUSTEES OF COLUMBIA UNIVERSITY IN THE CITY OF NEW YORK) 03 October 2019 (2019-10-03), whole document, particularly, claims, examples | 1, 13-15, 19<br>2-19 |
| A | JP 2019-531730 A (GENERAL AUTOMATION LAB TECHNOLOGIES INC.) 07 November 2019 (2019-11-07), whole document, particularly, claims, examples | 1-19 |

☐ Further documents are listed in the continuation of Box C.     ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 10 August 2021 | 17 August 2021 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**
Information on patent family members

International application No.

PCT/JP2021/020338

| | | |
|---|---|---|
| JP 2018-508198 A | 29 March 2018 | WO 2016/126871 A2<br>claims, particularly, claims 96-111,<br>paragraphs [0122]-[0134],<br>[0179], examples, fig. 19<br>US 2017/0009274 A1<br>EP 3253479 A2<br>CN 107530654 A |
| US 2019/0300968 A1 | 03 October 2019 | (Family: none) |
| JP 2019-531730 A | 07 November 2019 | US 2018/0024096 A1<br>whole document, particularly,<br>claims, examples<br>WO 2018/064385 A1<br>EP 3519096 A1<br>CN 109843435 A |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9260753 B
- US 10287630 A
- US 10584382 B
- WO 2018235938 A

**Non-patent literature cited in the description**

- **MAIDEN et al.** *PNAS,* 1998, vol. 95, 3140-3145
- **EWING et al.** *Genome Res.,* 1998, vol. 8 (3), 175-185
- **EWING ; GREEN.** *Genome Res.,* 1998, vol. 8 (3), 186-194
- **PARADIS E. ; SCHLIEP K.** *an environment for modern phylogenetic and evolutionary analyses in R. Bioinformatics,* 2019, vol. 35 (3), 526-528
- **HADLEY WICKHAM ; ROMAIN FRAN<U+00E7>OIS ; LIONEL HENRY ; KIRILL M<U+00FC>LLER.** *A Grammar of Data Manipulation.,* 2018, https://CRAN.R-project.org/package=dplyr
- **H. WICKHAM.** Elegant Graphics for Data Analysis. Springer-Verlag, 2016
- **BAPTISTE AUGUIE.** *Miscellaneous Functions for "Grid" Graphics.,* 2017, https://CRAN.R-project.org/package=gridExtra
- **CSARDI G ; NEPUSZ T.** The igraph software package for complex network research. *InterJournal, Complex Systems,* 2006, 1695, http://igraph.org
- **BATES DM ; MAECHLER M.** *Package 'Matrix'.,* 2017
- **RAIVO KOLDE.** *Pretty Heatmaps.,* 2019, https://CRAN.R-project.org/package=pheatmap
- **LEMON, J.** *Plotrix: a package in the red light district of R. R-News,* 2006, vol. 6 (4), 8-12
- **ERICH NEUWIRTH.** *RColorBrewer: ColorBrewer Palettes,* 2014, https://CRAN.R-project.org/package=RColorBrewer
- **HADLEY WICKHAM.** *scales: Scale Functions for Visualization.,* 2018, https://CRAN.R-project.org/package=scales
- **JAN DE LEEUW ; PATRICK MAIR.** Multidimensional Scaling Using Majorization: SMACOF. *R. Journal of Statistical Software,* 2009, vol. 31 (3), 1-30, http://www.jstatsoft.org/v31/i03
- **R CORE TEAM.** R: A language and environment for statistical computing. R Foundation for Statistical Computing, 2018
- **JARI OKSANEN ; F. GUILLAUME BLANCHET ; MICHAEL FRIENDLY ; ROELAND KINDT ; PIERRE LEGENDRE ; DAN MCGLINN ; PETER R. MINCHIN ; R. B. O'HARA ; GAVIN L. SIMPSON ; PETER SOLYMOS.** *vegan: Community Ecology Package,* 2019, https://CRAN.R-project.org/package=vegan
- **SCHIRMER M et al.** *BMC Bioinformatics,* 2016, vol. 17, 125
- **BORGSTROM E et al.** *Nat Commun,* 2015, vol. 6, 7173
- **SHETH RU et al.** *Nat Biotechnol,* 2019, vol. 37 (8), 877-883